# EUROPEAN PATENT SPECIFICATION

(11) **EP 1 737 462 B1**
(45) Date of publication and mention of the grant of the patent: **30.07.2008**
(21) Application number: 04806134.5
(22) Date of filing: 20.12.2004
(51) Int. Cl.: A61K 31/505, C07D 239/42, A61P 35/00, C07D 405/12, C07D 417/12, C07D 413/12, C07D 401/12, C07D 413/14, C07D 403/12, C07D 239/48

(54) **PYRIMIDINES WITH TIE2 (TEK) ACTIVITY**
PYRIMIDINE MIT TIE2 (TEK) AKTIVITÄT
PYRIMIDINES A ACTIVITE TIE2 (TEK)

(30) Priority: 24.12.2003 GB 0330001; 29.07.2004 GB 0416850
(43) Date of publication of application: 03.01.2007
(73) Proprietor: AstraZeneca AB, 151 85 Södertälje (SE)
(72) Inventor: JONES, Clifford David, AstraZeneca R & D Alderley, Macclesfield Cheshire SK10 4TG (GB); LUKE, Richard W. A., AstraZeneca R & D Alderley, Macclesfield Cheshire SK10 4TG (GB); MCCOULL, William, AstraZeneca R & D Alderley, Macclesfield Cheshire SK10 4TG (GB)
(86) International application number: PCT/GB2004/005332
(87) International publication number: WO 2005/060969

(56) References cited:
- WO-A-02/08205
- WO-A-03/080064
- WO-A-03/080625

## Description

This invention relates to compounds, or pharmaceutically acceptable salts thereof, which possess anti-angiogenic activity and are accordingly useful in methods of treatment of disease states associated with angiogenesis in the animal or human body. The invention also concerns processes for the preparation of the compounds, pharmaceutical compositions containing the compounds as active ingredient, and methods for the use of the compounds in the manufacture of medicaments for use in the production of anti-angiogenic effects in warm-blooded animals such as humans.

The Tie2 receptor tyrosine kinase (also known as TEK) is expressed predominantly in endothelial and haematopoietic cells and is essential for vessel formation and maintenance (Jones, N. et al. Nature Reviews Molecular Cell Biology. 2001: 2, 257-67).

Angiogenesis is a fundamental process defined as the generation of new blood vessels from existing vasculature. It is a vital yet complex biological process required for the formation and physiological functions of virtually all the organs. Normally it is transient in nature and is controlled by the local balance of angiogenic and angiostatic factors in a multistep process involving vessel sprouting, branching and tubule formation by endothelial cells (involving processes such as activation of endothelial cells (ECs), vessel destabilisation, synthesis and release of degradative enzymes, EC migration, EC proliferation, EC organisation and differentiation and vessel maturation).

Normal angiogenesis plays an important role in a variety of processes and is under stringent control. In the adult, physiological angiogenesis is largely confined to wound healing and several components of female reproductive function and embryonic development. In undesirable or pathological angiogenesis, the local balance between angiogenic and angiostatic factors is dysregulated leading to inappropriate and/or structurally abnormal blood vessel formation. Pathological angiogenesis has been associated with disease states including diabetic retinopath y, psoriasis, cancer, rheumatoid arthritis, atheroma, Kaposi's sarcoma and haemangioma (Fan et al, 1995, Trends Pharmacology. Science. 16: 57-66; Folkman, 1995, Nature Medicine 1: 27-31). In cancer, growth of primary and secondary tumours beyond 1-2 mm³ requires angiogenesis (Folkman, J. New England Journal of Medicine 1995; 33, 1757-1763).

In diseases such as cancer in which progression is dependant on aberrant angiogenesis, blocking the process can lead to prevention of disease advancement (Folkman, J. 1995, Nature Medicine. 1: 27-31). Many factors are described in the scientific literature that are believed to play important critical roles in the regulation of angiogenesis. Two major classes of angiogenic factors are the vascular endothelial growth factor (VEGF) and the angiopoietins. These polypeptide moieties interact with their respective receptors (transmembrane tyrosine kinases which are predominantly endothelial cell specific) and induce cellular responses via ligand mediated signal transduction. It has been speculated that VEGF and the angiopoietins co-operate to regulate various aspects of the angiogenic process during both normal and pathological angiogenesis via signalling through their respective receptors.

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity that leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt or Flt1, the kinase insert domain-containing receptor, KDR (also referred to as Flk-1), and another fms-like tyrosine kinase receptor, Flt4. Two of these related RTKs, Fit and KDR, have been shown to bind VEGF with high affinity (De Vries et al, 1992, Science 255: 989-991; Terman et al, 1992, Biochem. Biophys. Res. Comm. 1992, 187: 1579-1586): Binding of VEGF to these receptors expressed in heterologous cells has been associated with changes in the tyrosine phosphorylation status of cellular proteins and calcium fluxes.

Recently a second family of predominantly endothelial cell specific receptors that regulate vessel destabilisation and maturation have been identified. The Tie receptors and their ligands, the angiopoietins, co-operate closely with VEGF during both normal and pathological angiogenesis. The transmembrane receptors Tie1 and Tie2, constitute a family of endothelial cell specific tyrosine kinase receptors involved in maintenance of blood vessel integrity and which are involved in angiogenic outgrowth and vessel remodelling. Structurally Tie1 and Tie2 share a number of features (e.g. the intracellular domains of both these receptors each contain a tyrosine kinase domain interrupted by a kinase insert region) and thus constitute a distinct RTK subfamily. Overall sequence identity between Tie1 and Tie2 receptors at the amino acid level is 44% while their intracellular domains exhibit 76% homology. Targeted disruption of the Tie1 gene results in a lethal phenotype characterised by extensive haemorrhage and poor microvessel integrity (Puri, M. et al. 1995 EMBO Journal:14:5884-5891). Transgenic mice deficient in Tie2 display defects in vessel sprouting and remodelling and display a lethal phenotype in mid gestation (E9.5-10.5) caused by severe defects in embryonic vasculature (Sato, T. et al. 1995 Nature 370: 70-74).

To date no ligands have been identified for Tie1 and little is known regarding its signalling abilities. However, Tie1 is believed to influence Tie2 signalling via heterodimerisation with the Tie2 receptor hence potentially modulating the ability of Tie2 to autophosphorylate (Marron, M. et al. 2000 Journal of Biological Chemistry: 275, 39741-39746) and recent chimaeric Tie1 receptor studies have indicated that Tie-1 may inhibit apoptosis via the PI 3 kinase/Akt signal transduction pathway (Kontos, C.D., et al., 2002 Molecular and Cellular Biology : 22,1704-1713). In contrast, a number of ligands, designated the angiopoietins have been identified for Tie2 of which Angiopoietin 1 (Ang1) is the best characterised. Binding of Ang1 induces tyrosine phosphorylation of the Tie2 receptor via autophosphorylation and subsequently activation of its signalling pathways via signal transduction. Ang2 has been reported to antagonise these effects in endothelial cells (Maisonpierre, P. et al. 1997 Science: 277, 55-60). The knock-out and transgenic manipulation of Tie2 and its ligands suggest that stringent spatial and temporal control of Tie2 signalling is imperative for the correct development of new vasculature. There are also reports of at least another two ligands (Ang3 and Ang4) as well as the possibility of heterodimerisation between the angiopoietin ligands that has the potential to modify their activity (agonistic/antagonistic) on association with the receptor. Activation of the Tie2 receptor by Ang1 inhibits apoptosis (Papapetropoulos, A., et al., 2000 Journal of Biological Chemistry : 275 9102-9105), promotes sprouting in vascular endothelial cells (Witzenbicher, B., et al., 1998 Journal of Biological Chemistry: 273, 18514-18521) and in vivo promotes blood vessel maturation during angiogenesis and reduces the permeability and consequent leakage from adult microvessels (Thurston, G. et al., 2000 Nature Medicine: 6, 460-463). Thus activated Tie2 receptor is reported to be involved in the branching, sprouting and outgrowth of new vessels and recruitment and interaction of periendothelial support cells important in maintaining vessel integrity and overall appears to be consistent with promoting microvessel stability. Absence of Tie2 activation or inhibition of Tie2 auto phosphorylation may lead to a loss of vascular structure and matrix/cell contacts (Thurston, G., Cell Tissue Res (2003), 314: 61-69) and in turn may trigger endothelial cell death, especially in the absence of survival or growth stimuli. On the basis of the above reported effects due to Tie2 kinase activity, inhibiting Tie2 kinase may provide an anti-angiogenic effect and thus have application in the therapy of disease states associated with pathological angiogenesis. Tie2 expression has been shown to be up-regulated in the neovasculature of a variety of tumours (e.g. Peters, K.G. et al, British Journal of Cancer 1998; 77,51-56) suggesting that inhibiting Tie2 kinase activity will result in anti-angiogenic activity. In support of this hypothesis, studies with soluble Tie2 receptor (extracellular domain) (Pengnian, L. et al., 1997, Journal of Clinical Investigation 1997: 100, 2072-2078 and Pengnian, L. et al., 1998, Proceedings of the National Academy of Sciences 1998: 95, 8829-8834) have shown anti-tumour activity in *in vivo* tumour models. In addition these experiments also indicate that disruption of the Tie2 signalling pathways in a normal healthy individual may be well tolerated as no adverse toxicities were observed in these studies.

Examination of human primary breast cancer samples and human and murine breast cancer cell lines (Stratmann, A., et al., 2001, International Journal of Cancer: 91,273-282) indicate that Tie2 dependant pathways of tumour angiogenesis may exist alongside KDR dependant pathways and, in fact, may operate both independently (Siemeister G., et al., 1999 Cancer Research: 59,3185-3191) as well as in concert with each other (e.g. VEGF A and Ang1 reported to collaborate to induce angiogenesis and produce non-leaky mature vessels Thurston, G, et al., 1999 Science: 286, 2511-2514). It is quite possible that a mix of such angiogenic processes even exist within a single tumour.

Tie2 has also been shown to play a role in the vascular abnormality called venous malformation (VM) (Mulliken, J.B. & Young, A.E. 1998, Vascular Birthmarks: W. B. Saunders, Philadelphia). Such defects can either be inherited or can arise sporadically. VM's are commonly found in the skin or mucosal membranes but can affect any organ. Typically lesions appear as spongy, blue to purple vascular masses composed of numerous dilated vascular channels lined by endothelial cells. Among the inherited forms of this disease the most common defect appears to be a Tie2 kinase mutation C2545T in the Tie2 coding sequence (Calvert, J.T., et al., 1999 Human Molecular genetics: 8, 1279-1289), which produces a R849W amino acid substitution in the kinase domain. Analysis of this Tie2 mutant indicates that it is constitutively activated even in the absence of ligand (Vikkula, M., et al., 1996 Cell: 87, 1181-1190).

Upregulation of Tie2 expression has also been found within the vascular synovial pannus of arthritic joints in humans, which is consistent with the role of inappropriate neovascularisation.

Such examples provide further indications that inhibition of Tie2 phosphorylation and subsequent signal transduction will be useful in treating disorders and other occurrences of inappropriate neovascularisation. To date only a few inhibitors of Tie2 are known in the art. For example, Internation Application No: WO 04/013141 describes a group of condensed pyridines and pyrimidines and International Application No: WO 04/058776 describes a group of pryidine and pyrimidine compounds. There is thus a need to identify additional Tie2 inhibitors that could exploit the full therapeutic potential of inhibiting/ modulating the Tie2 signalling pathways.

We have found that certain compounds possess inhibitory activity for the Tie2 receptor tyrosine kinase and accordingly have value in the treatment of disease states associated with pathological angiogenesis such as cancer, rheumatoid arthritis, and other diseases where active angiogenesis is undesirable.

According to a first aspect of the present invention there is provided a compound of the Formula I: 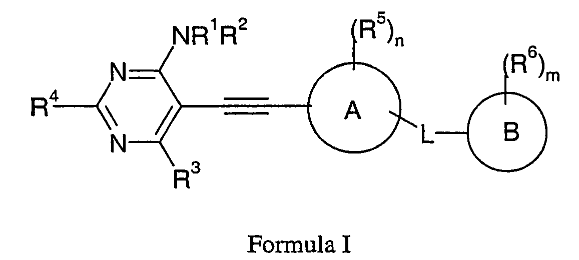 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6 , or a 5 or 6 membered heteroaryl ring, or **R¹ and R²** together with the nitrogen atom to which they are attached represent a saturated or partially saturated 3 to 7 membered heterocyclic ring optionally containing another heteroatom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring,
   wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (104C)alkoxy(1-4C)alkyl, hydroxy, amino, mono(1-6C)alkylamino or di-[(1-6C)alkyl]amino, or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³** is selected from hydrogen, (1-6C)alkyl or (1-6C)alkoxy, wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or **R³** represents a group **-NR¹R²** as defined above;
**R⁴** is selected from hydrogen, (1-6C)alkyl or (1-6C)alkoxy;
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O)O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl ;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
**R⁶** is selected from halo, cyano, oxo, a (3-7C)cycloalkyl ring, a saturated or partially saturated 3 to 7 membered heterocyclic ring, -S(O)ₚ-(1-6C)alkyl wherein p is 0, 1 or 2, -N(R^{a})C(O)(1-6C)alkyl in which R^{a} is hydrogen or (1-6C)alkyl; or
   **R⁶** is selected from (1-6C)alkyl or (1-6C)alkoxy, wherein the (1-6C)alkyl, -S(O)ₚ-(1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
   wherein the (3-7C)cycloalkyl ring and saturated or partially saturated 3 to 7 membered heterocyclic ring are optionally independently substituted by one or more groups selected from (1-6C)alkyl; and
m **is 0, 1, 2 or 3;**
and when B is a (3-7C)cycloalkyl ring or a saturated or partially saturated 3 to 7 membered heterocyclic ring or a saturated or partially saturated 8, 9 or 10 membered bicyclic group, the rings and bicyclic group optionally bear 1 or 2 oxo or thioxo substituents; and salts thereof, particularily pharmaceutically acceptable salts thereof.

According to a second aspect of the present invention there is provided a compound of the Formula I wherein:
**R¹** and **R²** are independently selected from hydrogen, (1-6C)alkylsulfonyl, phenyl(CH₂)ᵤ wherein u is 0, 1, 2, 3, 4, 5 or 6, (1-6C)alkanoyl, (1-6C)alkyl, (1-6C)alkoxycarbonyl, or (3-6C)cycloalkyl(CH₂)ₓ- in which x is 0, 1, 2, 3, 4, 5 or 6, or **R¹** and **R²** together with the nitrogen atom to which they are attached represent a saturated or partially saturated 3 to 7 membered heterocyclic ring optionally containing another hetero atom selected from N or O;
   wherein the alkyl and the cycloalkyl groups are optionally substituted by one or more groups selected from fluoro, hydroxy, (1-6C)alkyl, (1-6C)alkoxy, amino, mono(1-6C)alkylamino or di-[(1-6C)alkyl]amino, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, hydroxy, amino, mono(1-6C)alkylamino or di-[(1-6C)alkyl] amino or a saturated or partially saturated 3 to 7 membered heterocyclic ring;
   and wherein the phenyl is optionally substituted by one or more groups selected from halo, (1-6C)alkyl, (1-6C)alkoxy, amino, mono(1-6C)alkylamino or di-[(1-6C)alkyl]amino, wherein the (1-6C)alkyl or (1-6C)alkoxy are optionally substituted by hydroxy, amino, mono(1-6C)alkylamino or di-[(1-6C)alkyl]amino;
**R³** is selected from hydrogen, (1-6C)alkyl or (1-6C)alkoxy,
   wherein the alkyl and the alkoxy groups are optionally substituted by one or more groups selected from fluoro, hydroxy, (1-6C)alkyl, (1-6C)alkoxy, amino, mono(1-6C)alkylamino or di-[(1-6C)alkyl]amino, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, hydroxy, amino, mono(1-6C)alkylamino or di-[(1-6C)alkyl]amino or a saturated or partially saturated 3 to 7 membered heterocyclic ring; or **R³** represents a group **-NR¹R²** as defined above;
**R⁴** is selected from hydrogen, (1-6C)alkyl or (1-6C)alkoxy;
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyano, halo, (1-6C)alkoxy or (1-6C)alkyl optionally substituted by cyano or by one or more fluoro;
**n** is 0, 1, 2 or 3;
L is attached meta or para on ring A with respect to the point of attachment of the ethynyl group and represents -C(R^{a}R^{b})C(O)N(R⁹)-, -N(R⁸)C(O)C(R^{a}R^{b})- , -N(R⁸)C(O)N(R⁹)-, -N(R⁸)C(O)O-, or -OC(O)N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl ;
**B** represents a (3-7C)cycloalkyl ring, an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁶** is selected from halo, cyano, a saturated or partially saturated 3 to 7 membered heterocyclic ring or an alkanoylamino group -N(R^{c})C(O)(1-6C)alkyl in which R^{c} is hydrogen or (1-6C)alkyl; or **R⁶** is selected from (1-6C)alkyl or (1-6C)alkoxy, wherein the alkyl and the alkoxy groups are optionally substituted by one or more groups selected from cyano, fluoro, hydroxy, (1-6C)alkoxy, amino, mono(1-6C)alkylamino, di-[(1-6C)alkyl]amino, or a saturated or partially saturated 3 to 7 membered heterocyclic ring;
**m** is 0, 1, 2 or 3;
and when m is at least 2 then two substituents on adjacent carbon atoms in ring B may together represent a methylenedioxy group;
and salts thereof, particularily pharmaceutically acceptable salts thereof.

According to a third aspect of the present invention there is provided a compound of the Formula I:
wherein:
**R¹ and R²** are independently selected from hydrogen, (1-6C)alkylsulfonyl, phenyl(CH₂)ᵤ-wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6 , or a 5 or 6 membered heteroaryl ring, or **R¹ and R²** together with the nitrogen atom to which they are attached represent a saturated or partially saturated 3 to 7 membered heterocyclic ring optionally containing another heteroatom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or an alkanoylamino group -N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or alkanoylamino groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (104C)alkoxy(1-4C)alkyl, hydroxy, amino, mono(1-6C)alkylamino or di-[(1-6C)alkyl]amino, or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³** is selected from hydrogen, (1-6C)alkyl or (1-6C)alkoxy, wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or **R³** represents a group **-NR¹R²** as defined above;
**R⁴** is selected from hydrogen, (1-6C)alkyl or (1-6C)alkoxy;
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O)O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl ;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
**R⁶** is selected from halo, cyano, a (3-7C)cycloalkyl ring, a saturated or partially saturated 3 to 7 membered heterocyclic ring or an alkanoylamino group -N(R^{c})C(O)(1-6C)alkyl in which R^{c} is hydrogen or (1-6C)alkyl; or
   **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
**m** is 0, 1, 2 or 3;
   and when B is a (3-7C)cycloalkyl ring or a saturated or partially saturated 3 to 7 membered heterocyclic ring or a saturated or partially saturated 8, 9 or 10 membered bicyclic group, the rings and bicyclic group optionally bear 1 or 2 oxo or thioxo substituents; and salts thereof, particularily pharmaceutically acceptable salts thereof.
   According to another aspect of the present invention there is provided a compound of the Formula I wherein:
wherein:
**R¹ and R²** are independently selected from hydrogen, (1-6C)alkylsulfonyl, phenyl(CH₂)ᵤ-wherein u is 0, 1, 2, 3, 4, 5 or 6, (1-6C)alkanoyl, (1-6C)alkyl, (1-6C)allcoxycarbonyl, (3-6C)cycloalkyl(CH₂)ₓ- in which x is 0, 1, 2, 3, 4, 5 or 6 , or a 5 or 6 membered heteroaryl ring, or **R¹ and R²** together with the nitrogen atom to which they are attached represent a saturated or partially saturated 3 to 7 membered heterocyclic ring optionally containing another heteroatom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or an alkanoylamino group -N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or alkanoylamino groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (104C)alkoxy(1-4C)alkyl, hydroxy, amino, mono(1-6C)alkylamino or di-[(1-6C)alkyl]amino, or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³** is selected from hydrogen, (1-6C)alkyl or (1-6C)alkoxy, wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
**R⁴** is selected from hydrogen, (1-6C)alkyl or (1-6C)alkoxy;
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O)O-, or -OC(O)N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl ;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
R⁶ is selected from one of the two following groups:
   (i) **R⁶** is selected from halo, cyano, a (3-7C)cycloalkyl ring, a saturated or partially saturated 3 to 7 membered heterocyclic ring or an alkanoylamino group -N(R^{c})C(O)(1-6C)alkyl in which R^{c} is hydrogen or (1-6C)alkyl; or **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
      or
   (ii) **R⁶** is selected from halo, cyano, oxo, a (3-7C)cycloalkyl ring, a saturated or partially saturated 3 to 7 membered heterocyclic ring or an alkanoylamino group -N(R^{c})C(O)(1-6C)alkyl in which R^{c} is hydrogen or (1-6C)alkyl;
      or **R⁶** is selected from (1-6C)alkyl, -S(O)ₚ-(1-6C)alkyl wherein p is 0, 1 or 2, or (1-6C)alkoxy,
   wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and wherein the (3-7C)cycloalkyl ring and saturated or partially saturated 3 to 7 membered heterocyclic ring are optionally independently substituted by one or more groups selected from (1-6C)alkyl;
**m** is 0, 1, 2 or 3;
and when B is a (3-7C)cycloalkyl ring or a saturated or partially saturated 3 to 7 membered heterocyclic ring or a saturated or partially saturated 8, 9 or 10 membered bicyclic group, the rings and bicyclic group optionally bear 1 or 2 oxo or thioxo substituents; and salts thereof, particularily pharmaceutically acceptable salts thereof.

A particular embodiment of the compounds of the Formula I is a compound of the Formula Ia: 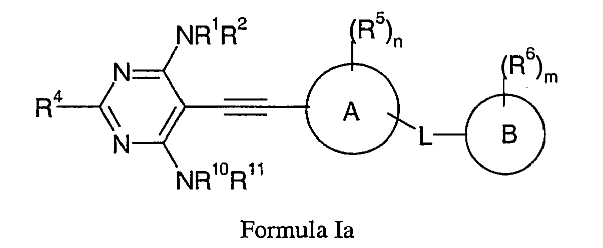 wherein:
**R¹ and R²** are independently selected from hydrogen, (1-6C)alkylsulfonyl, phenyl(CH₂)ᵤ-wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6 , or a 5 or 6 membered heteroaryl ring, or **R¹ and R²** together with the nitrogen atom to which they are attached represent a saturated or partially saturated 3 to 7 membered heterocyclic ring optionally containing another heteroatom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or an alkanoylamino group -N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring,
   wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or alkanoylamino groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino or di-[(1-6C)alkyl]arriino, or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring; and provided that when R¹ and/or R² is a (1C)alkanoyl group, then the (1C)alkanoyl is not substituted by fluoro or hydroxy;
**R⁴** is selected from hydrogen, (1-6C)alkyl or (1-6C)alkoxy;
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O)O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
R⁶ is selected from one of the two following groups:
   (i) **R⁶** is selected from halo, cyano, a (3-7C)cycloalkyl ring, a saturated or partially saturated 3 to 7 membered heterocyclic ring or an alkanoylamino group -N(R^{C})C(O)(1-6C)alkyl in which R^{c} is hydrogen or (1-6C)alkyl; or **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
      or
   (ii) **R⁶** is selected from halo, cyano, oxo, a (3-7C)cycloalkyl ring, a saturated or partially saturated 3 to 7 membered heterocyclic ring or an alkanoylamino group -N(R^{c})C(O)(1-6C)alkyl in which R^{c} is hydrogen or (1-6C)alkyl;
   or **R⁶** is selected from (1-6C)alkyl, -S(O)ₚ-(1-6C)alkyl wherein p is 0, 1 or 2, or (1-6C)alkoxy,
   wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and
   wherein the (3-7C)cycloalkyl ring and saturated or partially saturated 3 to 7 membered heterocyclic ring are optionally independently substituted by one or more groups selected from (1-6C)alkyl;
**m** is 0, 1, 2 or 3;
and when B is a (3-7C)cycloalkyl ring or a saturated or partially saturated 3 to 7 membered heterocyclic ring or a saturated or partially saturated 8, 9 or 10 membered bicyclic group, the rings and bicyclic group optionally bear 1 or 2 oxo or thioxo substituents; and
**R¹⁰ and R¹¹** are independently selected from hydrogen or (1-6C)alkyl;
and salts thereof, particularily pharmaceutically acceptable salts thereof.

Another particular embodiment of the compounds of the Formula I is a compound of the Formula Ia wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, or (3-6C)cycloalkyl(CH₂)ₓ- in which x is 0, 1, 2, 3, 4, 5 or 6, or **R¹ and R²** together with the nitrogen atom to which they are attached represent a saturated or partially saturated 3 to 7 membered heterocyclic ring optionally containing another heteroatom selected from N or O;
   wherein the alkyl and the cycloalkyl groups are optionally substituted by one or more groups selected from fluoro, hydroxy, (1-6C)alkyl, (1-6C)alkoxy, amino, mono(1-6C)alkylamino or di-[(1-6C)alkyl]amino, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, hydroxy, amino, mono(1-6C)alkylamino or di-[(1-6C)alkyl]amino or a saturated or partially saturated 3 to 7 membered heterocyclic ring;
   and wherein the phenyl is optionally substituted by one or more groups selected from halo, (1-6C)alkyl, (1-6C)alkoxy, amino, mono(1-6C)allcylamino or di-[(1-6C)alkyl]amino, wherein the (1-6C)alkyl or (1-6C)alkoxy are optionally substituted by hydroxy, amino, mono(1-6C)alkylamino or di-[(1-6C)alkyl]amino;
**R⁴** is selected from hydrogen, (1-6C)alkyl or (1-6C)alkoxy;
**A** represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyano, halo, (1-6C)alkoxy or (1-6C)alkyl optionally substituted by cyano or by one or more fluoro;
**n** is 0, 1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O)O-, or -OC(O)N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁶** is selected from halo, cyano, a saturated or partially saturated 3 to 7 membered heterocyclic ring or an alkanoylamino group -N(R^{c})C(O)(1-6C)alkyl in which R^{c} is hydrogen or (1-6C)alkyl; or **R⁶** is selected from (1-6C)alkyl or (1-6C)alkoxy, wherein the alkyl and the alkoxy groups are optionally substituted by one or more groups selected from cyano, fluoro, hydroxy, (1-6C)alkoxy, amino, mono(1-6C)alkylamino, di-[(1-6C)alkyl]amino, or a saturated or partially saturated 3 to 7 membered heterocyclic ring; and
**m** is 0, 1, 2 or 3;
   and when m is at least 2 then two substituents on adjacent carbon atoms in ring B may together represent a methylenedioxy group; and
**R¹⁰** and **R¹¹** are independently selected from hydrogen or (1-6C)alkyl; and salts thereof, particularily pharmaceutically acceptable salts thereof.

Another particular embodiment is a group of compounds of the Formula Ia wherein:
**R¹ and R²** are independently selected from hydrogen or (1-6C)alkyl;
**R⁴** is selected from hydrogen, (1-6C)alkyl or (1-6C)alkoxy;
**A** represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from halo, (1-6C)alkyl or (1-6C)alkoxy;
**n** is 0, 1, 2 or 3;
**L** is attached meta or para on ring A with respect to the point of attachment of the ethynyl group and represents -CH₂C(O)N(R⁸)-, -N(R⁹)C(O)CH₂-, -N(R⁸)C(O)N(R⁹)-, -N(R⁸)C(O)O-, or-OC(O) N(R⁹)- wherein R⁸ and R⁹ independently represent hydrogen or (1-6C)alkyl;
**B** represents a (3-7C)cycloalkyl ring, an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁶** is selected from halo, cyano, a saturated or partially saturated 3 to 7 membered heterocyclic ring or an alkanoylamino group -N(R^{c})C(O)(1-6C)alkyl in which R^{c} is hydrogen or (1-6C)alkyl; or **R⁶** is selected from (1-6C)alkyl or (1-6C)alkoxy, wherein the alkyl and the alkoxy groups are optionally substituted by one or more groups selected from cyano, fluoro, hydroxy, (1-6C)alkoxy, amino, mono(1-6C)alkylamino, di-[(1-6C)alkyl]amino, or a saturated or partially saturated 3 to 7 membered heterocyclic ring; and
**m** is 0, 1, 2 or 3;
   and when m is at least 2 then two substituents on adjacent carbon atoms in ring B may together represent a methylenedioxy group; and
**R¹⁰** and **R¹¹** are independently selected from hydrogen or (1-6C)alkyl;
and salts thereof, particularily pharmaceutically acceptable salts thereof.

Another particular embodiment is a group of compounds of the Formula Ia wherein:
**R¹** and **R²** are independently selected from hydrogen, (1-6C)alkyl or a 5 or 6 membered heteroaryl ring;
   wherein the (1-6C)alkyl group is optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or an alkanoylamino group N(R^{d})C(O)(1-6C)alkyl in which R^{d} is hydrogen or (1-6C)alkyl, or a saturated or partially saturated 3 to 7 membered heterocyclic ring or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or alkanoylamino groups are optionally substituted by one or more hydroxy groups;
   and wherein any heterocyclic and heteroaryl rings within R¹ and/or R² are optionally independently substituted by one or more of the following: (1-4C)alkyl, (1-4C)alkoxy, (I-4C)alkoxy(1-4C)alkyl, hydroxy, amino, mono(1-6C)alkylamino or di-[(1-6C)alkyl]amino or a saturated or partially saturated 3 to 7 membered heterocyclic ring or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
**R⁴** is selected from hydrogen, (1-6C)alkyl or (1-6C)alkoxy;
**A** represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, halo, (1-6C)alkyl or (1-6C)alkoxy;
**n** is 0, 1, 2 or 3;
**L** is attached meta or para on ring A with respect to the point of attachment of the ethynyl group and represents -CH₂C(O)N(R⁸), -N(R⁹)C(O)CH₂-, -N(R⁸)C(O)N(R⁹)-, -N(R⁸)C(O)O-, or -OC(O) N(R⁹)-, wherein R⁸ and R⁹ independently represent hydrogen or (1-6C)alkyl;
B represents a (3-7C)cycloalkyl ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
**R⁶** is selected from halo, cyano, a (3-7C)cycloalkyl ring, a saturated or partially saturated 3 to 7 membered heterocyclic ring or an alkanoylamino group -N(R^{c})C(O)(1-6C)alkyl in which R^{c} is hydrogen or (1-6C)alkyl; or **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; and
**m** is 0, 1, 2 or 3;
   and when B is a (3-7C)cycloalkyl ring, a saturated or partially saturated 3 to 7 membered heterocyclic ring or a saturated or partially saturated 8, 9 or 10 membered bicyclic group, the rings and the bicyclic group optionally bear 1 or 2 oxo or thioxo substituents; and
**R¹⁰** and **R¹¹** are independently selected from hydrogen or (1-6C)alkyl;
and salts thereof, particularily pharmaceutically acceptable salts thereof.

In this specification the generic term "alkyl" includes both straight-chain and branched-chain alkyl groups such as propyl, isopropyl and tert-butyl. However references to individual alkyl groups such as "propyl" are specific for the straight-chain version only, references to individual branched-chain alkyl groups such as "isopropyl" are specific for the branched-chain version only. An analogous convention applies to other generic terms, for example (1-6C)alkoxy includes methoxy, ethoxy and isopropoxy, (1-6C)alkylamino includes methylamino, isoproylamino and ethylamino, and di-[(1-6Calkyl]amino includes dimethylamino, diethylamino and N-methyl-N-isoproylamino. The generic term aryl refers to phenyl or naphthyl, particularily phenyl.

It is to be understood that, insofar as certain of the compounds of Formula I defined above may exist in optically active or racemic forms by virtue of one or more asymmetric carbon atoms, the invention includes in its definition any such optically active or racemic form which possesses the above-mentioned activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

Suitable values for the generic radicals referred to above include those set out below. Suitable 5 or 6 membered heteroaryl rings include, for example furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, 1,3,5-triazinyl or pyrazinyl. Particular 5 or 6 membered heteroaryl rings include imidazolyl, pyridyl, thiazolyl, thiadiazolyl, pyrimidinyl, isoxazolyl, pyrazolyl and isothiazolyl.

Suitable saturated or partially saturated 3 to 7 membered heterocyclic rings include, for example oxiranyl, oxetanyl, tetrahydrofuranyl, tetrahydropyranyl, 2,3-dihydro-1,3-thiazolyl, 1,3-thiazolidinyl, 1,3-oxazolidinyl, oxepanyl, pyrrolinyl, pyrrolidinyl, morpholinyl, thiamorpholinyl (perhydro-1,4-thiazinyl), (8-oxa-3-azabicyclo[3.2.1]octyl), (7-oxa-3-azabicyclo[3.1.1]heptyl), perhydroazepinyl, perhydrooxazepinyl, tetrahydro-1,4-thiazinyl, 1-oxotetrahydrothienyl, 1,1-dioxotetrahydro-1,4-thiazinyl, piperidinyl, homopiperidinyl, piperazinyl, homopiperazinyl, dihydropyridinyl, tetrahydropyridinyl, dihydropyrimidinyl or tetrahydropyrimidinyl, preferably tetrahydrofuranyl, tetrahydropyranyl, pyrrolidinyl, morpholinyl, 1,1-dioxotetrahydro-4H-1,4-thiazinyl, piperidinyl or piperazinyl, more preferably tetrahydrofuran-3-yl, tetrahydropyran-4-yl, pyrrolidin-3-yl, morpholino, 1,1-dioxotetrahydro-4H-1,4-thiazin-4-yl, piperidino, piperidin-4-yl or piperazin-1-yl. A suitable value for such a group which bears 1 or 2 oxo or thioxo substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl. The saturated or partially saturated 3 to 7 membered heterocyclic rings are optionally substituted by one or more (1-6C)alkyl groups and/or by one or more hydroxy.

Suitable 8, 9 or 10 membered bicyclic groups include thieno[2,3-b]furanyl, imidazolo[2,1-b]thiazolyl, dihydrocyclopentathiazolyl, tetrahydrocyclopenta[c]pyrazolyl, furo[3,2-b]furanyl, pyrrolopyrrole, thienopyrazolyl, thieno[2,3-b]thiophenyl, indolizinyl, indolyl, isoindolyl, 3H-indolyl, indolin-yl, benzo[b]furanyl, benzo[b]thiophenyl, IH-indazolyl, benzimidazolyl, benzthiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, chromanyl, isochromanyl, indenyl, naphthalenyl, 2,3-dihydro-1,4-benzodioxinyl, 1,3-benzodioxol-5-yl, decalin and norbornane. Particular 8, 9 or 10 membered bicyclic groups include thieno[2,3-b]furanyl, indolizinyl, indolyl, isoindolyl, 3H-indolyl, indolin-yl, benzo[b]furanyl, benzo[b]thiophenyl, 1H-indazolyl, benzimidazolyl, benzthiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, chromanyl, isochromanyl, indenyl, naphthalenyl, 2,3-dihydro-1,4-benzodioxinyl and 1,3-benzodioxol-5-yl.

The bicyclic group is optionally substituted by one or more groups R⁶ as hereinbefore defined.

The group A may particularly be attached to the ethynyl group via a carbon atom in the aryl group or in the 5 or 6 membered heteroaryl ring. The group B may particularly be attached to the group L via a carbon atom.

Suitable values for any of the substituents herein, for example the 'R' groups (R¹ to R¹¹) or for various groups within a A, B or L group include:
- for halo: fluoro, chloro, bromo and iodo;
- for (1-6C)alkyl:: methyl, ethyl, propyl, isopropyl and tert-butyl;
- for (1-6C)alkoxy:: methoxy, ethoxy, propoxy, isopropoxy and butoxy;
- for (1-6C)alkylsulfonyl:: methylsulfonyl and ethylsulfonyl;
- for (1-6C)alkylamino:: methylamino, ethylamino, propylamino, isopropylamino and butylamino;
- for di-[(1-6C)alkyl]amino:: dimethylamino, diethylamino, N-ethyl-N-methylamino and diisopropylamino;
- for (1-6C)alkoxycarbonyl:: methoxycarbonyl, ethoxycarbonyl, propoxycarbonyl and tert-butoxycarbonyl;
- for (2-6C)alkanoyl:: acetyl and propionyl;
- for (1-6C)alkanoylamino:: acetamido and propionamido;
- for amino-(1-6C)alkyl:: aminomethyl, 2-aminoethyl, 1-aminoethyl and 3-aminopropyl;
- for (1-6C)alkylamino-(1-6C)alkyl:: methylaminomethyl, ethylaminomethyl, 1-methylaminoethyl, 2-methylaminoethyl, 2-ethylaminoethyl and 3-ethylaminopropyl;
- for di-[(1-6C)alkyl]amino-(1-6C)alkyl:: dimethylaminomethyl, diethylaminomethyl, 1-dimethylaminoethyl, 2-dimethylaminoethyl and 3-dimethylaminopropyl;
- for halogeno-(1-6C)alkyl:: chloromethyl, 2-chloroethyl, 1-chloroethyl and 3-chloropropyl;
- for hydroxy-(1-6C)alkyl:: hydroxymethyl, 2-hydroxyethyl, 1-hydroxyethyl and 3-hydroxypropyl;
- for (1-6C)alkoxy-(1-6C)alkyl:: methoxymethyl, ethoxymethyl, 1-methoxyethyl, 2-methoxyethyl, 2-ethoxyethyl and 3-methoxypropyl;
- for cyano-(1-6C)alkyl:: cyanomethyl, 2-cyanoethyl, 1-cyanoethyl and 3-cyanopropyl;
- for (3-7C)cycloalkyl:: cyclopropyl, cyclobutyl, cyclopentyl, cyclohexyl and cycloheptyl;
- for (1-6C)alkoxy(1-6C)alkoxy:: methoxymethoxy, methoxyethoxy, methoxypropoxy, methoxybutoxy, methoxyhexoxy, ethoxyethoxy, ethoxypropoxy, ethoxybutoxy, propoxypropoxy and propoxybutoxy;
- for (1-6C)alkoxy(1-6C)alkoxy(1-6C)alkoxy:: methoxymethoxymethoxy, methoxyethoxyethoxy, methoxypropoxymethoxy, methoxybutoxyethoxy, methoxyhexoxymethoxy, ethoxyethoxyethoxy, ethoxypropoxyethoxy, ethoxybutoxymethoxy, propoxypropoxymethoxy and propoxybutoxymethoxy;
- for mono(1-6C)alkylcarbamoyl:: N-methylcarbamoyl, N-ethylcarbamoyl and N-propylcarbamoyl; and
- for di-[(1-6C)alkyl]carbamoyl:: N,N-dimethylcarbamoyl, N-ethyl-N-methylcarbamoyl and N,N-diethylcarbamoyl.

When in this specification reference is made to a (1-4C)alkyl group it is to be understood that such groups refer to alkyl groups containing up to 4 carbon atoms. A skilled person will realise that representative examples of such groups are those listed above under (1-4C)alkyl that contain up to 4 carbon atoms, such as methyl, ethyl, propyl, isopropyl, butyl and tert-butyl. Similarly, reference to a (1-3C)alkyl group refers to alkyl groups containing up to 3 carbon atoms such as methyl, ethyl, propyl and isopropyl. A similar convention is adopted for the other groups listed above such as (1-4C)alkoxy, (2-4C)alkenyl, (2-4C)alkynyl and (2-4C)alkanoyl.

It is to be understood that certain compounds of the formula I may exist in solvated as well as unsolvated forms such as, for example, hydrated forms. It is to be understood that the invention encompasses all such solvated forms which exhibit an inhibitory effect on a Tie2 receptor tyrosine kinase.

It is also to be understood that certain compounds of the formula I may exhibit polymorphism, and that the invention encompasses all such forms which exhibit an inhibitory effect on a Tie2 receptor tyrosine kinase.

It is also to be understood that the invention relates to all tautomeric forms of the compounds of the formula I forms which exhibit an inhibitory effect on a Tie2 receptor tyrosine kinase.

Whilst pharmaceutically-acceptable salts of compounds of the invention are preferred, other non-pharmaceutically-acceptable salts of compounds of the invention may also be useful, for example in the preparation of pharmaceutically-acceptable salts of compounds of the invention.
A suitable pharmaceutically acceptable salt of a compound of the formula I is, for example, an acid-addition salt of a compound of the formula I, for example an acid-addition salt with an inorganic or organic acid such as hydrochloric, hydrobromic, sulfuric, trifluoroacetic, citric or maleic acid; or, for example, a salt of a compound of the formula I which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or
tris-(2-hydroxyethyl)amine.

Particular novel compounds of the invention include, for example, compounds of the formula I, or salts, particularily pharmaceutically acceptable salts thereof, wherein, unless otherwise stated, each of R¹, R², R³, R⁴, R⁵, R⁶, A, B, L, m and n has any of the meanings defined hereinbefore or in paragraphs (a) to (dddd) hereinafter:-
(a) when n is 2 or 3, R⁵ may be the same or different;
(a') when m is 2 or 3, R⁶ may be the same or different;
(a") L is -CH₂C(O)N(R⁹)-, wherein R⁹ is hydrogen or (1-6C)alkyl (particularly R⁹ is hydrogen) ;
(b) L is -N(R⁸)C(O)CH₂-, wherein R⁸ is hydrogen or (1-6C)alkyl (particularly R⁸ is hydrogen);
(c) L is -N(R⁸)C(O)N(R⁹)-, wherein R⁸ and R⁹ are independently selected from hydrogen or (1-6C)alkyl (particularly R⁸ and R⁹ are both hydrogen or in another embodiment one of R⁸ and R⁹ is hydrogen and the other is methyl));
(d) L is -N(R⁸)C(O) O-, wherein R⁸ is hydrogen or (1-6C)alkyl (particularly R⁸ is hydrogen or in another embodiment R⁸ is methyl);
(e) L is -OC(O)-N(R⁹), wherein R⁹ is hydrogen or (1-6C)alkyl (particularly R⁹ is hydrogen or in another embodiment R⁹ is methyl);
(e') L is selected from -N(R⁸)C(O)N(R⁹)-, -N(R⁸)C(O)O- or -N(R⁸)C(O-CH₂- wherein R⁸ and R⁹ independently represent hydrogen or (1-6C)alkyl.
(f) Ring B-R⁶, where m is 1 or 2, is selected from 5-tert-butyl-1,3,4-thiadiazol-2-yl, 3-methylisoxazol-5-yl, 3-methoxyphenyl, 4-(trifluoromethyl)pyridin-2-yl, 2-(trifluoromethyl)phenyl, 3-(trifluoromethyl)phenyl, 4-(trifluoromethyl)phenyl, 2-fluoro-4-(trifluoromethyl)phenyl, 5-tert-butylisoxazol-3-yl, phenyl and 3-acetylaminophenyl;
(f') Ring B-R⁶, where m is 1 or 2, is selected from 3-acetylaminophenyl, 2-(trifluoromethyl)phenyl, 3-(trifluoromethyl)phenyl, 4-(trifluoromethyl)phenyl, 2-methoxyphenyl, 3-methoxyphenyl, 2-fluoro-5-(trifluoromethyl)phenyl, 3,4-dichloro-phenyl, 2-morpholin-4-ylphenyl, 5-tert-butyl-1,3,4-thiadiazol-2-yl, 3-methylisothiazol-5-yl, 3-methylisoxazol-5-yl, 5-tert-butylisoxazol-3-yl, 1-methyl-3-tert-butyl-pyrazol-5-yl, 1-methylpiperidin-4-yl, 1-propylpiperidin-4-yl, 4-(trifluoromethyl)pyrid-3-yl and 4-(trifluoromethyl)pyrid-2-yl;
(f') Ring B-R⁶, where m is 1 or 2, is selected from 2-oxo-piperidin-3-yl, 1-methylpiperidin-4-yl, 1-propylpiperidin-4-yl, 2,2-dimethyltetrahydropyran-4-yl, 2-fluoro-phenyl, 3-fluoro-phenyl, 4-fluoro-phenyl, 3,4-dichloro-phenyl, 2,5-difluoro-phenyl, 3,4-difluoro-phenyl, 4,5-difluoro-phenyl, 3-chloro-phenyl, 2-methoxyphenyl, 3-methoxyphenyl, 4-methoxyphenyl, 3-cyano-phenyl, 2-(trifluoromethyl)phenyl, 3-(trifluoromethyl)phenyl, 4-(trifluoromethyl)phenyl, 2-fluoro-5-(trifluoromethyl)phenyl, 3-acetylaminophenyl, 2-morpholin-4-ylphenyl, 5-methyl-1,3,4-thiadiazol-2-yl, 5-t-butyl-1,3,4-thiadiazol-2-yl, 5-triflluoromethyl-1,3,4-thiadiazol-2-yl, 5-cyclopropyl-1,3,4-thiadiazol-2-yl, 5-ethyl-1,3,4-thiadiazol-2-yl, 5-isopropyl-1,3,4-thiadiazol-2-yl, 5-ethylthio-1,3,4-thiadiazol-2-yl, 3-methylisoxazol-5-yl, 5-methylisoxazol-3-yl, 5-t-butyl-isoxazol-3-yl, 4-t-butyl-thiazol-2-yl, 3-methyl-isothiazol-5-yl, 1-methyl-3-t-butyl-pyrazol-5-yl, 1-methyl-3-cyclopropyl-pyrazol-5-yl, 1-methyl-3-isopropyl-pyrazol-5-yl, 1-t-butyl-pyrazol-4-yl, 1-t-butyl-3-cyclopropyl-pyrazol-5-yl, 1-ethyl-pyrazol-3-yl, 1-isopropyl-pyrazol-3-yl, 5-isopropyl-1,3,4-oxadiazol-2-yl, 4-trifluoro-pyrid-2-yl, 3-fluoro-5-(4-methylpiperazin-1-yl)phenyl, 4-(trifluoromethyl)pyrid-3-yl and 4-(trifluoromethyl)pyrid-2-yl;
(f") R¹ and R² are both hydrogen, R³ and R⁴ are both hydrogen, n is 0, L is -NHC(O)NH-, and ring B-R⁶, where m is 1 or 2, is selected from 3-acetylaminophenyl, 2-(trifluoromethyl)phenyl, 3-(trifluoromethyl)phenyl, 4-(trifluoromethyl)phenyl, 2-methoxyphenyl, 3-methoxyphenyl, 2-fluoro-5-(trifluoromethyl)phenyl,, 3,4-dichloro-phenyl, 2-morpholin-4-ylphenyl, 5-tert-butyl-1,3,4-thiadiazol-2-yl, 3-methylisothiazol-5-yl, 3-methylisoxazol-5-yl, 5-tert-butylisoxazol-3-yl, 1-methyl-3-tert-butyl-pyrazol-5-yl, 1-methylpiperidin-4-yl, 1-propylpiperidin-4-yl, 4-(trifluoromethyl)pyrid-3-yl and 4-(trifluoromethyl)pyrid-2-yl;
(g) R¹ and R² are independently selected from hydrogen, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more (for example 1 or 2) hydroxy groups;
   wherein the phenyl is optionally substituted by one or more groups (for example 1 or 2), which may be the same or different, selected from halo, (1-6C)alkyl, (1-6C)alkoxy, amino, mono(1-6C)alkylamino or di-[(1-6C)alkyl]amino, wherein the (1-6C)alkyl or (1-6C)alkoxy are optionally substituted by one or more groups (for example 1 or 2), which may be the same or different, selected from hydroxy, amino, mono(1-6C)alkylamino or di-[(1-6C)alkyl]amino;
   and wherein any heterocyclic and heteroaryl rings within R¹ and/or R² are optionally independently substituted by one or more groups (for example 1 or 2), which may be the same or different selected from (1-4C)alkyl, (1-4C)alkoxy, (1-4C)alkoxy(1-4C)alkyl, hydroxy, amino, mono(1-6C)alkylamino or di-[(1-6C)alkyl]amino, or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
(h) R¹ and R² are independently selected from hydrogen, (1-6C)alkanoyl, (1-6C)alkyl, (1-6C)alkoxycarbonyl or (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (g);
   and wherein any heterocyclic and heteroaryl rings within R¹ and/or R² are optionally independently substituted by one or more groups (for example 1 or 2), which may be the same or different, as hereinbefore defined in (g);
(i) R¹ and R² are independently selected from hydrogen, (1-6C)alkanoyl, (1-6C)alkyl or a 5 or 6 membered heteroaryl ring;
   wherein the (1-6C)alkyl and the (1-6C)alkanoyl groups are optionally substituted by one or more groups (for example 1 or 2), which may be the same or different, as hereinbefore defined in (g);
   and wherein any heterocyclic and heteroaryl rings within R¹ and/or R² are optionally independently substituted by one or more groups (for example 1 or 2), which may be the same or different, as hereinbefore defined in (g);
(j) R¹ is hydrogen and R² is selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalk-yl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (1-6C)cycloalkyl groups are optionally substituted by one or more groups (for example 1 or 2), which may be the same or different, as hereinbefore defined in (g);
   wherein the phenyl is optionally substituted by one or more groups (for example 1 or 2), which may be the same or different, as hereinbefore defined in (g);
   and wherein any heterocyclic and heteroaryl rings within R² are optionally independently substituted by one or more groups (for example 1 or 2), which may be the same or different, as hereinbefore defined in (g);
(k) R¹ hydrogen and R² is selected from hydrogen, (1-6C)alkanoyl, (1-6C)alkyl, (1-6C)alkoxycarbonyl or (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (g);
   and wherein any heterocyclic and heteroaryl rings within R² are optionally independently substituted by one or more groups (for example 1 or 2), which may be the same or different, as hereinbefore defined in (g);
(l) R¹ is hydrogen and R² is selected from hydrogen, (1-6C)alkanoyl, (1-6C)alkyl or a 5 or 6 membered heteroaryl ring;
   wherein the (1-6C)alkyl and the (1-6C)alkanoyl groups are optionally substituted by one or more groups (for example 1 or 2), which may be the same or different, as hereinbefore defined in (g);
   and wherein any heterocyclic and heteroaryl rings within R² are optionally independently substituted by one or more groups (for example 1 or 2), which may be the same or different, as hereinbefore defined in (g);
(m) R¹ and R² are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, selected from hydroxy, (1-6C)alkoxy, (1-6C)alkoxy(1-6C)alkoxy, amino, mono(1-6C)alkylamino, di-[(1-6C)alkyI]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy and (1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkly]carbamoyl and/or -N(R^{d})C(O)(1-6C)alkyl groups are optionally substituted by one or more (for example 1 or 2) hydroxy groups;
   wherein the phenyl is optionally substituted by one or more groups (for example 1 or 2), which may be the same or different, selected from halo, (1-6C)alkyl, (1-6C)alkoxy, amino, mono(1-6C)alkylamino or di-[(1-6C)alkyl]amino, wherein the (1-6C)alkyl or (1-6C)alkoxy are optionally substituted by one or more groups (for example 1 or 2), which may be the same or different, selected from hydroxy, amino, mono(1-6C)alkylamino or di-[(1-6C)alkyl]amino;
   and wherein any heterocyclic and heteroaryl rings within R¹ and/or R² are optionally independently substituted by one or more groups (for example 1 or 2), which may be the same or different, selected from (1-4C)alkyl, (1-4C)alkoxy, (1-4C)alkoxy(1-4C)alkyl, hydroxy, amino, mono(1-6C)alkylamino or di-[(1-6C)alkyl]amino or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
(n) R¹ and R² are independently selected from hydrogen, (1-6C)alkanoyl, (1-6C)alkyl, (1-6C)alkoxycarbonyl or (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (m);
   and wherein any heterocyclic and heteroaryl rings within R¹ and/or R² are optionally independently substituted by one or more groups (for example 1 or 2), which may be the same or different, as hereinbefore defined in (m);
(o) R¹ and R² are independently selected from hydrogen, (1-6C)alkanoyl, (1-6C)alkyl, or a 5 or 6 membered heteroaryl ring;
   wherein the (1-6C)alkyl and the (1-6C)alkanoyl groups are optionally substituted by one or more groups (for example 1 or 2), which may be the same or different, as hereinbefore defined in (m);
   and wherein any heterocyclic and heteroaryl rings within R¹ and/or R² are optionally independently substituted by one or more groups (for example 1 or 2), which may be the same or different, as hereinbefore defined in (m);
(p) R¹ is hydrogen and R² is selected from hydrogen, (1-6C)alkylsulfonyl, phenyl(CH₂)ᵤ- wherein u is 0,1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (m);
   wherein the phenyl is optionally substituted by one or more groups (for example 1 or 2), which may be the same or different, as hereinbefore defined in (m);
   and wherein any heterocyclic and heteroaryl rings within R² are optionally independently substituted by one or more groups (for example 1 or 2), which may be the same or different, as hereinbefore defined in (m);
(q) R¹ is hydrogen and R² is selected from hydrogen, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (m);
   and wherein any heterocyclic and heteroaryl rings within R² are optionally independently substituted by one or more groups (for example 1 or 2), which may be the same or different, as hereinbefore defined in (m);
(r) R¹ is hydrogen and R² is selected from hydrogen, (1-6C)alkanoyl, (1-6C)alkyl or a 5 or 6 membered heteroaryl ring;
   wherein the (1-6C)alkyl and the (1-6C)alkanoyl groups are optionally substituted by one or more groups (for example 1 or 2), which may be the same or different, as hereinbefore defined in (m);
   and wherein any heterocyclic and heteroaryl rings within R² are optionally independently substituted by one or more groups (for example 1 or 2), which may be the same or different, as hereinbefore defined in (m);
(s) R¹ is hydrogen and R² is selected from hydrogen, (1-6C)alkanoyl and (1-6C)alkyl;
   wherein the (1-6C)alkyl and the (1-6C)alkanoyl groups are optionally substituted by one or more groups (for example 1 or 2), which may be the same or different, as hereinbefore defined in (m);
   and wherein any heterocyclic and heteroaryl rings within R² are optionally independently substituted by one or more groups (for example 1 or 2), which may be the same or different, as hereinbefore defined in (m);
(t) R¹ is hydrogen and R² is selected from hydrogen, (1-6C)allcanoyl and (1-6C)alkyl,
   wherein the (1-6C)alkyl and the (1-6C)alkanoyl groups are optionally substituted by one or more groups (for example 1 or 2), which may be the same or different, selected from hydroxy, (1-4C)alkoxy, (1-4C)alkoxy(1-4C)alkoxy, amino, mono(1-3C)alkylamino, di(1-3C)alkylamino, carbamoyl or-N(R^{d})C(O)(1-3C)alkyl in which R^{d} is hydrogen or (1-3C)alkyl, or a saturated 5 or 6 membered heterocyclic ring, or a 5 or 6 membered heteroaryl ring, wherein the (1-4C)alkoxy and (1-4C)alkoxy(1-4C)alkoxy and the (1-3C)alkyl groups of the mono(1-3C)alkylamino, di-[(1-3C)alkyl]amino and/or -N(R^{d})C(O)(1-6C)alkyl groups are optionally substituted by one or more (for example 1 or 2) hydroxy groups;
   and wherein any heterocyclic and heteroaryl rings within R² are optionally independently substituted by one or more groups (for example 1 or 2), which may be the same or different, selected from (1-4C)alkyl, (1-4C)alkoxy, (1-4C)alkoxy(1-4C)alkyl, hydroxy, amino, mono(1-3C)alkylamino or di-[(1-3C)alkyl]amino, or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
(u) R¹ is hydrogen and R² is selected from hydrogen, (1-3C)alkanoyl and (1-3C)alkyl;
   wherein the (1-3C)alkyl and the (1-3C)alkanoyl groups are optionally substituted by one or more groups (for example 1 or 2), which may be the same or different, as hereinbefore defined in (t);
   and wherein any heterocyclic and heteroaryl rings within R² are optionally independently substituted by one or more groups (for example 1 or 2), which may be the same or different, as hereinbefore defined in (t);
(v) R¹ is hydrogen and R² is selected from hydrogen and (1-6C)alkyl (particularly (1-3C)alkyl);
   wherein the (1-6C)alkyl (particularly (1-3C)alkyl) group is optionally substituted by one or more groups (for example 1 or 2), which may be the same or different, as hereinbefore defined in (t);
   and wherein any heterocyclic and heteroaryl rings within R² are optionally independently substituted by one or more groups (for example 1 or 2), which may be the same or different, as hereinbefore defined in (t);
(w) R¹ is hydrogen and R² is (1-6C)alkyl (particularly (1-3C)alkyl),
   wherein the (1-6C)alkyl (particularly (1-3C)alkyl) group is optionally substituted by one or more groups (for example 1 or 2), which may be the same or different, as hereinbefore defined in (t);
   and wherein any heterocyclic and heteroaryl rings within R² are optionally independently substituted by one or more groups (for example 1 or 2), which may be the same or different, as hereinbefore defined in (t);
(w') R¹ and R² are both hydrogen or R¹ is hydrogen or (1-6C)alkyl and R² is (1-6C)alkyl
   wherein (1-6Calkyl) is optionally substituted by hydroxy, amino, mono(1-6C)alkylamino or di(1-6C)alkylamino, carbamoyl, (1-6C)alkoxy, (1-6C)alkoxy(1-6C)alkoxy, -N(R^{d})C(O)(1-6C)alkyl in which R^{d} is hydrogen or (1-6C)alkyl, a saturated or partially saturated 3 to 7 membered heterocyclic ring or a 5 or 6 membered heteroaryl ring;
   wherein the (1-6C)alkoxy, mono(1-6C)alkylamino and -N(R^{d})C(O)(1-6C)alkyl groups are optionally substituted by hydroxy;
   wherein the a saturated or partially saturated 3 to 7 membered heterocyclic ring is optionally substituted by (1-4C)alkyl or -C(O)CH₂Y wherein Y is selected from hydroxy or di(1-6C)alkylamino.
(w") R¹ and R² are both hydrogen or R¹ is hydrogen or (1-6C)alkyl and R² is (1-6C)alkyl
   wherein (1-6Calkyl) is optionally substituted by hydroxy, amino, mono(1-6C)alkylamino or di(1-6C)alkylamino, aryl (particularily phenyl) or a saturated or partially saturated 3 to 7 membered heterocyclic ring or a 5 or 6 membered heteroaryl ring;
   wherein the an aryl ring, saturated or partially saturated 3 to 7 membered heterocyclic ring or a 5 or 6 membered heteroaryl ring is optionally substituted by one or two groups independently selected from (1-4C)alkyl and (1-4C)alkoxy.
(w''') R¹ and R² are both hydrogen or R¹ is hydrogen or (1-6C)alkyl and R² is (1-6C)alkyl
   wherein (1-6Calkyl) is optionally substituted by hydroxy, amino, mono(1-6C)alkylamino or di(1-6C)alkylamino, carbamoyl, (1-6C)alkoxy, (1-6C)alkoxy(1-6C)alkoxy, -N(R^{d})C(O)(1-6C)alkyl in which R^{d} is hydrogen or (1-6C)alkyl, aryl (particularily phenyl), a saturated or partially saturated 3 to 7 membered heterocyclic ring or a 5 or 6 membered heteroaryl ring;
   wherein the (1-6C)alkoxy, mono(1-6C)alkylamino and -N(R^{d})C(O)(1-6C)alkyl groups are optionally substituted by hydroxy;
   wherein an aryl ring, a saturated or partially saturated 3 to 7 membered heterocyclic ring or a 5 or 6 membered heteroaryl ring is optionally substituted by (1-4C)alkyl, (1-4C)alkoxy or -C(O)CH₂Y wherein Y is selected from hydroxy or di(1-6C)alkylamino.
(w""') R¹ and R² are both hydrogen, R³ is amino and R⁴ is hydrogen;
(x) R¹ and R² are independently selected from hydrogen, methyl, ethyl, propyl, 2-hydroxyethyl, 3-hydroxypropyl, 2-methoxyethyl, 3-methoxypropyl, 2-aminoethyl, 3-aminopropyl, 2-(isopropylamino)ethyl, 3-(isopropylamino)propyl, 2-(dimethylamino)ethyl, 3-(dimethylamino)propyl, carbamoylmethyl, 2-carbamoylethyl, 3-carbamoylpropyl, 2-(2-methoxyethoxy)acetyl, 2-morpholin-4-ylethyl, 3-morpholin-4-ylpropyl, 2-pyrrolidin-1-ylethyl, 3-pyrrolidin-1-ylpropyl, 3-(4-methylpiperazin-1-yl)propyl, 3-piperidin-1-ylpropyl, 2-piperidin-1-ylethyl, 2-(1H-imidazol-4-yl)ethyl, 2-pyridin-2-ylethyl, 3-(1H-imidazol-1-yl)propyl, 2-pyridin-4-ylethyl, 2,4-dimethoxybenzyl and 5-tert-butylisoxazol-3-yl;
(y) R¹ is hydrogen and R² is selected from hydrogen, methyl, ethyl, propyl, 2-hydroxyethyl, 3-hydroxypropyl, 2-methoxyethyl, 3-methoxypropyl, 2-aminoethyl, 3-aminopropyl, 2-(isopropylamino)ethyl, 3-(isopropylamino)propyl, 2-(dimethylamino)ethyl, 3-(dimethylamino)propyl, carbamoylmethyl, 2-carbamoylethyl, 3-carbamoylpropyl, 2-(2-methoxyethoxy)acetyl, 2-morpholin-4-ylethyl, 3-morpholin-4-ylpropyl, 2-pyrrolidin-1-ylethyl, 3-pyrrolidin-1-ylpropyl, 3-(4-methylpiperazin-1-yl)propyl, 3-piperidin-1-ylpropyl, 2-piperidin-1-ylethyl, 2-(1H-imidazol-4-yl)ethyl, 2-pyridin-2-ylethyl, 3-(1H-imidazol-1-yl)propyl, 2-pyridin-4-ylethyl, 2,4-dimethoxybenzyl and 5-tert-butylisoxazol-3-yl;
(z) R¹ is hydrogen and R² is selected from hydrogen, methyl, ethyl, propyl, 3-(isopropylamino)propyl, 2-pyrrolidin-1-ylethyl, 5-tert-butylisoxazol-3-yl, 3-piperidin-1-ylpropyl, 2-morpholino-4-yl-ethyl, 2-pyrrolidin-1-ylethyl, 3-(dimethylamino)propyl, 2-hydroxyethyl and 2-piperidin-1-ylethyl;
(z') R¹ is hydrogen or methyl and R² is selected from hydrogen, methyl, 2-hydroxyethyl, 2-aminoethyl, 4-aminobutyl, 3-(N-isopropylamino)propyl, 2-(dimethylamino)ethyl, 4-(dimethylamino)butyl, 2-pyrolidin-1-ylethyl, 3-pyrolidin-1-ylpropyl, 5-t-butyl-isoxazol-3-yl, 2-morpholin-4-ylethyl, 3-morpholin-4-ylpropyl, 2-piperidin-1-ylethyl, 3-piperidin-1-ylpropyl, 3-(4-methyl-piperazin-1-yl)propyl and 2,4-dimethoxybenzyl;
(y") R¹ is hydrogen or methyl and R² is selected from hydrogen, methyl, 2-hydroxyethyl, 2-methoxyethyl, 3-methoxypropyl, 2-(2-hydroxyethoxy)ethyl, 2-aminoethyl, 3-aminopropyl, 4-aminobutyl, 2-(isopropylamino)ethyl, 3-(isopropylamino)propyl, 2-(dimethylamino)ethyl, 3-(dimethylamino)propyl, 4-(dimethylamino)butyl, 2-(dimethylamino)-1-methylethyl, carbamoylmethyl, 2-carbamoylethyl, 2-(2-methoxyethoxy)acetyl, 2-(2-hydroxyacetamido)ethyl, 3-[N-(2-hydroxyethyl)amino]propyl, 2-morpholin-4-ylethyl, 3-morpholin-4-ylpropyl, 2-[(1-methyl-2-morpholin-4-ylethyl), 2-pyrrolidin-1-ylethyl, 3-pyrrolidin-1-ylpropyl, 1-glycoloylpyrrolidin-2-yl)methyl, 1-(N,N-dimethylglycyl)pyrrolidin-2-yl, 2-piperazin-1-ylethyl, 3-piperazin-1-ylpropyl, 3-(4-methylpiperazin-1-yl)propyl, 3-piperidin-1-ylpropyl, 2-(1H-imidazol-4-yl)ethyl, 2-pyridin-2-ylethyl, 3-(1H-imidazol-1-yl)propyl, 5-t-butyl-isoxazol-3-yl, 2-pyridin-4-ylethyl and and 2,4-dimethoxybenzyl;
(z") R¹ is hydrogen and R² is selected from 2-morpholin-4-ylethyl, 3-morpholin-4-ylpropyl, 3-piperidin-1-ylpropyl, 2-piperidin-1-ylethyl, 2-pyrrolidin-1-ylethyl, 4-methyl-piperazin-1-ylpropyl and 3-pyrrolidin-1-ylpropyl;
(z'") R¹ is hydrogen and R² is selected from 2-morpholin-4-ylethyl, 3-morpholin-4-ylpropyl, 3-piperidin-1-ylpropyl, 2-piperidin-1-ylethyl, 2-pyrrolidin-1-ylethyl, 3-pyrrolidin-1-ylpropyl and 4-methyl-piperazin-1-yl;
(aa) R¹ and R² are both hydrogen;
(bb) R¹ and R² are both (1-6C)alkyl (particularly (1-3C)alkyl);
(cc) R¹ is hydrogen and R² is methyl;
(dd) R³ is selected from hydrogen, (1-3C)alkyl or (1-3C)alkoxy,
   wherein the (1-3C)alkyl and the (1-3C)alkoxy groups are optionally substituted by one or more groups (for example 1 or 2), which may be the same or different, selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more groups (for example 1 or 2), which may be the same or different, selected from (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or R³ represents a group -NR¹R² as defined above;
(ee) R³ is selected from hydrogen or (1-6C)alkyl,
   wherein the (1-6C)alkyl group is optionally substituted by one or more groups (for example 1 or 2), which may be the same or different, selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more groups (for example 1 or 2), which may be the same or different, selected from (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or R³ represents a group -NR¹R² as defined above;
(ff) R³ is selected from hydrogen and a group -NR¹R² as defined above (particularly -NH₂);
(gg) R³ is hydrogen;
(hh) R³ is a group -NR¹R² as defined above (particularly -NH₂);
(ii) R⁴ is independently selected from hydrogen and (1-6C)alkyl (particularly (1-3C)alkyl);
(jj) R⁴ is hydrogen;
(kk) R³ and R⁴ are both hydrogen;
(ll) R³ is a group NR¹R² as defined above (particularly -NH₂) and R⁴ is hydrogen;
(mm) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl;
(mm') A is selected from phenyl, oxazolyl, imidazolyl, pyrrolyl, pyrazolyl, thiazolyl, isothiazolyl, thiadiazolyl, pyridyl, pyridazinyl, pyrazinyl and pyrimidyl.
(nn) A is selected from phenyl, thiazolyl, thiadiazolyl, pyridyl and pyrimidinyl;
(oo) A is phenyl;
(oo') A is phenyl or pyridyl
(oo'') A is phenyl or pyridyl, wherein the nitrogen in the pyridyl ring is in the 3-position relateive to the alkyne bond.
(pp) A is phenyl and n is 0;
(pp') A is phenyl or pyridyl and n is 0;
(qq) n is 0, 1 or 2 (particularly 0 or 1, more particularly 0);
(rr) n is 1 or 2 and R⁵ is independently selected from halo, (1-6C)alkoxy and (1-6C)alkyl, wherein the (1-6C)alkyl and the (1-6C)alkoxy groups are optionally substituted by cyano or one or more fluoro;
(rr) n is 1 or 2 and R⁵ is independently selected from cyano, halo, (1-6C)alkoxy and (1-6C)alkyl, wherein the (1-6C)alkyl and the (1-6C)alkoxy groups are optionally substituted by cyano or one or more fluoro;
(rr') n is 0 or 1 and when n is 1, R⁵ is (1-4C)alkyl (particularily methyl);
(ss) n is 1 or 2 and R⁵ is independently selected from cyclopropyl and (1-6C)alkyl, wherein the (1-6C)alkyl groups are optionally substituted by cyano or one or more fluoro;
(tt) L is attached meta on ring A with respect to the point of attachment of the ethynyl group;
(uu) L is attached meta on ring A with respect to the point of attachment of the ethynyl group and represents -N(R⁸)C(0)C(R^{a}R^{b})-, -N(R⁸)C(O)N(R⁹)- or -N(R⁸)C(O)O-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
(vv) L is attached meta on ring A with respect to the point of attachment of the ethynyl group and represents -N(R⁸)C(O)C(R^{a}R^{b})-, -N(R⁸)C(O)N(R⁹)- or -N(R⁸)C(O) O-, wherein R⁸ and R⁹ independently represent hydrogen or (1-3C)alkyl and wherein R^{a} and R^{b} independently represent hydrogen or (1-3C)alkyl;
(vv') L is attached para on ring A with respect to the point of attachment of the ethynyl group;
(ww) L is attached para on ring A with respect to the point of attachment of the ethynyl group and represents -N(R⁸)C(O) O- or -N(R⁸)C(O)N(R⁹)-, wherein R⁸ and R⁹ independently represent hydrogen or (1-6C)alkyl;
(xx) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl (particularly phenyl, thiazolyl, thiadiazolyl, pyridyl and pyrimidinyl);
   n is 0; and
   L is attached meta on ring A with respect to the point of attachment of the ethynyl group and represents -N(R⁸)C(O)C(R^{a}R^{b})-, -N(R⁸)C(O)N(R⁹)- or -N(R⁸)C(O)O-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
(yy) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl (particularly phenyl, thiazolyl, thiadiazolyl, pyridyl and pyrimidinyl);
   n is 0; and
   L is attached meta on ring A with respect to the point of attachment of the ethynyl group and represents -N(R⁸)C(O)C(R^{a}R^{b})-, -N(R⁸)C(O)N(R⁹)- or -N(R⁸)C(O) O-, wherein R⁸ and R⁹ independently represent hydrogen or (1-3C)alkyl and wherein R^{a} and R^{b} independently represent hydrogen or (1-3C)alkyl;
(zz) When B is a (3-7C)cycloalkyl ring then B is selected grom cyclopropyl, cyclobutyl, cyclopentyl and cyclohexyl;
(zz') When B is a saturated or partially saturated 3 to 7 membered heterocyclic ring then B is selected from oxetanyl, azetidinyl, thietanyl, pyrrolidinyl, , morpholinyl, 1,3-dioxolanyl, tetrahydrofuranyl, piperidyl, piperazinyl, thiomorpholinyl, tetrahydropyranyl, homopiperazinyl, pyrrolinyl, imidazolinyl, pyrazolinyl, pyranyl, tetrahydropyridinyl, 1,2,4-oxadiazolyl and dihydrothiopyranyl;
(zz") When B is an 8, 9 or 10 membered bicyclic group which optionally contains 1,2,3 or 4 heteroatoms independently selected from N, O and S and which is saturated, partially saturated or aromatic then B is selected from 2,3-dihydro-1H-indenyl, benzodioxinyl, 1,2,3,4-tetrahydronaphthalenyl, 1,2,3,4-tetrahydropentalene, benzofuranyl, 2,3-dihydrobenzofuranyl, benzimidazolyl, benzthienyl, benzthiazolyl, benzisothiazolyl, benzoxazolyl, benzisoxazolyl, pyridoimidazolyl, pyrimidoimidazolyl, quinolinyl, isoquinolinyl, quinoxalinyl, quinazolinyl, phthalazinyl, cinnolinyl, indolyl, and naphthyridinyl.
   or B is a group of the formula: 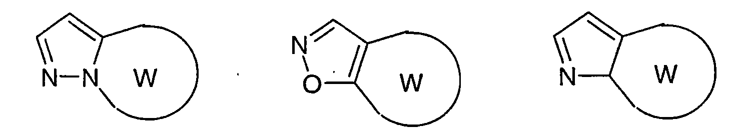 wherein W is a 5-7 membered ring (including the bridging atoms), said W ring comprising carbon atoms or optionally further heteroatoms independently selected from oxygen, nitrogen and sulphur, wherein said bicyclic ring contains no more that 4 heteroatoms in total. Examples of such rings include: pyrazolo[1,5-a]pyridinyl, pyrazolo[1,5-c]pyrimidinyl, pyrazolo[1,5-a][1,3,5]triazinyl, 4,5-dihydropyrazolo[1,5-a]pyridinyl, 4H-pyrazolo[5,1-c][1,4]thiazinyl, 4H-pyrazolo[5,1-c][1,4]oxazinyl, 1,2-benzisoxazolyl, isoxazolo[5,4-b]pyridinyl, isoxazolo[5,4-d]pyrimidinyl, 4H-thiopyrano[3,4-d]isoxazolyl, 4H-pyrano[3,4-d]isoxazolyl, 7aH-indolyl, 7aH-pyrrolo[2,3-b]pyridinyl, 7aH-pyrrolo[2,3-b]pyrimidinyl, 4,7a-dihydrothiopyrano[4,3-b]pyrrolyl and 4,7a-dihydropyrano[4,3-b]pyrrolyl.
(zz''') B is selected from a saturated or partially saturated 4 to 6 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
(aaa) B is selected from a saturated or partially saturated 4 to 6 membered heterocyclic ring, an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
(aaa') B is selected from a saturated or partially saturated 4 to 6 membered heterocyclic ring, an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
(aaa") B is selected from a saturated or partially saturated 4 to 6 membered heterocyclic ring, or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
(bbb) B is selected from cyclopentyl, cyclohexyl, piperidinyl, tetrahydropyranyl, phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazinyl, 2,3-dihydro-1,4-benzodioxinyl and 1,3-benzodioxol-5-yl;
(ccc) B is selected from piperidinyl, phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazinyl, 2,3-dihydro-1,4-benzodioxinyl and 1,3-benzodioxol-5-yl;
(ccc') B is selected from phenyl, 2,3-di-hydro-indenyl, piperidinyl, pyridyl, pyrazolyl, isothiazolyl, thiadiazolyl, isoxazolyl or benzodioxinyl;
(ccc") B is selected from phenyl, piperidinyl, pyridyl, pyrazolyl, isothiazolyl, thiadiazolyl, isoxazolyl, benzodioxolyl or tetrahydropyranyl.
(ccc''') B is selected from phenyl, 2,3-di-hydro-indenyl, piperidinyl, pyridyl, pyrazolyl, isothiazolyl, thiadiazolyl, isoxazolyl, benzodioxinyl, benzodioxolyl or tetrahydropyranyl.
(ddd) B is selected from piperidinyl, phenyl, isoxazolyl, isothiazolyl, thiadiazolyl, pyrazolyl and pyridyl;
(eee) B is selected from phenyl, pyrazolyl, thiadiazolyl and isoxazolyl;
(eee') Bis selected from isoxazolyl, thiadiazolyl and pyrazolyl;
(eee") Bis selected from isoxazolyl and pyrazolyl;
(fff) B is phenyl;
(ggg) B is isoxazolyl;
(hhh) B is pyrazolyl;
(iii) B is a saturated or partially saturated 3 to 7 (particularly 4 to 6) membered heterocyclic ring that contains one or two heteroatoms (particularly one heteroatom) selected from oxygen and nitrogen;
(jjj) B is a 8, 9 or 10 membered bicyclic group which optionally contains 1, 2 or 3 (particularly 1 or 2) heteroatoms independently selected from N and O and which is saturated, partially saturated or aromatic;
(kkk) A is phenyl;
   n is 0; and
   B is selected from a saturated or partially saturated 4 to 6 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl or a 8, 9 or 10 membered bicyclic group which optionally contains 1, 2, 3 or 4 heteroatoms independently selected from N, O and S and which is saturated, partially saturated or aromatic;
(lll) A is phenyl;
   n is 0; and
   B is selected from phenyl, pyrazolyl, thiadiazolyl and isoxazolyl;
(mmm) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl (particularly phenyl, thiazolyl, thiadiazolyl, pyridyl and pyrimidinyl);
   n is 0;
   L is attached meta on ring A with respect to the point of attachment of the ethynyl group and represents -N(R⁸)C(O)C(R^{a}R^{b})-, -N(R⁸)C(O)N(R⁹)- or -N(R⁸)C(O) O-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl; and
   B is selected from phenyl, pyrazolyl, thiadiazolyl and isoxazolyl;
(nnn) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl (particularly phenyl, thiazolyl, thiadiazolyl, pyridyl and pyrimidinyl);
   n is 0;
   L is attached meta on ring A with respect to the point of attachment of the ethynyl group and represents -N(R⁸)C(O)C(R^{a}R^{b})-, -N(R⁸)C(O)N(R⁹)- or -N(R⁸)C(O)O-, wherein R⁸ and R⁹ independently represent hydrogen or (1-3C)alkyl and wherein R^{a} and R^{b} independently represent hydrogen or (1-3C)alkyl; and
   B is selected from phenyl, pyrazolyl, thiadiazolyl and isoxazolyl;
(ooo) m is 0, 1 or 2 (particularly 1 or 2);
(ppp) R⁶ is selected from halo, cyano, a (3-4C)cycloalkyl ring, a saturated or partially saturated 3 to 7 membered heterocyclic ring or -N(R^{c})C(O)(1-6C)alkyl in which R^{c} is hydrogen or (1-6C)alkyl; or R⁶ is selected from (1-6C)alkyl or (1-6C)alkoxy, wherein the (1-6C)alkyl and the (1-6C)alkoxy groups are optionally substituted by one or more groups (for example 1 or 2), which may be the same or different, selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
(qqq) R⁶ is selected from halo, cyano, a saturated or partially saturated 3 to 7 membered heterocyclic ring or an -N(R^{c})C(O)(1-6C)alkyl in which R^{c} is hydrogen or (1-6C)alkyl; or R⁶ is selected from (1-6C)alkyl or (1-6C)alkoxy, wherein the (1-6C)alkyl and the (1-6C)alkoxy groups are optionally substituted by one or more groups (for example 1 or 2), which may be the same or different, selected from cyano, fluoro, hydroxy and amino (particularly fluoro);
(rrr) R⁶ is selected from halo, cyano, a saturated or partially saturated 3 to 7 membered heterocyclic ring or -N(R^{c})C(O)(1-6C)alkyl in which R^{c} is hydrogen or (1-3C)alkyl; or R⁶ is selected from (1-4C)alkyl or (1-4C)alkoxy, wherein the (1-4C)alkyl and the (1-4C)alkoxy groups are optionally substituted by one or more groups (for example 1 or 2), which may be the same or different, selected from cyano, fluoro, hydroxy and amino (particularly fluoro);
(sss) R⁶ is selected from fluoro, chloro, cyano, acetylamino, methyl, ethyl, propyl, isopropyl, butyl, tert-butyl, trifluoromethyl, cyclopropyl, cyclopropylmethyl, methoxy, ethoxy, propoxy, butoxy and morpholin-4-yl;
(ttt) R⁶ is selected from fluoro, chloro, acetylamino, methyl, propyl, tert-butyl, trifluoromethyl, cyclopropyl, cyclopropylmethyl, methoxy and morpholin-4-yl;
(ttt') R⁶ is independently selected from halo, (1-6C)alky(optionally substituted by up to three groups independently selected from halo, particularily fluoro), (1-6C)alkoxy, -N(R^{c})C(O)(1-6C)alkyl wherein R^{c} is hydrogen or (1-6C)alkyl (particularily (1-4C)alkyl) or a saturated 3 to 7 membered heterocyclic ring (particularily morpholino).
(ttt") R⁶ is independently selected from halo, trifluoromethyl, methyl, tert-butyl, methoxy, acetylamino or morpholino.
(ttt"') R⁶ is independently selected from halo, cyano, oxo, (3-7C)cycloalkyl, a saturated 3 to 7 membered heterocyclic ring (optionally substituted by (1-4C)alkyl), -N(R^{c})C(O)(1-6C)alkyl wherein R^{c} is hydrogen or (1-6C)alkyl (particularily (1-4C)alkyl), (1-6C)alkyl (optionally substituted by up to three groups independently selected from halo, particularily fluoro) or (1-6C)alkoxy.
(ttt'''') R⁶ is independently selected from halo, trifluoromethyl, cyano, methyl, isopropyl, tert-butyl, methoxy, acetylamino, oxo, cyclopropyl or 4-methyl-piperazin-1-yl.
(ttt''''') R⁶ is independently selected from (1-6C)alky or a (3-7C)cycloalkyl, particularily independently selected from (1-4C)alkyl or a (3-5C)cycloalkyl ring, more particularily independently selected from methyl, ethyl, propyl, isopropyl, sec-butyl, tert-butyl, cyclopropyl or cyclobutyl.
(uuu) B is selected from cyclopentyl, cyclohexyl, piperidinyl, tetrahydropyranyl, phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazinyl, 2,3-dihydro-1,4-benzodioxinyl and 1,3-benzodioxol-5-yl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, cyclopropylmethyl, methoxy, ethoxy, propoxy, butoxy and morpholin-4-yl;
(vvv) B is selected from phenyl, isoxazolyl, isothiazolyl, thiadiazolyl, pyrazolyl and pyridyl;
   m is 1 or 2; and
   R⁶ is independently selected from halo, cyano, a (3-4C)cycloalkyl ring, a saturated or partially saturated 3 to 7 membered heterocyclic ring or -N(R^{c})C(O)(1-6C)alkyl in which R^{c} is hydrogen or (1-6C)alkyl; or R⁶ is selected from (1-6C)alkyl or (1-6C)alkoxy, wherein the (1-6C)alkyl and the (1-6C)alkoxy groups are optionally substituted by one or more groups (for example 1 or 2), which may be the same or different, selected from cyano, fluoro, hydroxy and amino (particularly fluoro);
(www) B is selected from phenyl, isoxazolyl, isothiazolyl, thiadiazolyl, pyrazolyl and pyridyl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, cyclopropylmethyl, methoxy, ethoxy, propoxy, butoxy and morpholin-4-yl;
(xxx) B is phenyl;
   m is 1 or 2; and
   R⁶ is independently selected from fluoro, chloro, cyano, acetylamino, trifluoromethyl, cyclopropyl, cyclopropylmethyl, methoxy, ethoxy, propoxy, butoxy and morpholin-4-yl;
(yyy) B is phenyl;
   m is 1 or 2; and
   R⁶ is independently selected from fluoro and trifluoromethyl;
(zzz) B is isoxazolyl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, cyclopropylmethyl, methoxy, ethoxy, propoxy and butoxy;
(aaaa) B is isoxazolyl;
   m is 1 or 2; and
   R⁶ is independently selected from methyl, ethyl, propyl, isopropyl, butyl, tert-butyl (particularly methyl and tert-butyl, more particularly tert-butyl);
(bbbb) B is pyrazolyl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, cyclopropylmethyl, methoxy, ethoxy, propoxy and butoxy;
(bbbb') B is pyrazolyl;
   m is 1 or 2; and
   R⁶ is independently selected from methyl, ethyl, propyl, isopropyl, butyl, tert-butyl (particularly methyl and tert-butyl, more particularly tert-butyl);
(bbbb") B is thiadiazolyl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, methoxy, ethoxy, propoxy and butoxy;
(bbbb''')B is thiadiazolyl;
   m is 1 or 2; and
   R⁶ is independently selected from methyl, ethyl, propyl, isopropyl, butyl, tert-butyl (particularly methyl and tert-butyl, more particularly tert-butyl);
(cccc) Ring B-R⁶ wherein m is 0, 1 or 2 is selected from cyclopropyl, cyclobutyl, cyclopentyl, cyclohexyl, cycloheptyl, phenyl, 2-fluorophenyl, 3-fluorophenyl, 4-fluorophenyl, 2,5-difluorophenyl, 3,4-difluorophenyl, 3,5-difluorophenyl, 2-(trifluoromethyl)phenyl, 3-(trifluoromethyl)phenyl, 4-(trifluoromethyl)phenyl, 2-fluoro-5-(trifluoromethyl)phenyl, 2-chlorophenyl, 3-chlorophenyl, 4-chlorophenyl, 2,5-dichlorophenyl, 3,4-dichlorophenyl, 3,5-dichlorophenyl, 2-methoxyphenyl, 3-methoxyphenyl, 4-methoxyphenyl, 2-cyanophenyl, 3-cyanophenyl, 4-cyanophenyl, 2-acetamidophenyl, 3-acetamidophenyl, 4-acetamidophenyl, 5-tert-butyl-1,3,4-thiadizol-2-yl, 5-methyl-1,3,4-thiadizol-2-yl, 5-cyclopropyl-1,3,4-thiadizol-2-yl, 1-methyl-3-cyclopropyl-pyrazol-5-yl, 1-tert-butyl-3-cyclopropyl-pyrazol-5-yl, 3-methylisothiazol-5-yl, 3-methylisoxazol-5-yl, 5-methylisoxazol-3-yl, 5-tert-butylisoxazol-3-yl, 4-(trifluoromethyl)pyridin-2-yl, 2-oxopiperidin-3-yl, 2,2-dimethyltetrahydro-2H-pyran-4-yl, 2,3-dihydro-1,4-benzodioxinyl, 1,3-benzodioxol-5-yl, 2-morpholin-4-ylphenyl, 3-morpholin-4-ylphenyl, 4-morpholin-4-ylphenyl, 1-methylpiperidin-4-yl, 1-ethylpiperidin-4-yl and 1-propylpiperidin-4-yl; and
(dddd) Ring B-R⁶ wherein m is 1 or 2 is selected from 2-fluorophenyl, 3-fluorophenyl, 4-fluorophenyl, 2,5-difluorophenyl, 3,4-difluorophenyl, 3,5-difluorophenyl, 2-(trifluoromethyl)phenyl, 3-(trifluoromethyl)phenyl, 4-(trifluoromethyl)phenyl, 2-fluoro-5-(trifluoromethyl)phenyl, 2-chlorophenyl, 3-chlorophenyl, 4-chlorophenyl, 2,5-dichlorophenyl, 3,4-dichlorophenyl, 3,5-dichlorophenyl, 2-methoxyphenyl, 3-methoxyphenyl, 4-methoxyphenyl, 2-cyanophenyl, 3-cyanophenyl, 4-cyanophenyl, 2-acetamidophenyl, 3-acetamidophenyl, 4-acetamidophenyl, 5-tert-butyl-1,3,4-thiadizol-2-yl, 5-methyl-1,3,4-thiadizol-2-yl, 5-cyclopropyl-1,3,4-thiadizol-2-yl, 3-cyclopropyl-pyrazol-5-yl, 1-tert-butyl-3-cyclopropyl-pyrazol-5-yl, 3-methylisothiazol-5-yl, 3-methylisoxazol-5-yl, 5-methylisoxazol-3-yl, 5-tert-butylisoxazol-3-yl, 4-(trifluoromethyl)pyridin-2-yl, 2-oxopiperidin-3-yl, 2,2-dimethyltetrahydro-2H-pyran-4-yl, 2-morpholin-4-ylphenyl, 3-morpholin-4-ylphenyl, 4-morpholin-4-ylphenyl, 1-methylpiperidin-4-yl, 1-ethylpiperidin-4-yl and 1-propylpiperidin-4-yl.

A particular embodiment of the compounds of the Formula I is a compound of the Formula Ib: 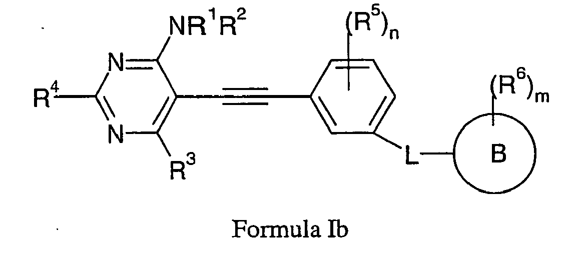 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is O, 1, 2, 3, 4, 5 or 6 , or a 5 or 6 membered heteroaryl ring, or **R¹ and R²** together with the nitrogen atom to which they are attached represent a saturated or partially saturated 3 to 7 membered heterocyclic ring optionally containing another heteroatom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl)]carbamoyl and/or -N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino or di-[(1-6C)alkyl]amino, or a saturated or partially saturated 3 to 7 membered heterocyclic ring, or -C(C)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³** is selected from hydrogen, (1-6C)alkyl or (1-6C)alkoxy, wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or **R³** represents a group **-NR¹R²** as defined above;
**R⁴** is selected from hydrogen, (1-6C)alkyl or (1-6C)alkoxy;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** represents -C(R^{a}R^{b})C(O)N(R⁹)-, -N(R⁸)C(O)C(R^{a}R^{b})-, -N(R⁸)C(O)N(R⁹)-, N(R⁸)C(O) O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl ;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
R⁶ is selected from one of the following 2 groups:
   (i) **R⁶** is selected from halo, cyano, oxo, a (3-7C)cycloalkyl ring, a saturated or partially saturated 3 to 7 membered heterocyclic ring and -N(R^{c})C(O)(1-6C)alkyl in which R^{c} is hydrogen or (1-6C)alkyl;
      or **R⁶** is selected from (1-6C)alkyl, -S(O)ₚ-(1-6C)alkyl wherein p is 0, 1 or 2, or (1-6C)alkoxy,
      wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and
      wherein the (3-7C)cycloalkyl ring and saturated or partially saturated 3 to 7 membered heterocyclic ring are optionally independently substituted by one or more groups selected from (1-6C)alkyl;
   (ii) **R⁶** is selected from halo, cyano, a (3-7C)cycloalkyl ring, a saturated or partially saturated 3 to 7 membered heterocyclic ring or -N(R^{c})C(O)(1-6C)alkyl in which R^{c} is hydrogen or (1-6C)alkyl; or
      **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
   **m** is 0, 1, 2 or 3;
   and when B is a (3-7C)cycloalkyl ring or a saturated or partially saturated 3 to 7 membered heterocyclic ring or a saturated or partially saturated 8, 9 or 10 membered bicyclic group, the rings and bicyclic group optionally bear 1 or 2 oxo or thioxo substituents; and salts thereof, particularily pharmaceutically acceptable salts thereof.

Another particular embodiment of the compounds of the Formula I is a compound of the Formula Ic: 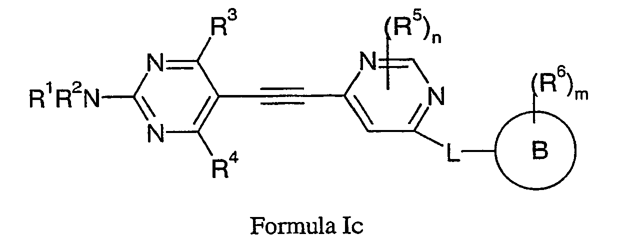 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6 , or a 5 or 6 membered heteroaryl ring, or **R¹ and R²** together with the nitrogen atom to which they are attached represent a saturated or partially saturated 3 to 7 membered heterocyclic ring optionally containing another heteroatom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (104C)alkoxy(1-4C)alkyl, hydroxy, amino, mono(1-6C)alkylanuno or di-[(1-6C)alkyl]amino, or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³** is selected from hydrogen, (1-6C)alkyl or (1-6C)alkoxy, wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or **R³** represents a group **-NR¹R²** as defined above;
**R⁴** is selected from hydrogen, (1-6C)alkyl or (1-6C)alkoxy;
**A** represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1 or 2;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O) O-, or -OC(O)-N(R⁹)-, wherein R⁸ and R⁹ independently represent hydrogen or (1-6C)alkyl and wherein R^{a} and R^{b} independently represent hydrogenor (1-6C)alkyl or R^{a} and R^{b} together with the carbon atom to which they are attached represent (3-6C)cycloalkyl ;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
R⁶ is selected from one of the following 2 groups:
   (i) **R⁶** is selected from halo, cyano, oxo, a (3-7C)cycloalkyl ring, a saturated or partially saturated 3 to 7 membered heterocyclic ring and -N(R^{c})C(O)(1-6C)alkyl in which R^{c} is hydrogen or (1-6C)alkyl;
      or **R⁶** is selected from (1-6C)alkyl, -S(O)ₚ-(1-6C)alkyl wherein p is 0, 1 or 2, or (1-6C)alkoxy,
      wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and
      wherein the (3-7C)cycloalkyl ring and saturated or partially saturated 3 to 7 membered heterocyclic ring are optionally independently substituted by one or more groups selected from (1-6C)alkyl;
   (ii) **R⁶** is selected from halo, cyano, a (3-7C)cycloalkyl ring, a saturated or partially saturated 3 to 7 membered heterocyclic ring or -N(R^{c})C(O)(1-6C)alkyl in which R^{c} is hydrogen or (1-6C)alkyl; or
      **R⁶** is selected from (1-6C)alkyl or(1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
   **m** is 0,1, 2 or 3;
   and when B is a (3-7C)cycloalkyl ring or a saturated or partially saturated 3 to 7 membered heterocyclic ring or a saturated or partially saturated 8, 9 or 10 membered bicyclic group, the rings and bicyclic group optionally bear 1 or 2 oxo or thioxo substituents; and salts thereof, particularily pharmaceutically acceptable salts thereof.

Another particular embodiment of the compounds of the Formula I is a compound of the Formula Id: 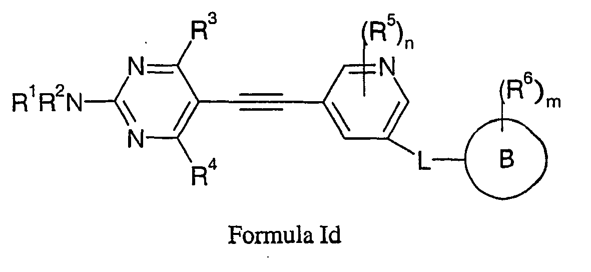 wherein:
**R¹** and **R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6 , or a 5 or 6 membered heteroaryl ring, or **R¹** and **R²** together with the nitrogen atom to which they are attached represent a saturated or partially saturated 3 to 7 membered heterocyclic ring optionally containing another heteroatom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or -N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (104C)alkoxy(1-4C)alkyl, hydroxy, amino, mono(1-6C)alkylamino or di-[(1-6C)alkyl]amino, or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³** is selected from hydrogen, (1-6C)alkyl or (1-6C)alkoxy, wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or **R³** represents a group **-NR¹R²** as defined above;
**R⁴** is selected from hydrogen, (1-6C)alkyl or (1-6C)alkoxy;
**A** represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O) O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl ;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
R⁶ is selected from one of the following 2 groups:
   (i) **R⁶** is selected from halo, cyano, oxo, a (3-7C)cycloalkyl ring, a saturated or partially saturated 3 to 7 membered heterocyclic ring and -N(R^{c})C(O)(1-6C)alkyl in which R^{c} is hydrogen or (1-6C)alkyl;
      or **R⁶** is selected from (1-6C)alkyl, -S(O)ₚ-(1-6C)alkyl wherein p is 0, 1 or 2, or (1-6C)alkoxy,
      wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and
      wherein the (3-7C)cycloalkyl ring and saturated or partially saturated 3 to 7 membered heterocyclic ring are optionally independently substituted by one or more groups selected from (1-6C)alkyl;
   (ii) **R⁶** is selected from halo, cyano, a (3-7C)cycloalkyl ring, a saturated or partially saturated 3 to 7 membered heterocyclic ring or -N(R^{c})C(O)(1-6C)alkyl in which R^{c} is hydrogen or (1-6C)alkyl; or
      **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylanurio, di-[(1-6C)alkyl]amino, a (3-7C)cycloalkyl ring or a saturated or partially saturated 3 to 7 membered heterocyclic ring;
**m** is 0, 1, 2 or 3;
and when B is a (3-7C)cycloalkyl ring or a saturated or partially saturated 3 to 7 membered heterocyclic ring or a saturated or partially saturated 8, 9 or 10 membered bicyclic group, the rings and bicyclic group optionally bear 1 or 2 oxo or thioxo substituents;
and salts thereof, particularily pharmaceutically acceptable salts thereof.

Another particular embodiment of the compounds of the Formula I is a compound of the Formula Ie: 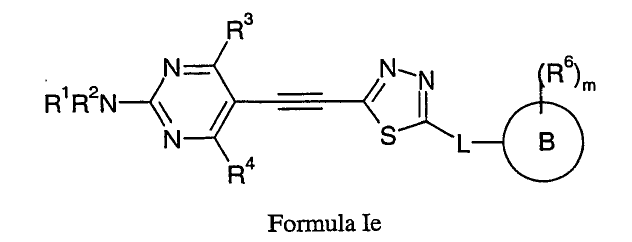 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6 , or a 5 or 6 membered heteroaryl ring, or **R¹ and R²** together with the nitrogen atom to which they are attached represent a saturated or partially saturated 3 to 7 membered heterocyclic ring optionally containing another heteroatom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (104C)alkoxy(1-4C)alkyl, hydroxy, amino, mono(1-6C)alkylamino or di-[(1-6C)alkyl]amino, or a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³** is selected from hydrogen, (1-6C)alkyl or (1-6C)alkoxy, wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)allcyl, (1-4C)alkoxy, hydroxy, amino, mono(1-6C)alkylamino or di-[(1-6C)a1kyl]amino or a saturated or partially saturated 3 to 7 membered heterocyclic ring;
   or **R³** represents a group **-NR¹R²** as defined above;
**R⁴** is selected from hydrogen, (1-6C)alkyl or (1-6C)alkoxy;
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
L is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O) O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
R⁶ is selected from one of the following 2 groups:
   (i) **R⁶** is selected from halo, cyano, oxo, a (3-7C)cycloalkyl ring, a saturated or partially saturated 3 to 7 membered heterocyclic ring and -N(R^{c})C(O)(1-6C)alkyl in which R^{c} is hydrogen or (1-6C)alkyl;
      or **R⁶** is selected from (1-6C)alkyl, -S(O)ₚ(1-6C)alkyl wherein p is 0, 1 or 2, or (1-6C)alkoxy,
      wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and
      wherein the (3-7C)cycloalkyl ring and saturated or partially saturated 3 to 7 membered heterocyclic ring are optionally independently substituted by one or more groups selected from (1-6C)alkyl;
   (ii) **R⁶** is selected from halo, cyano, a (3-7C)cycloalkyl ring, a saturated or partially saturated 3 to 7 membered heterocyclic ring or -N(R^{c})C(O)(1-6C)alkyl in which R^{c} is hydrogen or (1-6C)alkyl; or
      **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
**m** is 0, 1, 2 or 3;
and when B is a (3-7C)cycloalkyl ring or a saturated or partially saturated 3 to 7 membered heterocyclic ring or a saturated or partially saturated 8, 9 or 10 membered bicyclic group, the rings and bicyclic group optionally bear 1 or 2 oxo or thioxo substituents;
and salts thereof, particularily pharmaceutically acceptable salts thereof.

Specific compounds of the present invention are one or more of the following:
*N*-{3-[(4,6-diaminopyrimidin-5-yl)ethynyl]phenyl}-*N*'-phenylurea
N-{3-[(4,6-diaminopyrimidin-5-yl)ethynyl]phenyl}-N'-[2-fluoro-5-(trifluoromethyl) phenyl]urea
N-{3-[(4,6-diaminopyrimidin-5-yl)ethynyl]phenyl}-N'-(3,4-dichlorophenyl)urea
N-{3-[(4,6-diaminopyrimidin-5-yl)ethynyl]phenyl}-N'-[2-(trifluoromethyl)phenyl]urea
N-{3-[(4,6-diaminopyrimidin-5-yl)ethynyl]phenyl}-N'-[3-(trifluoromethyl)phenyl]urea
N-{3-[(4,6-diaminopyrimidin-5-yl)ethynyl]phenyl}-N'-[4-(trifluoromethyl)phenyl]urea
N-{4-[(4,6-diaminopyrimidin-5-yl)ethynyl]phenyl}-N'-[2-fluoro-5-(trifluoromethyl) phenyl]urea
N-{3-[(4,6-diaminopyrimidin-5-yl)ethynyl]phenyl}-N'-(3-methoxyphenyl)urea
phenyl {4-[(4,6-diaminopyrimidin-5-yl)ethynyl]phenyl}carbamate
phenyl {3-[(4,6-diaminopyrimidin-5-yl)ethynyl]phenyl}carbamate
N-(5-tert-butyl-1,3,4-thiadiazol-2-yl)-N'-{4-[(4,6-diaminopyrimidin-5-yl)ethynyl]phenyl} urea
N-{4-[(4,6-diaminopyrimidin-5-yl)ethynyl]phenyl}-N'-(3-methylisothiazol-5-yl)urea
N-{4-[(4,6-diaminopyrimidin-5-yl)ethynyl]phenyl}-N'-(3-methylisoxazol-5-yl)urea
N-{4-[(4,6-diaminopyrimidin-5-yl)ethynyl]phenyl}-N'-[4-(trifluoromethyl)pyridin-2-yl]urea
N-(3-{[({4-[(4,6-diaminopyrimidin-5-yl)ethynyl]phenyl}amino)carbonyl]amino} phenyl)acetamide
N-{3-[(4,6-diaminopyrimidin-5-yl)ethynyl]phenyl}-N'-(3-methylisothiazol-5-yl)urea
N-(3-{[({3-[(4,6-diaminopyrimidin-5-yl)ethynyl]phenyl}amino)carbonyl]amino} phenyl)acetamide
N-{3-[(4,6-diaminopyrimidin-5-yl)ethynyl]phenyl}-N'-[4-(trifluoromethyl)pyridin-2-yl]urea
N-{3-[(4,6-diaminopyrimidin-5-yl)ethynyl]phenyl}-N'-(3-methylisoxazol-5-yl)urea
N-(5-tert-butyl-1,3,4-thiadiazol-2-yl)-N'-{3-[(4,6-diaminopyrimidin-5-yl)ethynyl]phenyl}urea
N-(5-tert-butylisoxazol-3-yl)-N'-{3-[(4,6-diaminopyrimidin-5-yl)ethynyl]phenyl}urea
N-{3-[(4,6-diaminopyrimidin-5-yl)ethynyl]phenyl}-N'-(2,3-dihydro-1,4-benzodioxin-6-yl)urea
N-{3-[(4,6-diaminopyrimidin-5-yl)ethynyl]phenyl}-N'-(2-morpholin-4-ylphenyl)urea
N-{3-[(4,6-diaminopyrimidin-5-yl)ethynyl]phenyl}-N'-(1-methylpiperidin-4-yl)urea
N-{3-[(4,6-diaminopyrimidin-5-yl)ethynyl]phenyl}-N'-(1-propylpiperidin-4-yl)urea
*N*-{3-[(4,6-diaminopyrimidin-5-yl)ethynyl]phenyl}-2-phenylacetamide
N-{3-[(4,6-diaminopyrimidin-5-yl)ethynyl]phenyl}-2-(2-methoxyphenyl)acetamide
N-{3-[(4,6-diaminopyrimidin-5-yl)ethynyl]phenyl}-2-[3-(trifluoromethyl)phenyl]acetamide
N-{3-[(4,6-diaminopyrimidin-5-yl)ethynyl]phenyl}-2-[4-(trifluoromethyl)phenyl]acetamide
N-{3-[(4,6-diaminopyrimidin-5-yl)ethynyl]phenyl}-2-(3-methoxyphenyl)acetamide
*N*-(5-*tert*-butylisoxazol-3-yl)-*N*'-{3-[(4-chloropyrimidin-5-yl)ethynyl]phenyl}urea
*N*-(5-*tert*-butylisoxazol-3-yl)-N-(3-{[4-(methylamino)pyrimidin-5-yl]ethynyl}phenyl)urea
N-(5-tert-butylisoxazol-3-yl)-N'-{3-[(4-{[3-(isopropylamino)propyl] amino }pyrimidin-5-yl)ethynyl]phenyl}urea
*N*-(5-*tert*-butylisoxazol-3-yl)-*N*'-[3-({4-[(2-pyrrolidin-1-ylethyl)amino]pyrimidin-5-yl}ethynyl)phenyl]urea
*N*-(5-*tert*-butylisoxazol-3-yl)-*N*'-[3-({4-[(5-*tert*-butylisoxazol-3-yl)amino]pyrimidin-5-yl }ethynyl)phenyl]urea
and salts thereof, particularily pharmaceutically acceptable salts thereof.

Further specific compounds of the present invention are one or more of the following:
N-(5-tert-butylisoxazol-3-yl)-N'-{3-[(4-{[3-(dimethylamino)propyl]amino}pyrimidin-5-yl)ethynyl]phenyl }urea
N-(5-tert-butylisoxazol-3-yl)-N'-[3-({4-[(2-hydroxyethyl)amino]pyrimidin-5-yl }ethynyl)phenyl]urea
N-(5-tert-butylisoxazol-3-yl)-N'-[3-({4-[(2-morpholin-4-ylethyl)amino]pyrimidin-5-yl }ethynyl)phenyl]urea
N-(5-tert-butylisoxazol-3-yl)-N'-[3-({4-[(3-pyrrolidin-1-ylpropyl)amino]pyrimidin-5-yl }ethynyl)phenyl]urea
N-(5-tert-butylisoxazol-3-yl)-N'-[3-({4-[(2,4-dimethoxybenzyl)amino]pyrimidin-5-yl }ethynyl)phenyl]urea
N-{3-[(4-aminopyrimidin-5-yl)ethynyl]phenyl}-N'-(5-tert-butylisoxazol-3-yl)urea
N-{3-[(4,6-diaminopyrimidin-5-yl)ethynyl]phenyl}-2-[4-(trifluoromethyl)phenyl]acetamide;
N-(5-tert-butylisoxazol-3-yl)-N'-[3-({4-[(2-morpholin-4-ylethyl)amino]pyrimidin-5-yl }ethynyl)phenyl]urea;
and salts thereof, particularily pharmaceutically acceptable salts thereof.

A compound of the Formula I, or a pharmaceutically acceptable salt thereof, may be prepared by any process known to be applicable to the preparation of chemically-related compounds. Such processes, when used to prepare a compound of the Formula I are provided as a further feature of the invention and are illustrated by the following representative process variants. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.

According to a further aspect of the present invention provides a process for preparing a compound of formula I or a pharmaceutically acceptable salt thereof (wherein R¹, R², R³, R⁴, R⁵, R⁶, R⁷, R⁸, R⁹, R¹⁰, R¹¹,L , ring A and ring B, n and m are, unless otherwise specified, as defined in formula I) as described schematically below.
Process (a) For compounds of the formula I wherein L is -N(R⁸)C(O)N(H)-, the reaction of a compound of the formula II: 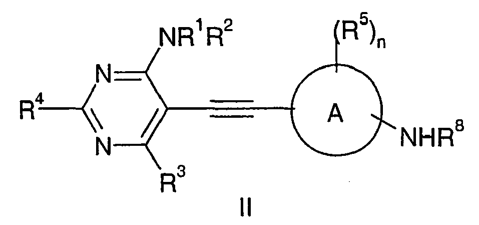 wherein R¹, R², R³, R⁴, R⁵, R⁸, n and A have any of the meanings defined hereinbefore except that any functional group is protected if necessary, with an isocyanate of the formula IV: 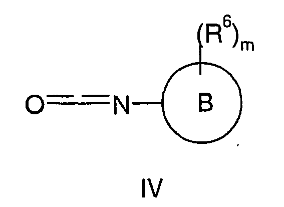 wherein R⁶, m and B have any of the meanings defined hereinbefore except that any functional group is protected if necessary;
or
Process (b) For compounds of the formula I wherein L is -N(R⁸)C(O)N(H)-, the reaction of a compound of the formula II as defined above with an aryl carbamate of the formula III: 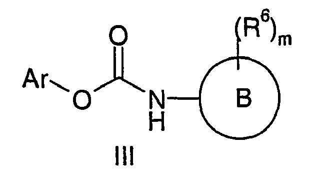 wherein Ar is a suitable aryl group, for example phenyl, and R⁶, m and B have any of the meanings defined hereinbefore except that any functional group is protected if necessary; or
Process (c) For compounds of the formula I wherein L is N(R⁸)C(O)-O-, the reaction of a compound of the formula II as defined above with a compound of the formula XI: 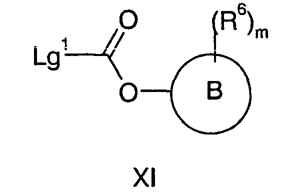 wherein Lg¹ is a suitable displaceable group, for example halogeno (such as fluoro, chloro or bromo) and R⁶, m and B have any of the meanings defined hereinbefore except that any functional group is protected if necessary;
or
Process (d) For compounds of the formula I wherein L is N(R⁸)C(O)C(R^{a}R^{b}), the reaction of a compound of the formula II as defined above with a compound of the formula IX: 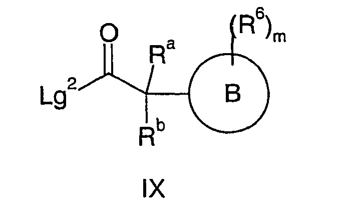 wherein Lg² is a suitable displaceable group for example hydroxy, halogeno (such as fluoro, chloro or bromo), R^{x}-C(O)-O- or R^{x}-O- (wherein R^{x} is a suitable alkyl or aryl group and R⁶, R^{a}, R^{b}, m and B have any of the meanings defined hereinbefore except that any functional group is protected if necessary;
or
Process (e) For compounds of the formula I wherein L is -N(R⁸)C(O)N(H)-, the reaction of a compound of the formula II as defined above with a trichloroacetylamine of the formula XIII: 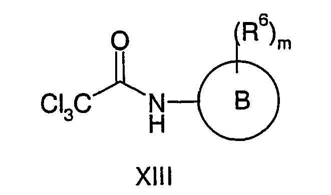 wherein R⁶, m and B have any of the meanings defined hereinbefore except that any functional group is protected if necessary;
or
Process (f) For compounds of the formula I wherein L is -C(R^{a}R^{b})C(O)N(R⁹)-, the reaction of a compound of the formula XIV: 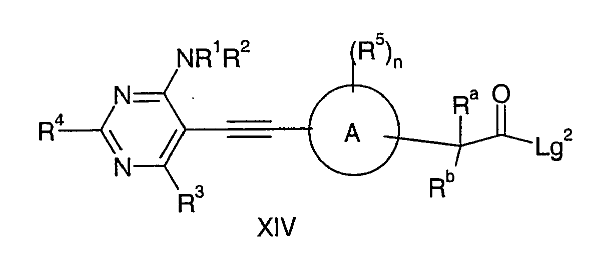 wherein Lg² is a suitable displaceable group as described above and R¹, R², R³, R⁴, R⁵, R^{a}, R^{b}, n and A have any of the meanings defined hereinbefore except that any functional group is protected if necessary, with an amine of the formula XV: 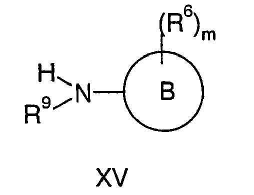 wherein R⁶, R⁹, m and B have any of the meanings defined hereinbefore except that any functional group is protected if necessary;
or
Process (g) The reaction of a compound of the formula XVI: 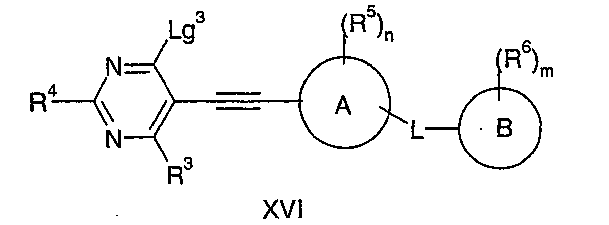 wherein Lg³ is a suitable displaceable group for example halogeno (such as fluoro, chloro, bromo or iodo), methylsulfonyl, methylthio or aryloxy (such as phenoxy) and R³, R⁴, R⁵, R⁶, n, m, A, B and L have any of the meanings defined hereinbefore except that any functional group is protected if necessary, with an amine of the formula HNR¹R², wherein R¹ and R² have any of the meanings defined hereinbefore except that any functional group is protected if necessary;
or
Process (h) The reaction of a compound of the formula XVII: 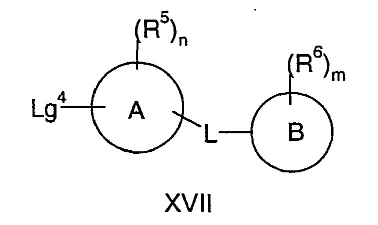 wherein Lg⁴ is a suitable displaceable group for example halogeno (such as chloro, bromo or iodo) or a sulfonyloxy group (such as trifluoromethylsulfonyloxy) and R⁵, R⁶, n, m, A, B and L have any of the meanings defined hereinbefore except that any functional group is protected if necessary, with an alkyne of the formula XVIII: 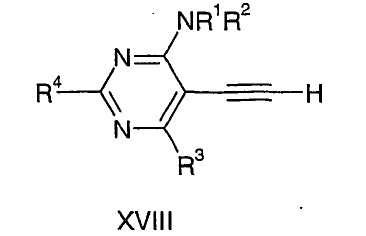 wherein R¹, R², R³ and R⁴ have any of the meanings defined hereinbefore except that any functional group is protected if necessary;
or
Process (i) For compounds of the formula I wherein L is -N(H)C(O)N(R⁹)-, the reaction of an isocyanate of the formula XIX: 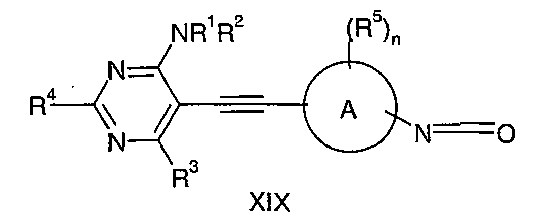 wherein R¹, R², R³, R⁴, R⁵, n and A have any of the meanings defined hereinbefore except that any functional group is protected if necessary, with an amine of the formula XV as defined above;
or
Process (j) For compounds of the formula I wherein L is -N(H)C(O)N(R⁹)-, the reaction of a compound of the formula XX: 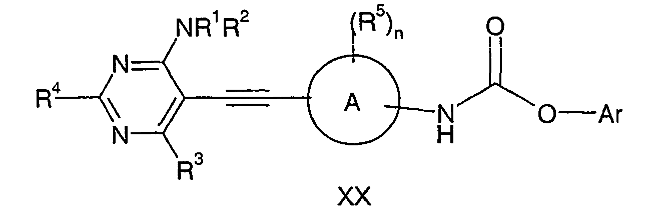 wherein Ar is a suitable aryl group, for example phenyl, and R¹, R², R³, R⁴, R⁵, n and A have any of the meanings defined hereinbefore except that any functional group is protected if necessary, with an amine of the formula XV as defined above.
and thereafter if necessary:
i) converting a compound of the Formula (I) into another compound of the Formula (I);
ii) removing any protecting groups;
iii) forming a salt.

### Reaction Conditions for Process (a)

The reaction of process (a) is conveniently carried out in the presence of a suitable inert solvent or diluent, for example a halogenated solvent such as dichloromethane, chloroform or carbon tetrachloride, an ether such as tetrahydrofuran or 1,4-dioxane, an amine such as pyridine or a dipolar aprotic solvent such as N,N-dimethylformamide or N,N-dimethylacetamide. The reaction is conveniently carried out at a temperature in the range, for example, from ambient temperature to about 60°C, preferably at or near ambient temperature.

### Reaction Conditions for Process (b)

The reaction of process (b) is conveniently carried out in the presence of a suitable base. A suitable base is, for example, an organic amine base such as pyridine or a trialkylamine (such as triethylamine or diisopropylethylamine).

The reaction of process (b) is conveniently carried out in the presence of a suitable inert solvent or diluent, for example an ether such as tetrahydrofuran or 1,4-dioxane or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from ambient temperature to about 120°C, preferably from about 80°C to about 100°C.

Conveniently, this reaction may also be performed by heating the reactants in a sealed vessel using a suitable heating apparatus such as a microwave heater.

### Reaction Conditions for Process (c)

The reaction of process (c) is conveniently carried out in the presence of a suitable base. A suitable base is, for example, an organic amine base such as pyridine or a trialkylamine (such as triethylamine or diisopropylethylamine) or, for example, an alkali or alkaline earth metal carbonate such as sodium carbonate or potassium carbonate.

The reaction of process (c) is conveniently carried out in the presence of a suitable inert solvent or diluent, for example a halogenated solvent such as dichloromethane, chloroform or carbon tetrachloride or an ether such as tetrahydrofuran or 1,4-dioxane. The reaction is conveniently carried out at a temperature in the range, for example, from about - 10°C to about 30°C, preferably at or near 0°C.

### Reaction Conditions for Process (d)

When Lg² is hydroxy, the reaction of process (d) is conveniently carried out in the presence of a suitable coupling agent. A suitable coupling agent is, for example, a suitable peptide coupling agent, for example O-(7-azabenzotriazol-1-yl)-N,N,N',N'-tetramethyluronium hexafluorophosphate (HATU) or a suitable carbodiimide such as dicyclohexylcarbodiimide (DCC) or carbonyldiimidazole (CDI), optionally in the presence of a catalyst such as dimethylaminopyridine or hydroxybenzotriazole.

When Lg² is any suitable displaceable group as described above, the reaction of process (d) may conveniently be carried out in the presence of a suitable base. A suitable base is, for example, an organic amine base such as, for example, pyridine, 2,6-lutidine, collidine, 4-dimethylaminopyridine, triethylamine, diisopropylethylamine, N-methylmorpholine or diazabicyclo[5.4.0]undec-7-ene. Another suitable base is, for example, an alkali or alkaline earth metal carbonate, for example sodium carbonate, potassium carbonate, caesium carbonate or calcium carbonate.

The reaction of process (d) is conveniently carried out in the presence of a suitable inert solvent or diluent, for example an ester such as ethyl acetate, a halogenated solvent such as dichloromethane, chloroform or carbon tetrachloride, an ether such as tetrahydrofuran or 1,4-dioxane, an aromatic solvent such as toluene or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from about 0°C to about 120°C, preferably at or near ambient temperature.

### Reaction Conditions for Process (e)

The reaction of process (e) is conveniently carried out in the presence of a suitable base. A suitable base is, for example, an organic amine base such as pyridine or a trialkylamine (such as triethylamine or diisopropylethylamine) or, for example, an alkali or alkaline earth metal carbonate, such as sodium carbonate, potassium carbonate, caesium carbonate or calcium carbonate.

The reaction of process (e) is conveniently carried out in the presence of a suitable inert solvent or diluent, for example an ether such as tetrahydrofuran or 1,4-dioxane or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from ambient temperature to about 120°C, preferably from about 100°C to about 120°C.

Conveniently, this reaction may also be performed by heating the reactants in a sealed vessel using a suitable heating apparatus such as a microwave heater.

### Reaction Conditions for Process (f)

The reaction of process (f) is conveniently carried out under the conditions as described above for process (d).

### Reaction Conditions for Process (g)

The reaction of process (g) is conveniently carried out in the presence of a catalytic amount of a suitable acid. A suitable acid is, for example, hydrogen chloride,

The reaction of process (g) may conveniently be carried out in the absence or the presence of a suitable inert solvent or diluent. A suitable inert solvent or diluent, when used, is for example an alcohol such as ethanol, isopropanol or butanol or a dipolar aprotic solvent such as acetonitrile, N,N-dimethylformamide, N,N-dimethylacetamide, N-methylpyrrolidin-2-one or dimethylsulfoxide. The reaction is conveniently carried out at a temperature in the range, for example, from ambient temperature to about 120°C, preferably from about 80°C to about 90°C.

### Reaction Conditions for Process (h)

The reaction of process (h) is conveniently carried out in the presence of a suitable palladium catalyst, optionally in combination with a suitable copper catalyst. A suitable palladium catalyst is, for example, bis(triphenylphosphine)palladium dichloride, [1,1'-bis(diphenylphosphino)ferrocene] palladium dichloride or tetrakis(triphenylphosphine)palladium(0). A suitable copper catalyst is, for example, copper (I) iodide.

The reaction of process (h) is conveniently carried out in the presence of a suitable base. A suitable base is, for example, an organic amine base, such as a trialkylamine (for example triethylamine) or tetramethylguanidine.

The reaction of process (h) may conveniently be carried out in the absence or presence of a suitable inert solvent or diluent, for example an ester such as ethyl acetate, an ether such as tetrahydrofuran or 1,4-dioxane or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from about -20°C to about 100°C.

### Reaction Conditions for Process (i)

The reaction of process (i) is conveniently carried out under the conditions as described above for process (a).

### Reaction Conditions for Process (j)

The reaction of process (j) is conveniently carried out under the conditions as described above for process (b).

### Starting Materials for Process (a)

Compounds of the formula II may be obtained by conventional procedures. For example, compounds of the formula II may be obtained by reaction of a pyrimidine of the formula VI with an alkyne of the formula VII as illustrated in *Reaction Scheme 1:* 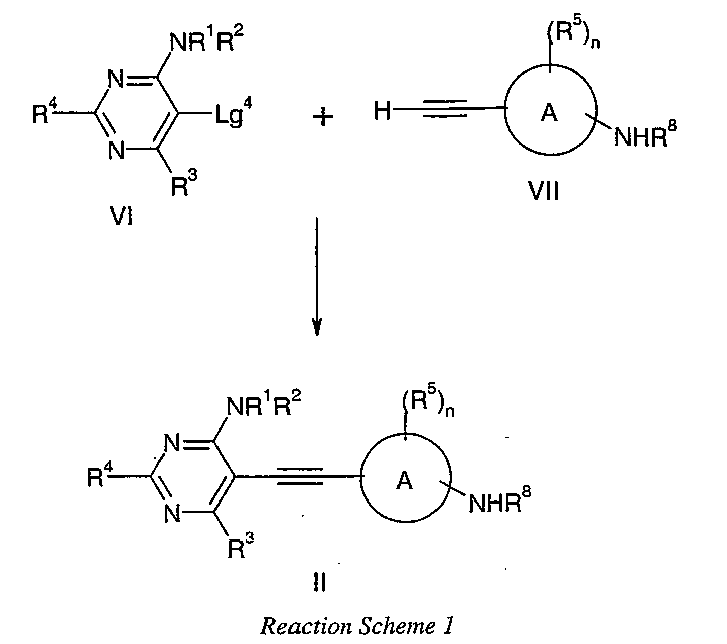 wherein Lg⁴ is a suitable displaceable group as described above and R¹, R², R³, R⁴, R⁵, R⁸, n and A have any of the meanings defined hereinbefore except that any functional group is protected if necessary.

The reaction of *Reaction Scheme 1* is conveniently carried out under the conditions as described above for process (h).

Alternatively, compounds of the formula II may be obtained by reaction of a pyrimidine of the formula VI with a protected alkyne of the formula VIa and then with an amine of the formula VIb as illustrated in *Reaction Scheme 2:* 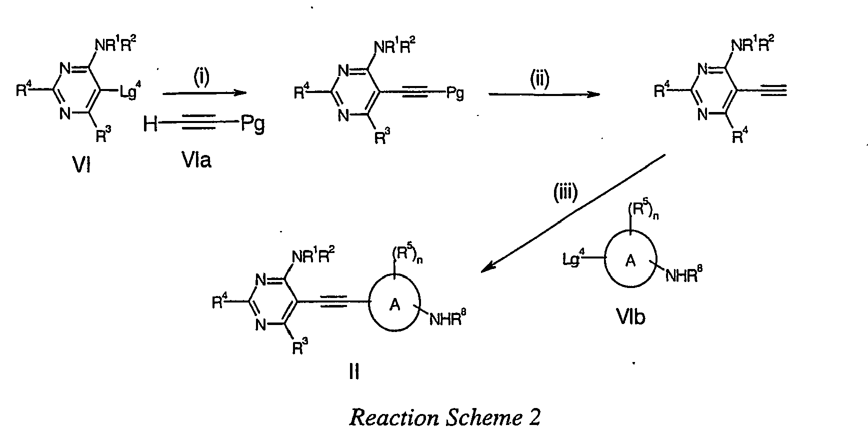 wherein Lg⁴ in the compounds of the formulae VI and VIb are each a suitable displaceable group as described above, Pg is a suitable protecting group, for example a trialkylsilyl group, such as trimethylsilyl or tert-butyldimethylsilyl or Me₂(OH)C- and R¹, R², R³, R⁴, R⁵, R⁸, n and A have any of the meanings defined hereinbefore except that any functional group is protected if necessary.

Step (i) of *Reaction Scheme 2* is the coupling of a protected alkyne of the formula VIa to a pyrimidine of the formula VI. Step (i) is carried out under conditions as described above for process (h). Step (ii) of *Reaction Scheme 2* is the deprotection of the alkyne under basic or acidic conditions to provide an unsubstituted alkyne. A person skilled in the art would readily be able to select the appropriate conditions for deprotection in step (ii). Step (iii) of *Reaction Scheme 2* is the coupling of the alkyne to an amine of the formula VIb. Step (iii) of *Reaction Scheme 2* is carried out under conditions as described above for process (h).

Alternatively, compounds of the formula II may be obtained by reaction of a compound of the formula VIc, wherein Lg³ is a suitable displaceable group as described above and R³, R⁴, R⁵, R⁸, n and A have any of the meanings defined hereinbefore except that any functional group is protected if necessary, with an amine of the formula HNR¹R² using reaction conditions as described above for process (g).

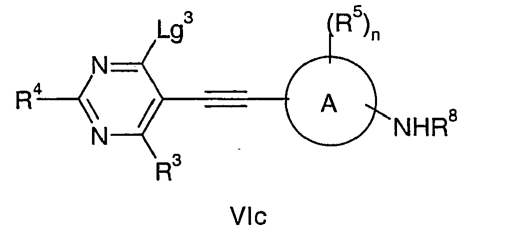

The starting materials of the formulae VI, VII, VIa, VIb and VIc and the amine HNR¹R² are commercially available or they are known in the literature, or they can be prepared by standard processes known in the art.

Isocyanates of the formula IV are commercially available or they are known in the literature, or they can be prepared by standard processes known in the art. For example, as the skilled person would appreciate, the isocyanates can conveniently be prepared from the corresponding acids or acid chlorides via a Curtis reaction with for example azide or diphenylphosphoryl azide. Alternatively, the isocyanates can conveniently be prepared by reaction of the corresponding amine with phosgene or a phosgene equivalent, for example triphosgene, diphosgene or N,N'-carbonyldiimidazole (March J., Adv. Org. Chem., 4th edition, 1992, page 1290, Wiley Interscience).

### Starting Materials for Process (b)

Compounds of the formula II may be obtained by conventional procedures as discussed above.

Aryl carbamates of the formula III are commercially available or they are known in the literature, or they can be prepared by standard processes known in the art. For example, the aryl carbamates can be prepared by reaction of an amine of the formula V with an arylchloroformate as illustrated in *Reaction Scheme 3:* 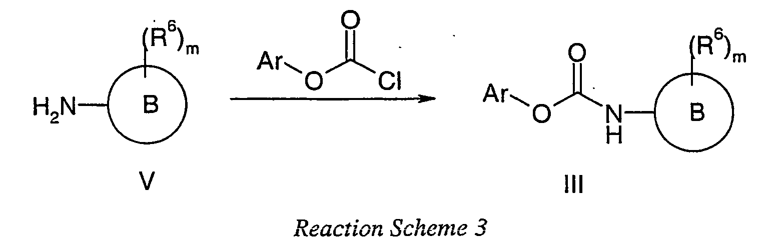 wherein R⁶, m, B and Ar have any of the meanings defined hereinbefore except that any functional group is protected if necessary.

The reaction of *Reaction Scheme 3* is conveniently carried out in the presence of a suitable base. A suitable base is, for example, an organic amine base such as pyridine or a trialkylamine (such as triethylamine).

The reaction is conveniently carried out in the presence of a suitable inert solvent or diluent, for example an ether such as tetrahydrofuran or 1,4-dioxane. The reaction is conveniently carried out at a temperature in the range, for example, from about -20°C to about 100°C, preferably at or near 0°C.

The starting material of the formula V and the arylchloroformate are commercially available or they are known in the literature, or they can be prepared by standard processes known in the art.

### Starting Materials for Process (c)

Compounds of the formula II may be obtained by conventional procedures as discussed above.

Compounds of the formula XI are commercially available or they are known in the literature, or they can be prepared by standard processes known in the art.

### Starting Materials for Process (d)

Compounds of the formula II may be obtained by conventional procedures as discussed above.

Compounds of formula IX are commercially available or they are known in the literature, or they can be prepared by standard processes known in the art.

### Starting Materials for Process (e)

Compounds of the formula II may be obtained by conventional procedures as discussed above.

Trichloroacetylamines of the formula XIII are commercially available or they are known in the literature, or they can be prepared by standard processes known in the art.

### Starting Materials for Process (f)

Compounds of the formula XIV may be obtained by conventional procedures as discussed above.

Amines of the formula XV are commercially available or they are known in the literature, or they can be prepared by standard processes known in the art.

### Starting Materials for Process (g)

As the skilled person would appreciate, compounds of the formula XVI can be prepared using similar processes to those described above using the appropriate starting materials, for example, wherein the starting materials carry an, optionally protected, group Lg³ in place of the -NR¹R² group.

Amines of the formula HNR¹R² are commercially available or they are known in the literature, or they can be prepared by standard processes known in the art.

### Starting Materials for Process (h)

Compounds of formula XVII are commercially available or they are known in the literature, or as the skilled person would appreciate they can be prepared using similar processes to those described above using the appropriate starting materials. For example, compounds of the formula XVII wherein L is -N(R⁸)C(O)N(H)- may conveniently be obtained by reaction of an amine of the formula XVIIa with an aryl carbamate of the formula XVIIb as illustrated in *Reaction Scheme 4:* 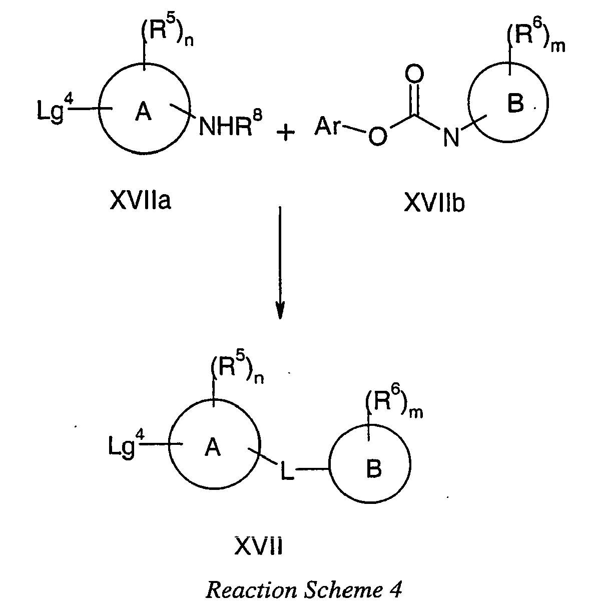 wherein Lg⁴ is a suitable displaceable group as described above, L is -N(R⁸)C(O)N(H)- and R⁵, R⁶, R⁸, n, m, A and B have any of the meanings defined hereinbefore except that any functional group is protected if necessary.

The reaction of *Reaction Scheme 4* is conveniently carried out under the conditions as described above for process (b).

The starting materials of the formulae XVIIa and XVIIb are commercially available or they are known in the literature, or they can be prepared by standard processes known in the art.

Alkynes of the formula XVIII are commercially available or as the skilled person would appreciate they can be prepared using similar processes to those described above using the appropriate starting materials. For example, compounds of the formula XVIII may conveniently be obtained by reaction of a pyrimidine of the formula XVIIIa: 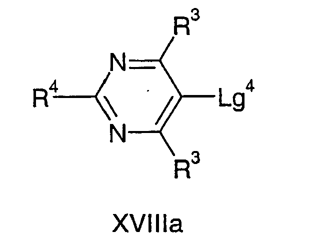 wherein Lg⁴ is a suitable displaceable group as described above and R¹, R², R³ and R⁴ have any of the meanings defined hereinbefore except that any functional group is protected if necessary, with trimethylsilylacetylene or 2-methyl-3-butyn-2-ol conveniently under the conditions as described above for process (h), followed by the removal of the protecting group using standard procedures known in the art.

### Starting Materials for Process (i)

As the skilled person would appreciate, isocyanates of the formula XIX can conveniently be prepared from the corresponding acids or acid chlorides via a Curtis reaction for example with azide or diphenylphosphoryl azide. Alternatively, the isocyanates can conveniently be prepared by reaction of the corresponding amine with phosgene or a phosgene equivalent, for example triphosgene, diphosgene or N,N'-carbonyldiimidazole (March J., Adv. Org. Chem., 4th edition, 1992, page 1290, Wiley Interscience).

Amines of the formula XV are commercially available or they are known in the literature, or they can be prepared by standard processes known in the art.

### Starting Materials for Process (j)

Compounds of the formula XX are commercially available or they are known in the literature, or as the skilled person would appreciate they can be prepared using similar processes to those described above using the appropriate starting materials.

Amines of formula XV are commercially available or they are known in the literature, or they can be prepared by standard processes known in the art.

Compounds of the formula I can be converted into further compounds of the formula I using standard procedures conventional in the art.

Examples of the types of conversion reactions that may be used include introduction of a substituent by means of an aromatic substitution reaction or of a nucleophilic substitution reaction, reduction of substituents, alkylation of substituents and oxidation of substituents. The reagents and reaction conditions for such procedures are well known in the chemical art.

Particular examples of aromatic substitution reactions include the introduction of an alkyl group using an alkyl halide and Lewis acid (such as aluminium trichloride) under Friedel Crafts conditions; and the introduction of a halogeno group. Particular examples of nucleophilic substitution reactions include the introduction of an alkoxy group or of a monoalkylamino group, a dialkyamino group or a N-containing heterocycle using standard conditions. Particular examples of reduction reactions include the reduction of a carbonyl group to a hydroxy group with sodium borohydride or of a nitro group to an amino group by catalytic hydrogenation with a nickel catalyst or by treatment with iron in the presence of hydrochloric acid with heating.

An example of a suitable conversion reaction is the conversion of a carbamate compound of the formula I wherein R¹, R², R³, R⁴, R⁵, n and A are as defined in claim 1, L is N(H)C(O)-O-, B is an optionally substituted phenyl group, to a compound of the formula I wherein L is N(H)C(O)NH and R¹, R², R³, R⁴, R⁵, n, B and A are as defined in claim 1. Such a conversion may be achieved using standard procedures, for example by reaction of the carbamate with an appropriate amine, for example under conditions as described above for process (b).

Another example of a suitable conversion reaction is the conversion of a compound of the formula I wherein R², R³, R⁴, R⁵, R⁶, n, m, A, B and L are as defined in claim 1 and R¹ and/or R² is hydrogen to a compound of the formula I wherein R¹ and/or R² is, for example, an optionally substituted (1-6C)alkoxycarbonyl group. Such a conversion may be achieved using standard procedures, for example by substitution of one or both of the hydrogen atoms R¹ and/or R² for a desired, optionally substituted, (1-6C)alkoxycarbonyl group.

Certain compounds of Formula I are capable of existing in stereoisomeric forms. It will be understood that the invention encompasses all geometric and optical isomers of the compounds of formula I and mixtures thereof including racemates. Tautomers and mixtures thereof also form an aspect of the present invention.

Isomers may be resolved or separated by conventional techniques, e.g. chromatography or fractional crystallisation. Enantiomers may be isolated by separation of a racemic or other mixture of the compounds using conventional techniques (e.g. chiral High Performance Liquid Chromatography (HPLC)). Alternatively the desired optical isomers may be made by reaction of the appropriate optically active starting materials under conditions which will not cause racemisation, or by derivatisation, for example with a homochiral acid followed by separation of the diastereomeric derivatives by conventional means (e.g. HPLC, chromatography over silica) or may be made with achiral starting materials and chiral reagents. All stereoisomers are included within the scope of the invention.

The compounds of the invention may be isolated from their reaction mixtures using conventional techniques.

It will be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

It will also be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulfinyl or alkylsulfonyl.

It is believed that certain intermediate compounds of the Formulae II, XIV, XVI, XIX, XX and VIc are novel and are herein claimed as another aspect of the present invention.

### Biological Assays

The following assays can be used to measure the effects of the compounds of the present invention as Tie2 inhibitors in vitro and as inhibitors of Tie2 autophosphorylation in whole cells.

### a. In vitro receptor tyrosine kinase inhibition assay

To test for inhibition of Tie2 receptor tyrosine kinase, compounds are evaluated in a non-cell based protein kinase assay by their ability to inhibit the protein kinase enzyme phosphorylation of a tyrosine containing polypeptide substrate in an ELISA based microtitre plate assay. In this particular case, the assay was to determine the IC₅₀, for three different recombinant human tyrosine kinases Tie2, KDR and Flt.

To facilitate production of the tyrosine kinases, recombinant receptor genes were produced using standard molecular biology cloning and mutagenesis techniques. These recombinant proteins fragments encoded within these genes consist of only the intracellular portion C-terminal portion of the respective receptor, within which is found the kinase domain. The recombinant genes encoding the kinase domain containing fragments were cloned and expressed in standard baculovirus/Sf21 system (or alternative equivalent).

Lysates were prepared from the host insect cells following protein expression by treatment with ice-cold lysis buffer (20mM N-2-hydroxyethylpiperizine-N'-2-ethanesulphonic acid (HEPES) pH7.5, 150 mM NaCl, 10% glycerol, 1% Triton X-100, 1.5 mM MgCl₂, 1 mM ethylene glycol-bis (β-aminoethyl ether) N',N',N',N'- tetraacetic acid (EGTA), plus protease inhibitors and then cleared by centrifugation. Tie2, KDR and Flt1 lysates were stored in aliquots at -80 °C.

Constitutive kinase activity of these recombinant proteins was determined by their ability to phosphorylate a synthetic peptide (made up of a random co-polymer of Glutamic Acid, Alanine and Tyrosine in the ratio of 6:3:1). Specifically, Nunc Maxisorb^{™} 96-well immunoplates were coated with 100 microlitres of synthetic peptide Sigma P3899 (1mg/ml stock solution in PBS diluted 1:500 in PBS prior to plate coating) and incubated at 4 °C overnight. Plates were washed in 50 mM HEPES pH 7.4 at room temperature to remove any excess unbound synthetic peptide.

Tie2, KDR or Flt1 activities were assessed by incubation of the appropriate freshly diluted lysates (1:200, 1:400 and 1:1000 respectively) in peptide coated plates for 60 minutes (Tie2) or 20 minutes for (KDR, Flt) at room temperature in 100 mM HEPES pH 7.4 , adenosine trisphosphate (ATP) at 5 micromolar (or Km concentration for the respective enzyme, 10 mM MnCl₂, 0.1 mM Na₃VO₄, 0.2 mM DL-dithiothreitol (DTT), 0.1% Triton X-100 together with the test compound(s) in dissolved in DMSO (final concentration of 2.5%) with final compound concentrations ranging from 0.05 micromolar-100 micromolar. Reactions were terminated by the removal of the liquid components of the assay followed by washing of the plates with PBS-T (phosphate buffered saline with 0.5% Tween 20) or an alternative equivalent wash buffer.

The immobilised phospho-peptide product of the reaction was detected by immunological methods. Firstly, plates were incubated for 4 hours at room temperature with murine monoclonal anti-phosphotyrosin -HRP (Horseradish Peroxidase) conjugated antibodies (4G10 from Upstate Biotechnology UBI 16-105). Following extensive washing with PBS-T, HRP activity in each well of the plate was measured colorimetrically using 22'-Azino-di-[3-ethylbenzthiazoline sulfonate (6)] diammonium salt crystals ABTS (Sigma P4922 - prepared as per manufactures instructions) as a substrate incubated for 30-45 minutes to allow colour development, before 100ul of 1M H2SO4 was added to stop the reaction.

Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values.

### b. Cellular Tie2 autophosphorylation assay

This assay is based on measuring the ability of compounds to inhibit autophosphorylation of the Tie2 receptor which normally leads to the production of "activated" receptor that in turn initiates the particular signal transduction pathways associated with the receptor function.

Autophosphorylation can be achieved by a number of means. It is known that expression of recombinant kinase domains in baculoviral systems can lead to the production of phosphorylated and activated receptor. It is also reported that over expression of receptors in recombinant cell lines can itself lead to receptor autophosphorylation in the absence of the ligand (Heldin C-H. 1995 Cell : 80, 213-223; Blume-J. P, Hunter T. 2001 Nature: 411, 355-65). Furthermore, there are numerous literature examples in which chimaeric receptors have been constructed. In these cases the natural, external cell surface domain of the receptor has been replaced with that of a domain which is known to be readily dimerised via the addition of the appropriate ligand (e.g. TrkA-Tie2/NGF ligand (Marron, M.B., et al., 2000 Journal of Biological Chemistry : 275:39741-39746) or C-fms-Tie-1/CSF-1 ligand (Kontos, C.D., et al., 2002 Molecular and Cellular Biology : 22, 1704-1713). Thus when the chimaeric receptor expressed in a host cell line and the respective ligand is added, this induces autophosphorylation of the chimeric receptor's kinase domain. This approach has the advantage of often allowing a known (and often easily obtained) ligand to be used instead of having to identify and isolate the natural ligand for each receptor of interest.

Naturally if the ligand is available one can use natural cell lines or primary cells which are known to express the receptor of choice and simply stimulate with ligand to achieve ligand induced phosphorylation. The ability of compounds to inhibit autophosphorylation of the Tie2 receptor, which is expressed for example in EA.hy926/B3 cells (supplied by J. McLean/ B. Tuchi, Univ.of N. Carolina at Chapel Hill, CB- 4100, 300 Bynum Hall, Chapel Hill, N.C. 27599-41000, USA) or primary HUVEC (human umbilical vein endothelial cells - available from various commercial sources), can measured by this assay.

Natural Ang1 ligand can be isolated using standard purification technology from either tumour cell supernatants or alternatively the Ang1 gene can be cloned and expressed recombinantly using stand molecular biology techniques and expression systems. In this case one can either attempt to produce the ligand either in its native state or as recombinant protein which for example may have been genetically engineered to contain additional of purification tags (eg. polyhistidine peptides, antibody Fc domains) to facilitate the process.

Using the ligand stimulation of either EA.hy926/B3 or HUVEC cellular Tie2 receptor as the example, a Ang1 ligand stimulated cellular receptor phosphorylation assay can be constructed which can be used to analyse to determine the potential of compounds to inhibit this process. For example EA.hy926/B3 cells were grown in the appropriate tissue culture media plus 10% foetal calf serum (FCS) for two days in 6 well plates starting with an initial seeding density of 5x10⁵ cells/well. On the third day the cells were serum starved for a total of 2 hours by replacing the previous media with media containing only 1% FCS. After 1 hour 40 minutes of serum starvation the media was removed and replace with 1 ml of the test compound dilutions (compound dilutions made in serum starvation media yet keeping the DMSO concentration below 0.8%). After 1.5 hours of serum starvation orthovanidate was added to a final concentration of 0.1 mM for the final 10 minutes of serum starvation.

Following a total of 2 hours of serum starvation, the ligand plus orthovandiate was added to stimulate autophosphorylation of the cellular Tie2 receptor (ligand can be added either as purified material diluted in serum starvation media or non-purified cell supernatant containing ligand e.g. when recombinantly expressed mammalian cells). After 10 minutes incubation at 37 °C with the ligand, the cells were cooled on ice washed with approximately 5mls with cold PBS containing 1 mM orthovanadate, after which 1 ml of ice cold lysis buffer ((20 mM Tris pH 7.6, 150 mM NaCl, 50 mM NaF, 0.1 % SDS, 1 % NP40, 0.5 % DOC, 1 mM orthovanadate, 1 mM EDTA, 1 mM PMSF, 30 µl / ml Aprotinin, 10 µg/ml Pepstatin, 10 µg/ml Leupeptin) was addedthe cells and left on ice for 10- 20 minutes. The lysate was removed and transferred to a 1.5 ml Eppendorf tube and centrifuged for 3 minutes at 13000 rpm at 4 °C. 800 µl of each lysate was transferred to fresh 2 ml Eppendorf tubes for the immuno-precipitation. 3 mg = 15 µl of anti-phospho-tyrosine antibody (Santa Cruz PY99 -sc-7020) was added to the lysates and left to incubate for 2 hours at 4 °C. 600 µl washed MagnaBind beads (goat anti-mouse IgG, Pierce 21354) were added to the lysates and the tubes left to rotate over night at 4 °C.

Samples were treated for 1 minute in the magnet before carefully removing the lysis supernatant. 1 ml of lysis buffer was then added to the beads and this step repeated twice more. The beads were suspended in 25 µl of 94 °C hot 2 x Laemmli loading buffer plus betamercaptoethanol and left to stand for 15 minutes at room temperature.

The beads were removed by exposing the tubes for 1 minutes in the magnet, and the total liquid separated from the beads from each immuno-precipitate loaded onto Polyacrylamide/SDS protein gels (pre-cast 4-12 % BisTris NuPAGE / MOPS 12 well gels from Novex). Protein gels were run at 200 V and then blotted onto NC membrane for 1hours30 minutes at 50 V / 250 mA. All blots were treated with 5% Marvel in PBS-Tween for1 hour at room temperature to reduce non-specific binding of the detection antibody. A rabbit anti-Tie2 (Santa Cruz sc-324) was added in a 1:500 dilution in 0.5 % Marvel / PBS-Tween and left to incubate overnight at 4 °C. The blots were rigorously washed with PBS-Tween before adding the goat anti rabbit -POD conjugate (Dako P0448) at a 1:5000 dilution in 0.5 % Marvel / PBS-Tween. The antibody was left on for 1 hour at room temperature before subsequently washing the blots with PBS-Tween. The western blots of the various immuno-precipitated samples were developed the blots with LumiGLO (NEB 7003). And transferred to an X-Ray cassette and films exposed for 15 sec / 30 sec and 60 sec. The relative strength of the protein band which pertains to the phosphorylated Tie2 receptor was evaluated using a FluorS BioRad image analyser system. The percentage phosphorylation for each test compound dilution series was determined from which IC₅₀ values were calculated by standard methods using the appropriate control samples as reference.

Although the pharmacological properties of the compounds of the Formula I vary with structural change as expected, in general activity possessed by compounds of the Formula I, may be demonstrated at the following concentrations or doses in one or more of the above tests (a) and (b):-
1(a):- IC₅₀ in the range, for example, < 100µM;
Test (b):- IC₅₀ in the range, for example, < 50µM;

By way of example, Table A illustrates the activity of representative compounds according to the invention. Column 2 of Table A shows IC₅₀ data from Test (a) for the inhibition of Tie2 receptor tyrosine kinase *in vitro* and column 3 shows IC₅₀ data from Test (b) for the inhibition of autophosphorylation of Tie2 receptor tyrosine kinase.

**Table A:**

| **Example Number** | **IC₅₀ (µM) Test (a): Inhibition of Tie2 receptor tyrosine kinase *in vitro*** | **IC₅₀ (µM) Test (b): Inhibition of autophosphorylation of Tie2 receptor tyrosine kinase** |
|---|---|---|
| 11 | 0.879 | 5.557 |
| 12 | 2.849 | 0.3513 |
| 19 | 2.141 | 3.539 |

According to a further aspect of the invention there is provided a pharmaceutical composition which comprises a compound of the Formula I, or a pharmaceutically acceptable salt thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the Formula I will naturally vary according to the nature and severity of the conditions, the age and sex of the animal or patient and the route of administration, according to well known principles of medicine.

In using a compound of the Formula I for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a compound of this invention.

The compounds according to the present invention as defined herein are of interest for, amongst other things, their antiangiogenic effect. The compounds of the invention are expected to be useful in the treatment or prophylaxis of a wide range of disease states associated with undesirable or pathological angiogenesis, including cancer, diabetes, psoriasis, rheumatoid arthritis, Kaposi's sarcoma, haemangioma, lymphoedema, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation. Cancer may affect any tissue and includes leukaemia, multiple myeloma and lymphoma. In particular such compounds of the invention are expected to slow advantageously the growth of primary and recurrent solid tumours of, for example, the colon, breast, prostate, lungs and skin.

We believe that the antiangiogenic properties of the compounds according to the present invention arise from their Tie2 receptor tyrosine kinase inhibitory properties. Accordingly, the compounds of the present invention are expected be useful to produce a Tie2 inhibitory effect in a warm-blooded animal in need of such treatment. Thus the compounds of the present invention may be used to produce an antiangiogenic effect mediated alone or in part by the inhibition of Tie2 receptor tyrosine kinase.

More particularly the compounds of the invention are expected to inhibit any form of cancer associated with Tie2. For example, the growth of those primary and recurrent solid tumours which are associated with Tie2, especially those tumours which are significantly dependent on Tie2 receptor tyrosine kinase for their growth and spread.

According to a further aspect of the invention there is provided a compound of the Formula I, or a pharmaceutically-acceptable salt thereof, as defined hereinbefore, for use as a medicament.

According to another aspect of the invention, there is provided the use of a compound of the formula I, or a pharmaceutically acceptable salt thereof, as defined hereinbefore, in the manufacture of a medicament for use as a Tie2 receptor tyrosine kinase inhibitor in a warm-blooded animal such as man.

According to another aspect of the invention, there is provided the use of a compound of the formula I, or a pharmaceutically acceptable salt thereof, as defined hereinbefore, in the manufacture of a medicament for use in the production of an anti-angiogenic effect in a warm-blooded animal such as man.

According to another aspect of the invention, there is provided the use of a compound of the formula I, or a pharmaceutically acceptable salt thereof, as defined hereinbefore in the manufacture of a medicament for use in the treatment of cancers in a warm-blooded animal such as man.

According to another aspect of the invention, there is provided the use of a compound of the formula I, or a pharmaceutically acceptable salt thereof, as defined hereinbefore in the manufacture of a medicament for use in the treatment of a cancer selected from leukaemia, breast, lung, colon, rectal, stomach, prostate, bladder, pancreas, ovarian, lymphoma, testicular, neuroblastoma, hepatic, bile duct, renal cell, uterine, thyroid and skin cancer in a warm-blooded animal such as man.

According to another aspect of the invention there is provided compound of formula (I) for the manufacture of a medicament to be used in a method of inhibiting Tie2 receptor tyrosine kinase in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided compound of formula (I) for the manufacture of a medicament to be used in a method for producing an anti-angiogenic effect in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided compound of formula (I) for the manufacture of a medicament to be used in a method of treating cancers in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided compound of formula (I) for the manufacture of a medicament to be used in a method of treating a cancer selected from leukaemia, breast, lung, colon, rectal, stomach, prostate, bladder, pancreas, ovarian, lymphoma, testicular, neuroblastoma, hepatic, bile duct, renal cell, uterine, thyroid or skin cancer, in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided a compound of the formula I, or a pharmaceutically acceptable salt thereof, as defined hereinbefore, for use in inhibiting Tie2 receptor tyrosine kinase in a warm-blooded animal, such as man.

According to an another aspect of the invention there is provided a compound of the formula I, or a pharmaceutically acceptable salt thereof, as defined hereinbefore, for use in producing an anti-angiogenic effect in a warm-blooded animal, such as man.

According to another aspect of the invention there is provided a compound of the formula I, or a pharmaceutically acceptable salt thereof, as defined hereinbefore, for use in the treatment of cancer.

According to another aspect of the invention there is provided a compound of the formula I, or a pharmaceutically acceptable salt thereof, as defined hereinbefore, for use in the treatment of a cancer selected from leukaemia, breast, lung, colon, rectal, stomach, prostate, bladder, pancreas, ovarian, lymphoma, testicular, neuroblastoma, hepatic, bile duct, renal cell, uterine, thyroid or skin cancer.

As hereinbefore mentioned it is further expected that a compound of the present invention will possess activity against other diseases mediated by undesirable or pathological angiogenesis including psoriasis, rheumatoid arthritis, Kaposi's sarcoma, haemangioma, lymphoedema, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation.

The anti-angiogenic activity defined herein may be applied as a sole therapy or may involve, in addition to a compound of the invention, one or more other substances and/or treatments. Such conjoint treatment may be achieved by way of the simultaneous, sequential or separate administration of the individual components of the treatment. In the field of medical oncology it is normal practice to use a combination of different forms of treatment to treat each patient with cancer. In medical oncology the other component(s) of such conjoint treatment in addition to the cell cycle inhibitory treatment defined hereinbefore may be: surgery, radiotherapy or chemotherapy. Such chemotherapy may include one or more of the following categories of anti-tumour agents:
(i) anti-invasion agents (for example metalloproteinase inhibitors like marimastat and inhibitors of urokinase plasminogen activator receptor function);
(ii) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea, or, for example, one of the preferred antimetabolites disclosed in European Patent Application No. 562734 such as (2S)-2-{o-fluoro-p-[N-{2,7-dimethyl-4-oxo-3,4-dihydroquinazolin-6-ylmethyl)-N-(prop-2-ynyl)amino]benzamido}-4-(tetrazol-5-yl)butyric acid); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(iii) cytostatic agents such as antioestrogens (for example tamoxifen, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrazole, vorazole and exemestane) and inhibitors of 5 α-reductase such as finasteride;
(iv) inhibitors of growth factor function, for example such inhibitors include growth factor antibodies, growth factor receptor antibodies, farnesyl transferase inhibitors, tyrosine kinase inhibitors and serine/threonine kinase inhibitors, for example inhibitors of the epidermal growth factor family (for example the EGFR tyrosine kinase inhibitors N-(3-chloro-4-fluorophenyl)-7-methoxy-6-(3-morpholinopropoxy)quinazolin-4-amine (ZD 1839), N-(3-ethynylphenyl)-6,7-bis(2-methoxyethoxy)quinazolin-4-amine (CP 358774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents that work by different mechanisms to those defined hereinbefore, such as those which inhibit vascular endothelial growth factor such as the compounds disclosed in International Patent Applications WO 97/22596, WO 97/30035, WO 97/32856 and WO 98/13354 and those that work by other mechanisms (for example linomide, inhibitors of integrin αvβ3 function and angiostatin);
(vi) biotherapeutic therapeutic approaches for example those which use peptides or proteins (such as antibodies or soluble external receptor domain constructions) which either sequest receptor ligands, block ligand binding to receptor or decrease receptor signalling (e.g. due to enhanced receptor degradation or lowered expression levels)
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product comprising a compound of the Formula I as defined hereinbefore and an additional anti-tumour substance as defined hereinbefore for the conjoint treatment of cancer.

In addition to their use in therapeutic medicine, the compounds of Formula I and their pharmaceutically acceptable salts, are also useful as pharmacological tools in the development and standardisation of in vitro and *in vivo* test systems for the evaluation of the effects of inhibitors of cell cycle activity in laboratory animals such as cats, dogs, rabbits, monkeys, rats and mice, as part of the search for new therapeutic agents.

The invention will now be illustrated by the following non limiting examples in which, unless stated otherwise:
(i) temperatures are given in degrees Celsius (°C); operations were carried out at room or ambient temperature, that is, at a temperature in the range of 18-25 °C;
(ii) organic solutions were dried over anhydrous magnesium sulfate; evaporation of solvent was carried out using a rotary evaporator under reduced pressure (600-4000 Pascals; 4.5-30 mmHg) with a bath temperature of up to 60 °C;
(iii) chromatography means flash chromatography on silica gel; thin layer chromatography (TLC) was carried out on silica gel plates;
(iv) in general, the course of reactions was followed by TLC and / or analytical LC-MS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulphoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms; and
(x) mass spectra (MS) were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is MH⁺;
(xi) unless stated otherwise compounds containing an asymmetrically substituted carbon and/or sulphur atom have not been resolved;
(xii) where a synthesis is described as being analogous to that described in a previous example the amounts used are the millimolar ratio equivalents to those used in the previous example;
(xvi) the following abbreviations have been used:
   - AcOH: Acetic acid
   - AIBN: 2,2'-Azobisisobutyronitrile
   - DCM: Dichloromethane
   - DIPEA: Diisopropylethylamine
   - DMA: *N,N*-Dimethylacetamide
   - DMF: *N,N*-Dimethylformamide
   - DMSO: Dimethylsulfoxide
   - DMTMM: 4-(4,6-Dimethoxy-1,3,5-triazin-2-yl)-4-methylmorpholin-4-ium chloride
   - dppf: 1,1'-Bis(diphenylphosphino)ferrocene
   - EtOAc: Ethylacetate
   - HATU: O-(7-Azabenzotriazol-1-yl)-N,N,N',N'-tetramethyluronium hexafluorophosphate
   - *ⁱ*PrMgCl: Isopropylmagnesium chloride
   - LDA: Lithium diisopropylamide
   - LHMDS: Lithium bis(trimethylsilyl) amide
   - *m*-CPBA: *meta*-Chloroperbenzoic acid
   - MeOH: Methanol
   - MeCN: Acetonitrile
   - MCX: Mixed cation exchange resin
   - MTBE: Methyl *tert*-butyl ether
   - LCMS: Liquid Chromatograpy - Mass Spectrometry
   - NMP: 1-Methyl-2-pyrrolidinone
   - PhTosMIC: α-Tosylbenzyl isocyanide
   - POCl₃: Phosphorus oxychloride
   - RPHPLC: Reversed phase high peformance liquid chromatography
   - TFA: Trifluoroacetic acid
   - THF: Tetrahydrofuran
xvii) where a synthesis is described as leading to an acid addition salt (e.g. HCl salt), no comment is made on the stoichiometry of this salt. Unless otherwise stated, all NMR data is reported on free-base material, with isolated salts converted to the free-base form prior to characterisation.

### Example 1

### N-{3-[(4,6-diaminopyrimidin-5-yl)ethynyl]phenyl}-N'-phenylurea

5-[(3-Aminophenyl)ethynyl]pyrimidine-4,6-diamine (M875810) (45.0 mg) was stirred in THFand phenyl isocyanate (33.4 mg) was added dropwise. After 30 min, methylethylenediamine-polystyrene (400 mg) was added and stirring continued for 30 min. The reaction mixture was filtered and concentrated to give a yellow solid which was triturated with DCM (7 mL) to give the title compound as a beige solid (38 mg, 56%);
¹H NMR (DMSO-d₆) 6.61 (bs, 4H), 7.01-7.06 (m, 2H), 7.31-7.40 (m, 4H), 7.46-7.54 (m, 3H), 7.77-7.79 (m, 1H), 7.90 (s, 1H), 8.72-8.76 (m, 2H);
MS m/e MH⁺ 345.

### Preparation of Intermediate

### 5-[(3-aminophenyl)ethynyl]pyrimidine-4,6-diamine

4,6-Diamino-5-iodopyrimidine (J. Med. Chem., 2001, 44, 2133-2138) (2.36 g), bis(triphenylphosphine)palladium dichloride (350 mg) and copper(I) iodide (40 mg) were stirred in DMF (100 mL)-triethylamine (20 mL) and degassed with nitrogen for 10 min. 3-Ethynyl aniline (1.29 g) was added and the mixture heated to 95 °C for 20 hours. The solvent was evaporated and the residue was purified by flash chromatography on silica using 1-10% (7M ammonia in MeOH) in DCM as eluent. Further purification by trituration with DCM (20 mL) gave the title compound as a brown solid (970 mg, 43%);
¹H NMR (DMSO-d₆) 3.72 (bs, 2H), 5.17 (bs, 4H), 6.67-6.71 (m, 1H), 6.80-6.82 (m, 1H), 6.88-6.92 (m, 1H), 7.11-7.17 (m, 1H), 8.08 (s, 1H);
MS m/e MH⁺ 226.

### Example 2

### N-{3-[(4,6-diaminopyrimidin-5-yl)ethynyl]phenyl}-2-phenylacetamide

Phenylacetyl chloride (55.7 mg) and pyridine (47.5 mg) were added to a stirred solution of 5-[(3-aminophenyl)ethynyl]pyrimidine-4,6-diamine (67.6 mg) in THF. After 60 min, the reaction mixture was concentrated to give a yellow solid. Purification by flash chromatography on silica using 1-5% (7M ammonia in MeOH) in DCM as eluent to give the title compound as a yellow solid (23.2 mg, 23%);
¹H NMR (DMSO-d₆) 3.66 (s, 2H), 6.55 (bs, 4H), 7.26-7.42 (m, 8H), 7.52-7.55 (m, 1H), 7.86 (s, 1H), 10.20 (bs, 1H);
MS m/e MH⁺ 344.

### Example 3

### N-{3-[(4,6-diaminopyrimidin-5-yl)ethynyl]phenyl}-N'-(3,4-dichlorophenyl)urea

Starting Materials: Intermediate 1 and 3,4-dichlorophenyl isocyanate.
¹H NMR (DMSO-d₆) 6.57 (bs, 2H), 7.31-7.45 (m, 6H), 7.76-8.84 (m, 4H), 9.50-9.60 (m, 1H), 12.74 (bs, 1H);
MS m/e MH⁺ 414.

### Example 4

### N-{3-[(4,6-diaminopyrimidin-5-yl)ethynyl]phenyl}-N'-[2-(tritluoromethyl)phenyl]urea

Starting Materials: Intermediate 1 and 2-trifluoromethylphenyl isocyanate.
¹H NMR (DMSO-d₆) 6.54 (s, 4H), 7.26-7.72 (m, 6H), 7.83 (s, 1H), 7.93-7.95 (m, 1H), 8.10 (s, 1H), 9.37 (bs, 1H), 11.65 (bs, 1H);
MS m/e MH⁺ 413.

### Example 5

### N-{3-[(4,6-diaminopyrimidin-5-yl)ethynyl]phenyl}-N'-[3-(trifluoromethyl)phenyl]urea

Starting Materials: Intermediate 1, 3-trifluoromethylphenyl isocyanate.
¹H NMR (DMSO-d₆) 6.55 (s, 4H), 7.28-7.55 (m, 5H), 7.75 (s, 1H), 7.83 (s, 1H), 8.03 (s, 1H), 8.77 (s, 1H), 9.07 (s, 1H), 11.65 (bs, 1H);
MS m/e MH⁺ 413.

### Example 6

### N-{3-[(4,6-diaminopyrimidin-5-yl)ethynyl]phenyl}-N'-[4-(trifluoromethyl)phenyl]urea

Starting Materials: Intermediate 1 and 4-trifluoromethylphenyl isocyanate.
¹H NMR (DMSO-d₆) 6.55 (s, 4H), 7.268-7.84 (m, 8H), 8.80 (s, 1H), 9.14 (s, 1H), 11.65 (bs, 1H);
MS m/e MH⁺ 413.

### Example 7

### N-{4-[(4,6-diaminopyrimidin-5-yl)ethynyl]phenyl}-N'-[2-fluoro-5-(trifluoromethyl)phenyl]urea

Example 7 was prepared by an analogous method to Example 1, using Intermediate 2 in place of Intermediate 1 and 2-fluoro-5-trifluoromethylphenyl isocyanate in place of phenyl isocyanate.
¹H NMR (DMSO-d₆) 6.54 (s, 4H), 7.38-7.57 (m, 4H), 7.66-7.69 (m, 2H), 7.85 (s, 1H), 8.62-8.65 (m, 1H), 8.95 (bs, 1H), 9.32 (bs, 1H);
MS m/e MH⁺ 431.

### Example 8

### N-{3-[(4,6-diaminopyrimidin-5-yl)ethynyl]phenyl}-N'-(3-methoxyphenyl)urea

A solution of phosgene (20% solution in toluene) (2.3 mL) was added dropwise to a stirred suspension of 5-[(3-aminophenyl)ethynyl]pyrimidine-4,6-diamine (Intermediate 1) (50 mg) in EtOAc (1.2 mL) at 60°C. After 1 hr the temperature was increased to reflux overnight. The reaction mixture was concentrated *in vacuo* and azeotroped with toluene. *m*-Anisidine (27 mg) and DIPEA (0.08 mL) in THF (1 mL) was added to a stirred suspension of the isocyanate in THF (1 mL). After 10 min water (10 mL) was added, the reaction mixture extracted with EtOAc (2x5 mL), the combined organics dried (MgSO₄), filtered and concentrated *in vacuo.* Purification by flash chromatography on silica using 1-10% MeOH in DCM as eluent gave the title compound as a white solid (32 mg, 39%);
¹H NMR (DMSO-d₆) 3.72 (s, 3H), 6.48-6.61 (m, 5H), 6.89-6.95 (m, 1H), 7.13-7.21 (m, 2H), 7.23-7.34 (m, 2H), 7.38-7.43 (m, 1H), 7.70 (s, 1H), 7.83 (s, 1H), 8.63 (s, 1H), 8.70 (s, 1H); MS m/e MH⁺ 375.

### Example 9

### Phenyl {4-[(4,6-diaminopyrimidin-5-yl)ethynyl]phenyl}carbamate

Phenylchloroformate (0.84 mL) was added dropwise to a stirred solution of 5-[(4-aminophenyl)ethynyl]pyrimidine-4,6-diamine (Intermediate 2) (1.0 g) and pyridine (0.72 mL) in THF (75 mL) at 0°C. After 2 hr water (10 mL) was added, the reaction mixture stirred for 10 min then concentrated *in vacuo.* The solid obtained was triturated with water followed by ether, then dried under vacuum at 60°C to give the title compound as a brown solid (1.21 g, 79%);
¹H NMR (DMSO-d₆) 6.47-6.61 (bs, 4H), 7.18-7.29 (m, 3H), 7.38-7.54 (m, 4H), 7.62-7.69 (d, 2H), 7.83 (s, 1H), 10.35 (s, 1H);
MS m/e MH⁺ 346.

### Example 10

### Phenyl {3-[(4,6-diaminopyrimidin-5-yl)ethynyl]phenyl}carbamate

Example 10 was prepared by an analogous method to Example 9 and purification was carried out by trituration with ether then by flash chromatography on silica using 1-10% MeOH in DCM as eluent, followed by trituration with methanol.
Starting Materials: Intermediate 1 and phenylchloroformate.
¹H NMR (DMSO-d₆) 6.53 (s, 4H), 7.17-7.46 (m, 8H), 7.77 (s, 1H), 7.83 (s, 1H), 10.24 (bs, 1H);
MS m/e MH⁺ 346.

### Example 11

### N-(5-tert-butyl-1,3,4-thiadiazol-2-yl)-N'-{4-[(4,6-diaminopyrimidin-5-yl)ethynyl]phenyl}urea

5-[(4-Aminophenyl)ethynyl]pyrimidine-4,6-diamine (Intermediate 2) (30.0 mg), triethylamine (0.022 mL) and phenyl(5-tert-butyl-l,3,4-thiadiazol-2-yl)carbamate (Intermediate 3) (44.0 mg) in THF (2 mL) were irradiated under microwave conditions (CEM explorer, 80°C, 50W) for 20 min. The reaction mixture was concentrated *in vacuo,* purification by flash chromatography on silica using 1-10% MeOH in DCM as eluent gave the title compound as a yellow solid (11 mg, 21%);
¹H NMR (DMSO-d₆) 1.38 (s, 9H), 6.52 (bs, 4H), 7.49-7.52 (d, 2H), 7.63-7.66 (d, 2H), 7.82 (s, 1H), 9.18 (bs, 1H);
MS m/e MH⁺ 409.

### Intermediate 3

### Phenyl (5-tert-butyl-1,3,4-thiadiazol-2-yl)carbamate

Phenylchloroformate (0.6 mL) was added dropwise to 2-amino-5-tert-butyl-1,3,4-thiadiazole (0.5 g) and pyridine (0.51 mL) in THF (40 mL) at 0°C. After 2 hour, the reaction mixture was quenched with water (10 mL) and extracted with EtOAc (3x 10 mL). The combined organics were dried (MgSO₄), filtered and concentrated *in vacuo.* Purification by flash chromatography on silica using 20-50% EtOAc in isohexane gave the title compound as a yellow solid (0.819 mg, 93%)
¹H NMR (DMSO-d₆) 1.38 (s, 9H), 7.22-7.28 (m, 3H), 7.41-7.44 (m, 2H);
MS m/e MH⁺ 278.

Intermediates 4 to 8 were prepared by an analogous method to Intermediate 3 by using the appropriate aminoheterocycle.

### Intermediate 4

### Phenyl (3-methylisoxazol-5-yl)carbamate

Starting Materials: Phenylchloroformate and 5-amino-3-methylisoxazole.
¹H NMR (DMSO-d₆) 2.17 (s, 3H), 5.93 (s, 1H), 7.21-7.30 (m, 3H), 7.41-7.46 (m, 2H), 11.79 (bs, 1H);
MS m/e MH⁺ 219.

### Intermediate 5

### Phenyl (5-tert-butylisoxazol-3-yl)carbamate

Starting Materials: Phenylchloroformate and 3-amino-5-*tert*-butylisoxazole.
¹H NMR (DMSO-d₆) 1.28 (s, 9H), 6.42 (s, 1H), 7.18-7.26 (m, 3H), 7.39-7.45 (m, 2H), 11.13 (bs, 1H);
MS m/e MH⁺ 261.

### Intermediate 6

### phenyl [4-(trifluoromethyl)pyridin-2-yl]carbamate

Starting Materials: Phenylchloroformate and 2-amino-4-trifluoromethylpyridine.
¹H NMR (DMSO-d₆) 7.22-7.30 (m, 3H), 7.41-7.46 (m, 3H), 8.11 (s, 1H), 8.59-8.61 (d, 1H), 11.23 (bs, 1H);
MS m/e MH⁺ 283.

### Intermediate 7

### Phenyl [3-(acetylamino)phenyl]carbamate

Starting Materials: Phenylchloroformate and 3-amino-acetanilide.
¹H NMR (DMSO-d₆) 2.01 (s, 3H), 7.17-7.30 (m, 6H), 7.38-7.44 (m, 2H), 7.77 (s, 1H), 9.90 (bs, 1H), 10.16 (bs, 1H);
MS m/e MH⁺ 271.

### Intermediate 8

### Phenyl (3-methylisothiazol-5-yl)carbamate

Starting Materials: Phenylchloroformate and 5-amino-3-methylisothiazole.
¹H NMR (DMSO-d₆) 2.30 (s, 3H), 6.68 (s, 1H), 7.25-7.31 (m, 3H), 7.41-7.46 (m, 2H), 11.90 (bs, 1H);
MS m/e MH⁺ 235.

Examples 12 to 15 were prepared by an analogous method to Example 11 by reacting Intermediate 2 with the appropriate phenyl carbamate:

### Example 12

### N-{4-[(4,6-diaminopyrimidin-5-yl)ethynyl]phenyl}-N'-(3-methylisothiazol-5-yl)urea

Starting Materials: Intermediate 2 and Intermediate 8.
¹H NMR (DMSO-d₆) 2.28 (s, 3H), 6.65 (s, 1H), 6.68 (bs, 4H), 7.46-7.49 (d, 2H), 7.64-7.66 (d, 2H), 7.86 (s, 1H), 9.28 (s, 1H), 10.44 (bs, 1H);
MS m/e MH⁺ 366.

### Example 13

### N-{4-[(4,6-diaminopyrimidin-5-yl)ethynyl]phenyl}-N'-(3-methylisoxazol-5-yl)urea

Starting Materials: Intermediate 2 and Intermediate 4.
¹H NMR (DMSO-d₆) 2.16 (s, 3H), 5.95 (s, 1H), 6.66 (bs, 4H), 7.44-7.47 (d, 2H), 7.62-7.65 (d, 2H), 7.85 (s, 1H), 9.12 (s, 1H), 10.20 (bs, 1H);
MS m/e MH⁺ 350.

### Example 14

### N-{4-[(4,6-diaminopyrimidin-5-yl)ethynyl]phenyl}-N'-[4-(trifluoromethyl)pyridin-2-yl]urea

Starting Materials: Intermediate 2 and Intermediate 6.
¹H NMR (DMSO-d₆) 7.37 (d, 1H), 7.54-7.57 (d, 2H), 7.70-7.73 (d, 2H), 7.88 (bs, 4H), 8.06 (s, 1H), 8.16 (s, 1H), 8.53-8.55 (d, 1H), 9.77 (s, 1H), 9.91 (s, 1H);
MS m/e MH⁺ 414.

### Example 15

### N-(3-{[({4-[(4,6-diaminopyrimidin-5-yl)ethynyl]phenyl}amino)carbonyl]amino}phenyl)acetamide

Starting Materials: Intermediate 2 and Intermediate 7.
¹H NMR (DMSO-d₆) 2.05 (s, 3H), 6.85-7.05 (m, 1H), 7.08-7.10 (m, 1H), 7.15 (bs, 4H), 7.48-7.50 (d, 2H), 7.64-7.68 (d, 2H), 7.79 (s, 1H), 8.18 (s, 1H), 8.78-8.82 (d, 1H), 9.88 (s, 1H);
MS m/e MH⁺ 402.

Examples 16 to 18 were prepared by an analogous method to Example 11 but using Intermediate 1 in place of Intermediate 2 with the appropriate phenyl carbamate.

### Example 16

### N-{3-[(4,6-diaminopyrimidin-5-yl)ethynyl]phenyl}-N'-(3-methylisothiazol-5-yl)urea

Starting Materials: Intermediate 1 and Intermediate 8.
¹H NMR (DMSO-d₆) 2.27 (s, 3H), 6.56 (bs, 4H), 6.65 (s, 1H), 7.27-7.33 (m, 1H), 7.36-7.44 (m, 2H), 7.74 (m, 1H), 7.83 (s, 1H), 9.08 (s, 1H), 10.35 (bs, 1H);
MS m/e MH⁺ 366.

### Example 17

### N-(3-{[({3-[(4,6-diaminopyrimidin-5-yl)ethynyl]phenyl}amino)carbonyl]amino}phenyl)acetamide

Starting Materials: Intermediate 1 and Intermediate 7.
¹H NMR (DMSO-d₆) 2.03 (s, 3H), 6.54 (bs, 4H), 7.14-7.19 (m, 3H), 7.23-7.34 (m, 2H), 7.39-7.44 (m, 1H), 7.69 (s, 1H), 7.77 (s, 1H), 7.83 (s, 1H), 8.57 (s, 1H), 8.74 (s, 1H), 9.88 (s, 1H); MS m/e MH⁺ 402.

### Example 18

### N-{3-[(4,6-diaminopyrimidin-5-yl)ethynyl]phenyl}-N'-[4-(trifluoromethyl)pyridin-2-yl]urea

Starting Materials: Intermediate 1 and Intermediate 6.
¹H NMR (DMSO-d₆) 6.57 (bs, 4H), 7.28-7.41 (m, 3H), 7.47-7.52 (m, 1H), 7.79 (s, 1H), 7.84 (s, 1H), 8.04 (s, 1H), 8.52-8.55 (d, 1H), 9.76 (s, 1H), 9.78 (s, 1H);
MS m/e MH⁺ 414.

Examples 19 and 20 were prepared by an analogous method to Example 16, except purification was carried out by trituration with ether.

### Example 19

### N-{3-[(4,6-diaminopyrimidin-5-yl)ethynyl]phenyl}-N'-(3-methylisoxazol-5-yl)urea

Starting Materials: Intermediate 1 and Intermediate 4.
¹H NMR (DMSO-d₆) 2.16 (s, 3H), 5.95 (s, 1H), 6.56 (bs, 4H), 7.27-7.33 (m, 1H), 7.37-7.45 (m, 2H), 7.71 (s, 1H), 7.83 (s, 1H), 8.80 (s, 1H), 10.11 (bs, 1H);
MS m/e MH⁺ 350.

### Example 20

### N-(5-tert-butyl-1,3,4-thiadiazol-2-yl)-N'-{3-[(4,6-diaminopyrimidin-5-yl)ethynyl]phenyl}urea

Starting Materials: Intermediate 1 and Intermediate 3.
¹H NMR (DMSO-d₆) 1.38 (s, 9H), 6.56 (bs, 4H), 7.27-7.33 (m, 1H), 7.36-7.41 (m, 1H), 7.43-7.49 (m, 1H), 7.78 (s, 1H), 7.83 (s, 1H), 9.06 (s, 1H);
MS m/e MH⁺ 409.

### Example 21

### N-(5-tert-butylisoxazol-3-yl)-N'-{3-[(4,6-diaminopyrimidin-5-yl)ethynyl]phenyl}urea

5-[(3-Aminophenyl)ethynyl]pyrimidine-4,6-diamine (Intermediate 1) (0.494 g), triethylamine (0.4 mL) and phenyl (5-tert-butylisoxazol-3-yl)carbamate (Intermediate 5) (0.69 g) in THF (20 mL) were refluxed for 4 hr. The reaction mixture was concentrated *in vacuo,* purification by flash chromatography on silica using 1-10% MeOH in DCM as eluent to give the title compound as a beige solid (558 mg, 65%);
¹H NMR (DMSO-d₆) 1.29 (s, 9H), 6.49 (s, 1H), 6.57 (bs, 4H), 7.25-7.32 (m, 1H), 7.34-7.39 (m, 1H), 7.40-7.45 (m, 1H), 7.72 (s, 1H), 7.83 (s, 1H), 8.79 (s, 1H), 9.56 (s, 1H);
MS m/e MH⁺ 409.

### Example 22

### N-{3-[(4,6-diaminopyrimidin-5-yl)ethynyl]phenyl}-N'-(2,3-dihydro-1,4-benzodioxin-6-yl)urea

5-[(3-Aminophenyl)ethynyl]pyrimidine-4,6-diamine (Intermediate 1) (50 mg), sodium carbonate (141 mg), N-(trichloroacetyl)-3,4 ethylenedioxyaniline (Intermediate 9) (79 mg) in DMF (2 mL) were heated at 100°C for 5 days. Purification by RPHPLC (H2O:MeCN 0-70%) gave the title compound as a beige solid (8 mg, 9%);
¹H NMR (DMSO-d₆) 4.14-4.24 (m, 4H), 6.75-6.79 (m, 2H), 7.07-7.10 (d, 1H), 7.26-7.39 (m, 3H), 7.82 (s, 1H), 7.86 (bs, 4H), 8.16 (s, 1H), 8.56 (s, 1H), 8.66 (s, 1H);
MS m/e MH⁺ 403.

### Intermediate 9

### 2,2,2-Trichloro-N-(2,3-dihydro-1,4-benzodioxin-6-yl)acetamide

Trichloroacetyl chloride (12.3 mL) was added dropwise over 10 min to a cooled (ice bath), stirred, solution of 3,4-ethylenedioxyaniline (15.12 g) in EtOAc (150 mL) under an inert atmosphere. A purple precipitate was observed which redissolved on further stirring at ambient temperature. After 4 hours, the reaction mixture was concentrated *in vacuo* and recrystallised from ethanol to give the title compound as a solid (25.79 g, 87%);
¹H NMR (DMSO-d₆) 4.22 (s, 4H), 6.83-6.87 (m, 1H), 7.07-7.13 (m, 1H), 7.19 (s, 1H), 10.59 (bs, 1H);
MS m/e MH⁺ 296.

### Intermediate 10

### 2,2,2-Trichloro-N-(2-morpholin-4-ylphenyl)acetamide

Intermediate 10 was prepared by an analogous method to intermediate 9 by using N-(2-aminophenyl)morpholine in place of 3,4-ethylenedioxyaniline:
¹H NMR (DMSO-d₆) 2.81-2.89 (m, 4H), 3.70-3.78 (m, 4H), 7.18-7.25 (m, 2H), 7.27-7.40 (m, 1H), 7.85-7.92 (m, 1H), 10.21 (bs, 1H);
MS m/e MH⁺ 323.

### Example 23

### N-{3-[(4,6-diaminopyrimidin-5-yl)ethynyl]phenyl}-N'-(2-morpholin-4-ylphenyl)urea

Example 23 was prepared by an analogous method to Example 22 but using Intermediate 10 in place of Intermediate 9.
¹H NMR (DMSO-d₆) 2.76-2.83 (m, 4H), 3.80-3.88 (m, 4H), 6.95-7.02 (m, 1H), 7.03-7.10 (m, 1H), 7.16-7.21 (m, 1H), 7.31-7.49 (m, 3H), 7.87-7.90 (s, 1H), 7.99-8.10 (m, 4H), 8.17 (s, 1H), 8.20 (s, 1H), 9.59 (s, 1H);
MS m/e MH⁺ 430.

### Example 24

### N-{3-[(4,6-diaminopyrimidin-5-yl)ethynyl]phenyl}-N'-(1-methylpiperidin-4-yl)urea

Phenyl {3-[(4,6-diaminopyrimidin-5-yl)ethynyl]phenyl}carbamate (Example 10) (50 mg), 1-methylpiperidin-4-amine (34 mg) and triethylamine (44 mg) in THF (2 mL) were heated at reflux under an inert atmosphere for 18 hours. The solvent was evaporated and the residue was diluted with diethyl ether (10 mL). The product was filtered and dried *in vacuo* to afford the title compound as a beige solid (41 mg, 77%);
¹H NMR (DMSO-d₆) 1.36-1.46 (m, 2H), 1.79-1.83 (m, 2H), 1.98-2.06 (m, 2H), 2.17 (s, 3H), 2.63-2.67 (m, 2H), 3.44-3.48 (m, 1H), 6.18 (m, 1H), 6.53 (s, 4H), 7.20-7.27 (m, 2H), 7.34-7.38 (m, 1H), 7.63 (s, 1H), 7.86 (s, 1H), 8.32 (s, 1H);
MS m/e MH⁺ 366.

### Example 25

### N-{3-[(4,6-diaminopyrimidin-5-yl)ethynyl]phenyl}-N'-(1-propylpiperidin-4-yl)urea

Example 25 was prepared by an analogous method to Example 24 by using 1-propylpiperidin-4-amine in place of 1-methylpiperidin-4-amine.
¹H NMR (DMSO-d₆) 0.87 (t, 3H), 1.37-1.48 (m, 4H), 1.76-1.83 (m, 2H), 1.99-2.06 (m, 2H), 2.23 (t, 2H), 2.71-2.75 (m, 2H), 3.41-3.52 (m, 1H), 6.17 (d, 1H), 6.52 (s, 4H), 7.19-7.26 (m, 2H), 7.34-7.38 (m, 1H), 7.63 (s, 1H), 7.85 (s, 1H), 8.34 (s, 1H);
MS m/e MH⁺ 394.

### Example 26

### N-{3-[(4,6-diaminopyrimidin-5-yl)ethynyl]phenyl}-2-phenylacetamide

Phenylacetyl chloride (55.7 mg) and pyridine (47.5 mg) were added to a stirred solution of 5-[(3-aminophenyl)ethynyl]pyrimidine-4,6-diamine (intermediate 1)(67.6 mg) in THF. After 60 min, the reaction mixture was concentrated to give a yellow solid. Purification by flash chromatography on silica using 1-5% (7M ammonia in MeOH) in DCM as eluent to give the title compound as a yellow solid (23 mg, 23%);
¹H NMR (DMSO-d₆) 3.66 (s, 2H), 6.55 (bs, 4H), 7.26-7.42 (m, 8H), 7.52-7.55 (m, 1H), 7.86 (s, 1H), 10.20 (bs, 1H);
MS m/e MH⁺ 344.

### Example 27

### N-{3-[(4,6-diaminopyrimidin-5-yl)ethynyl]phenyl}-2-(2-methoxyphenyl)acetamide

A solution of 2-methoxyphenylacetic acid (50 mg) in DMF (1 mL) was added to HATU (120 mg) and polymer supported DIPEA (Argonaut Technologies, 3.9 mmolg⁻¹, 300 mg). The mixture was shaken for 5 min. A solution of 5-[(3-aminophenyl)ethynyl]pyrimidine-4,6-diamine (intermediate 1) (68 mg) in DMF (1 mL) was added, and agitation continued overnight. The resin was removed by filtration and washed with DCM (15 mL). The filtrate was evaporated and the residue partitioned between EtOAc (15 mL) and water (10 mL). The organics were washed with further water (10 mL) and brine (4 x 10 mL), dried over anhydrous MgSO₄ and evaporated *in vacuo.* Trituration with DCM gave the title compound as a solid (63 mg, 56%);
¹H NMR (DMSO-d₆) 3.63 (s, 2H), 3.77 (s, 3H), 6.51 (bs, 4H) 6.89 (t, 1H), 6.97 (d, 2H), 7.21 (d, 2H), 7.28 (t, 1H), 7.37 (d, 1H), 7.51 (s, 1H), 7.81-7.86 (m, 2H), 10.04, s, 1H);
MS m/e MH⁺ 374.
Examples 28 to 30 were prepared by an analogous method to Example 27 by using the appropriate acid in place of 2-methoxyphenylacetic acid.

### Example 28

### N-{3-[(4,6-diaminopyrimidin-5-yl)ethynyl]phenyl}-2-[3-(trifluoromethyl)phenyl]acetamide

Starting Materials: Intermediate 1 and 3-trifluoromethylphenylacetic acid.
¹H NMR (DMSO-d₆) 3.78 (s, 2H), 6.52 (bs, 4H), 7.29 (t, 1H), 7.40 (d, 1H), 7.49 (d, 1H), 7.53-7.66 (m, 4H), 7.69 (s, 1H), 7.81-7.87 (m, 2H), 10.25 (s, 1H);
MS m/e MH⁺ 412.

### Example 29

### N-{3-[(4,6-diaminopyrimidin-5-yl)ethynyl]phenyl}-2-[4-(trifluoromethyl)phenyl]acetamide

Starting Materials: Intermediate 1 and 4-trifluoromethylphenylacetic acid.
¹H NMR (DMSO-d₆) 3.77 (s, 2H), 6.44-6.63 (bs, 4H), 7.29 (t, 1H), 7.39 (d, 1H), 7.50 (d, 1H), 7.55 (d, 2H), 7.70 (d, 2H), 7.83 (s, 2H), 10.25 (s, 1H);
MS m/e MH⁺ 412.

### Example 30

### N-{3-[(4,6-diaminopyrimidin-5-yl)ethynyl]phenyl}-2-(3-methoxyphenyl)acetamide

Starting Materials: Intermediate 1 and 3-methoxyphenylacetic acid.
¹H NMR (DMSO-d₆) 3.61 (s, 2H), 3.73 (s, 3H), 6.78-6.95 (m, 6H), 7.19-7.33 (m, 2H), 7.38 (d, 1H), 7.87 (s, 1H), 7.90 (s, 1H), 10.30 (s, 1H);
MS m/e MH⁺ 374.

### Example 31

### N-(5-tert-butylisoxazol-3-yl)-N'-{3-[(4-chloropyrimidin-5-yl)ethynyl]phenyl}urea

A solution of *N*-(5-*tert*-butylisoxazol-3-yl)-*N'*-{3-[(4-chloropyrimidin-5-yl)ethynyl]phenyl}urea (Intermediate 12) (195 mg), HCl in ether (1.0M, 0.1 mL), liquid ammonia (3 mL) and THF (10 mL) was irradiated in a microwave (130°C, 600W) for 4 hours. The reaction mixture was concentrated *in vacuo* then purified by RPHPLC (H₂O:MeCN, 10-90%) to give the title compound as a colourless solid (11 mg);
¹H NMR (DMSO-d₆) 9.61 (s, 1H), 8.95 (s, 1H), 8.57 (s, 1H), 8.50 (s, 1H), 7.86 (s, 1H), 7.26-7.44 (m, 3H), 6.48 (s, 1H), 1.29 (s, 9H);
MS m/e MH⁺ 377.

### Intermediate 11

### N-(5-tert-butylisoxazol-3-yl)-N'-(3-ethynylphenyl)urea

A solution of 5-*tert*-butylisoxazol-3-amine (1.4 g) and di-succinimidyl carbonate (2.56 g) in MeCN (30 mL) was heated to 80°C for 4 hours. The solution was cooled to ambient temperature, 3-ethynylaniline (1.17 g) added and the mixture heated for a further 16 hours at 80°C. The reaction mixture was cooled to room temperature and concentrated *in vacuo.* The residue was partitioned between ether and water, the organic phase washed with water and then concentrated *in vacuo.* Trituration from isohexane gave the title compound as a colourless solid (1.57 g);
¹H NMR (DMSO-d₆) 9.53 (s, 1H), 8.87 (s, 1H), 7.65 (d, 1H), 7.29 (t, 1H), 7.11 (d, 1H), 6.49 (s, 1H), 4.14 (s, 1H), 1.28 (s, 9H);
MS m/e MH⁺ 284.

### Intermediate 12

### N-(5-tert-butylisoxazol-3-yl)-N'-{3-[(4-chloropyrimidin-5-yl)ethynyl]phenyl}urea

Bis(triphenylphosphine)palladium dichloride (170 mg), copper(I) iodide (12.5 mg) and triethylamine (10 mL) were added to a degassed solution of 4-chloro-5-iodopyrimidine (Chem.Pharm.Bull. 1986, 34(7), 2719-2724) (2.40 g) and *N*-(5-*tert-*butylisoxazol-3-yl)-*N*'-(3-ethynylphenyl)urea (Intermediate 11) (3.1 g) in DMF (50 mL) and then heated at 50°C for 1.5 hours. The reaction mixture was concentrated, water added and then extracted into DCM. The organic layer was washed with water then brine and concentrated *in vacuo* to give the title compound as a brown foam (4.50 g) which was used without further purification.
MS m/e MH⁺ 396 (³⁵Cl), 398 (³⁷Cl).

### Example 32

### N-(5-tert-butylisoxazol-3-yl)-N'-(3-{[4-(methylamino)pyrimidin-5-yl]ethynyl}phenyl)urea

Example 32 was prepared using an analogous method to Example 31 by using methylamine in place of ammonia.
¹H NMR (DMSO-d₆) 9.61 (s, 1H), 8.97 (s, 1H), 8.66 (s, 1H), 8.47 (s, 1H), 8.25 (s, br, 1H), 7.86 (s, 1H), 7.31-7.61 (m, 3H), 6.48 (s, 1H), 2.98 (d, 3H), 1.28 (s, 9H);
MS m/e MH⁺ 391.

### Example 33

### N-(5-tert-butylisoxazol-3-yl)-N'-{3-[(4-{[3-(isopropylamino)propyl]amino}pyrimidin-5-yl)ethynyl]phenyl}urea

A solution of *N*-(5-*tert*-butylisoxazol-3-yl)-*N'*-{3-[(4-chloropyrimidin-5-yl)ethynyl]phenyl}urea (Intermediate 12) (200 mg), HCl (1.0M in ether) (0.1 mL) and *N-*isopropylpropane-1,3-diamine (0.14 mL) were heated in MeCN (5.0 mL) at 50°C for 3 hours. The reaction mixture was concentrated *in vacuo,* NaHCO₃ (50% saturated aq.) added and the mixture extracted into DCM, washed with water and concentrated *in vacuo.* Purification by flash chromatography on silica (0-10% (7N NH₃ in MeOH) in DCM) gave the title compound as a yellow solid (72 mg);
¹H NMR (DMSO-d₆) 9.41 (s, br, 1H), 8.52 (s, 1H), 8.29 (s, 1H), 7.79 (s, 1H), 7.50 (d, 1H), 7.31 (t, 1H), 7.23 (d, 1H), 6.38 (t, 1H), 5.89 (s, 1H), 3.63 (q, 1H), 2.71-2.81 (m, 3H), 1.82 (p, 2H), 1.35 (s, 9H), 1.00 (d, 6H);
MS m/e MH⁺ 476.

Examples 34 and 35 were prepared by an analogous method to Example 33 by using the appropriate amine in place of *N*-isopropylpropane-1,3-diamine and with further purification by RPHPLC.

### Example 34

### N-(5-tert-butylisoxazol-3-yl)-N'-[3-({4-[(2-pyrrolidin-1-ylethyl)amino]pyrimidin-5-yl}ethynyl)phenyl]urea

Starting Materials: Intermediate 12 and 1-(2-aminoethyl)pyrrolidine.
¹H NMR (DMSO-d₆) 9.68 (s, 1H), 9.51 (s, br, 1H), 9.06 (s, 1H), 8.62 (s, 1H), 8.58 (s, 1H), 7.87 (s, 1H), 7.71 (s, br, 1H), 7.28-7.40 (m, 3H), 6.47 (s, 1H), 3.80 (q, 1H), 3.60-3.70 (m, 2H), 3.39 (dd, 2H), 3.00-3.15 (m, 2H), 1.94-2.06 (m, 2H), 1.80-1.92 (m, 2H), 1.28 (s, 9H); MS m/e MH⁺ 474.

### Example 35

### N-(5-tert-butylisoxazol-3-yl)-N'-[3-({4-[(5-tert-butylisoxazol-3-yl)amino]pyrimidin-5-yl}ethynyl)phenyl]urea

Starting Materials: Intermediate 12 and 3-amino-5-*tert*-butylisoxazole.
¹H NMR (DMSO-d₆) 10.01 (s, 1H), 9.57 (s, 1H), 8.91 (s, 1H), 8.69 (s, 1H), 8.64 (s, 1H), 7.86 (s, 1H), 7.33-7.45 (m, 3H), 6.73 (s, 1H), 6.50 (s, 1H), 1.32 (s, 9H), 1.29 (s, 9H);
MS m/e MH⁺ 500.

The following Examples were made in a similar way to Example 31 by using the appropriate amine in place of ammonia.

### Example 36

### N-(5-tert-butylisoxazol-3-yl)-N'-{3-[(4-{[3-(dimethylamino)propyl]amino}pyrimidin-5-yl)ethynyl]phenyl}urea

SM: *N*-(5-*tert*-butylisoxazol-3-yl)-*N'*-{3-[(4-chloropyrimidin-5-yl)ethynyl]phenyl}urea (Intermediate 12), *N,N*-dimethylpropylamine. Purification by silica gel chromatography using 0-10% (10% aq NH₃ in MeOH) in DCM as eluent, followed by RPHPLC (H2O:MeCN 0-70%).
¹H NMR (DMSO-d₆) 1.28 (s, 9H), 1.88-2.03 (m, 2H), 2.49 (s, 6H), 3.02-3.18 (m, 2H), 3.48-3.62 (m, 2H), 6.47 (s, 1H), 7.27-7.46 (m, 3H), 7.89 (s, 1H), 8.23 (t, 1H), 8.50 (s, 1H), 8.64 (s, 1H), 9.30 (s, 1H), 9.62 (br, 1H), 9.89 (s, 1H);
MS m/e MH⁺ 462.

### Example 37

### N-(5-tert-butylisoxazol-3-yl)-N'-[3-({4-[(2-hydroxyethyl)amino]pyrimidin-5-yl}ethynyl)phenyl]urea

SM: *N*-(5-*tert*-butylisoxazol-3-yl)-*N*'-{3-[(4-chloropyrimidin-5-yl)ethynyl]phenyl}urea (Intermediate 12), ethanolamine. Purification by silica gel chromatography using 0-10% (10% aq NH₃ in MeOH) in DCM as eluent, followed by RPHPLC (H2O:MeCN 0-70%).
¹H NMR (DMSO-d₆) 1.29 (s, 9H), 3.55-3.61 (m, 4H), 6.48 (s, 1H), 7.29-7.48 (m, 3H), 7.85 (s, 1H), 8.19 (s, br, 1H), 8.45 (s, 1H), 8.64 (s, 1H), 8.98 (s, 1H), 9.61 (s, 1H);
MS m/e MH⁺ 421.

### Example 38

### N-(5-tert-butylisoxazol-3-yl)-N'-[3-({4-[(2-morpholin-4-ylethyl)amino]pyrimidin-5-yl}ethynyl)phenyl]urea

SM: *N*-(5-*tert*-butylisoxazol-3-yl)-*N'*-{3-[(4-chloropyrimidin-5-yl)ethynyl]phenyl}urea (Intermediate 12), 2-morpholin-4-ylethanamine. Purification by silica gel chromatography using 0-10% (10% aq NH₃ in MeOH) in DCM as eluent.
¹H NMR (DMSO-d₆) 1.29 (s, 9H), 2.40-2.57 (m, 6H), 3.50-3.59 (m, 6H), 6.50 (s, 1H), 7.08 (t, 1H), 7.24-7.42 (m, 3H), 7.83 (s, 1H), 8.32 (s, 1H), 8.46 (s, 1H), 8.90 (s, 1H), 9.56 (s, 1H);
MS m/e MH⁺ 490.

### Example 39

### N-[3-({4-[(4-aminobutyl)amino]pyrimidin-5-yl}ethynyl)phenyl]-N'-(5-tert-butylisoxazol-3-yl)urea

SM: *N*-(5-*tert*-butylisoxazol-3-yl)-*N*'-{3-[(4-chloropyrimidin-5-yl)ethynyl]phenyl}urea (Intermediate 12), 1,4-butanediamine. Purification by silica gel chromatography using 0-10% (10% aq NH₃ in MeOH) in DCM as eluent.
¹H NMR (CDCl₃) 1.29 (s, 9H), 1.33-1.45 (m, 2H), 1.53-1.66 (m, 2H), 2.57 (t, 2H), 3.37-3.49 (m, 2H), 6.49 (s, 1H), 7.30 (d, 1H), 7.35 (d, 1H), 7.38-7.46 (m, 2H), 7.78 (s, 1H), 8.29 (s, 1H), 8.43 (s, 1H);
MS m/e MH⁺ 448.

### Example 40

### N-(5-tert-butylisoxazol-3-yl)-N'-[3-({4-[(3-pyrrolidin-1-ylpropyl)amino]pyrimidin-5-yl}ethynyl)phenyl]urea

SM: *N*-(5-*tert*-butylisoxazol-3-yl)-*N*'-{3-[(4-chloropyrimidin-5-yl)ethynyl]phenyl} urea (Intermediate 12), 3-pyrrolidin-1-ylpropan-1-amine. Purification by silica gel chromatography twice using 0-10% (1% aq NH₃, 10% MeOH in DCM) in DCM and then 20% EtOH in EtOAc as eluent.
¹H NMR (DMSO-d₆) 1.29 (s, 9H), 1.57-1.64 (m, 4H), 1.68-1.79 (m, 2H), 2.37-2.57 (m, 6H), 3.49 (q, 2H), 6.49 (s, 1H), 7.25-7.44 (m, 4H), 7.78 (s, 1H), 8.29 (s, 1H), 8.44 (s, 1H), 8.90 (s, 1H), 9.56 (s, 1H);
MS m/e MH⁺ 488.

### Example 41

### N-(5-tert-butylisoxazol-3-yl)-N'-[3-({4-[(2,4-dimethoxybenzyl)amino]pyrimidin-5-yl}ethynyl)phenyl]urea

SM: *N*-(5-*tert*-butylisoxazol-3-yl)-*N*'-{3-[(4-chloropyrimidin-5-yl)ethynyl]phenyl} urea (Intermediate 12), 2,4-dimethoxybenzylamine. Purification by silica gel chromatography twice using 0-10% (10% aq NH₃ in MeOH) in DCM and then 20% EtOAc in isohexane as eluent.
¹H NMR (DMSO-d₆) 1.29 (s, 9H), 3.71 (s, 3H), 3.81 (s, 3H), 4.57 (d, 2H), 6.45 (d, 1H), 6.49 (s, 1H), 6.57 (d, 1H), 7.00 (d, 1H), 7.30 (d, 1H), 7.36 (t, 1H), 7.41 (d, 1H), 7.54 (t, 1H), 7.60 (s, 1H), 8.35 (s, 1H), 8.41 (s, 1H), 8.90 (s, 1H), 9.56 (s, 1H);
MS m/e MH⁺ 527.

### Example 42

### N-[3-({4-[(2-aminoethyl)amino]pyrimidin-5-yl}ethynyl)phenyl]-N'-(5-tert-butylisoxazol-3-yl)urea

SM: *N*-(5-*tert*-butylisoxazol-3-yl)-*N'*-{3-[(4-chloropyrimidin-5-yl)ethynyl]phenyl} urea (Intermediate 12), 1,2-diaminoethane. Purification by silica gel chromatography using 0-10% (10% aq NH₃ in MeOH) in DCM as eluent, followed by trituration from ether and DCM.
¹H NMR (DMSO-d₆) 1.29 (s, 9H), 2.75 (t, 2H), 3.36-3.49 (m, 2H), 6.49 (d, 1H), 6.49 (s, 1H), 7.21-7.46 (m, 3H), 7.77 (s, 1H), 8.30 (s, 1H), 8.43 (s, 1H), 8.94 (s, 1H), 9.56 (s, 1H);
MS m/e MH⁺ 420.

### Example 43

### N-(5-tert-butylisoxazol-3-yl)-N'-{3-[(4-{[2-(dimethylamino)ethyl]amino}pyrimidin-5-yl)ethynyl]phenyl}urea

SM: *N*-(5-*tert*-butylisoxazol-3-yl)-*N*'-{3-[(4-chloropyrimidin-5-yl)ethynyl]phenyl}urea (Intermediate 12), *N,N*-dimethyl-1,2-diaminoethane. Purification as for Example 42.
¹H NMR (DMSO-d₆) 1.29 (s, 9H), 2.12-2.37 (m, 8H), 3.41-3.57 (m, 2H), 6.48 (s, 1H), 7.17 (t, 1H), 7.26-7.46 (m, 3H), 7.79 (s, 1H), 8.31 (s, 1H), 8.45 (s, 1H), 8.91 (s, 1H), 9.57 (s, 1H);
MS m/e MH⁺ 448.

### Example 44

### N-(5-tert-butylisoxazol-3-yl)-N'-{3-[(4-{[4-(dimethylamino)butyl]amino}pyrimidin-5-yl)ethynyl]phenyl}urea

SM: *N*-(5-*tert*-butylisoxazol-3-yl)-*N'*-{3-[(4-chloropyrimidin-5-yl)ethynyl)phenyl}urea (Intermediate 12), *N,N*-dimethyl-1,4-diaminobutane. Purification as for Example 42.
¹H NMR (DMSO-d₆) 1.29 (s, 9H), 1.44-1.64 (m, 4H), 2.38 (s, 6H), 2.50-2.61 (m, 2H), 3.36-3.54 (m, 2H), 6.48 (s, 1H), 7.24-7.42 (m, 4H), 7.80 (s, 1H), 8.30 (s, 1H), 8.44 (s, 1H), 8.92 (s, 1H), 9.58 (s, 1H);
MS m/e MW 476.

### Example 45

### N-(5-tert-butylisoxazol-3-yl)-N'-[3-({4-[N-{2-(dimethylamino)ethyl}-N-methyl-amino]pyrimidin-5-yl}ethynyl)phenyl]urea

SM: *N*-(5-*tert*-butylisoxazol-3-yl)-*N*'-{3-[(4-chloropyrimidin-5-yl)ethynyl]phenyl}urea (Intermediate 12), *1,1,2*-trimethyl-1,2-diaminoethane. Purification as for Example 42.
¹H NMR (DMSO-d₆) 1.29 (s, 9H), 2.48 (s, 6H), 2.57-2.70 (m, 2H), 3.28 (s, 3H), 4.00 (t, 2H), 6.48 (s, 1H), 7.18 (d, 1H), 7.33-7.42 (m, 2H), 7.73 (s, 1H), 8.44 (s, 1H), 8.50 (s, 1H), 9.04 (s, 1H), 9.59 (s, 1H);
MS m/e MH⁺ 462.

### Example 46

### N-(5-tert-butylisoxazol-3-yl)-N'-[3-({4-[(2-piperidin-1-ylethyl)amino]pyrinudin-5-yl}ethynyl)phenyl]urea

SM: *N*-(5-*tert*-butylisoxazol-3-yl)-*N*'-{3-[(4-chloropyrimidin-5-yl)ethynyl]phenyl}urea (Intermediate 12), 2-piperidin-1-ylethanamine. Purification as for Example 42.
¹H NMR (DMSO-d₆) 1.29 (s, 9H), 1.30-1.80 (m, 6H), 2.39-2.60 (m, 2H), 2.82-3.03 (m, 2H), 3.46-3.85 (m, 4H), 6.47 (s, 1H), 7.25-7.51 (m, 4H), 7.85 (s, 1H), 8.39 (s, 1H), 8.51 (s, 1H), 8.92 (s, 1H), 9.58 (s, 1H);
MS m/e MH⁺ 488.

### Example 47

### N-(5-tert-butylisoxazol-3-yl)-N'-[3-({4-[(3-morpholin-4-ylpropyl)amino]pyrimidin-5-yl}ethynyl)phenyl]urea

SM: *N*-(5-*tert*-butylisoxazol-3-yl)-*N'*-{3-[(4-chloropyrimidin-5-yl)ethynyl]phenyl}urea (Intermediate 12), 3-morpholin-4-ylpropan-1-amine. Purification as for Example 42.
¹H NMR (DMSO-d₆) 1.29 (s, 9H), 1.85-2.04 (m, 2H), 2.45-2.52 (m, 4H), 3.00-3.15 (m, 2H), 3.22-3.70 (m, 6H), 6.47 (s, 1H), 7.27-7.51 (m, 4H), 7.63 (s, 1H), 8.35 (s, 1H), 8.47 (s, 1H), 8.92 (s, 1H), 9.57 (s, 1H);
MS m/e MH⁺ 504.

### Example 48

### N-(5-tert-butylisoxazol-3-yl)-N'-[3-({4-[(3-piperidin-1-ylpropyl)amino]pyrimidin-5-yl}ethynyl)phenyl]urea

SM: *N*-(5-*tert*-butylisoxazol-3-yl)-*N'*-{3-[(4-chloropyrimidin-5-yl)ethynyl]phenyl}urea (Intermediate 12), 3-piperidin-1-ylpropan-1-amine. Purification as for Example 42.
¹H NMR (DMSO-d₆) 1.29 (s, 9H), 1.48-2.05 (m, 8H), 2.67-3.62 (m; 8H), 6.47 (s, 1H), 7.25-7.48 (m, 4H), 7.83 (s, 1H), 8.35 (s, 1H), 8.47 (s, 1H), 8.92 (s, 1H), 9.57 (s, 1H);
MS m/e MH⁺ 502.

### Example 49

### N-(5-tert-butylisoxazol-3-yl)-N'-{3-[(4-{[3-(4-methylpiperazin-1-yl)propyl]amino}pyrimidin-5-yl)ethynyl]phenyl}urea

SM: *N*-(5-*tert*-butylisoxazol-3-yl)-*N'*-{3-[(4-chloropyrimidin-5-yl)ethynyl]phenyl }urea (Intermediate 12), 3-(4-methylpiperazin-1-yl)propan-1-amine. Purification as for Example 42.
¹H NMR (DMSO-d₆) 1.29 (s, 9H), 1.68-1.84 (m, 2H), 2.22-2.78 (m, 13H), 3.41-3.56 (m, 2H), 6.49 (s, 1H), 7.26-7.46 (m, 4H), 7.79 (s, 1H), 8.31 (s, 1H), 8.44 (s, 1H), 8.94 (s, 1H), 9.58 (s, 1H);
MS m/e MH⁺ 516

### Example 50

### N-(3-tert-butyl-1-methyl-1H-pyrazol-5-yl)-N'-[3-({4-[(2-morpholin-4-ylethyl)amino]pyrimidin-5-yl}ethynyl)phenyl]urea.

The solution of N-(3-tert-butyl-1-methyl-1H-pyrazol-5-yl)-N'-[3-({4-[(2-morpholin-4-ylethyl)amino]pyrimidin-5-yl}ethynyl)phenyl]urea (Intermediate 15) in THF (6 mL), 2-morpholin-4-ylethanamine (0.18 mL) and 2.0 M HCl in ether (1 drop) were heated together at 45°C for 16 hours. After cooling to ambient temperature silica gel was added and the solvent removed *in vacuo.* Purification by silica gel chromatography using 0-10% (10% aq NH₃ in MeOH) in DCM as eluent, followed by RPHPLC (H2O:MeCN 0-70%), then treatment with aqueous Na₂CO₃ and collection by filtration gave the title compound as a colourless solid (35 mg);
¹H NMR (DMSO-d₆) 1.20 (s, 9H), 2.42 (t, 4H), 2.53 (t, 2H), 3.50-3.58 (m, 6H), 3.59 (s, 3H), 6.05 (s, 1H), 7.08 (t, 1H), 7.25 (d, 1H), 7.31-7.40 (m, 2H), 7.84 (s, 1H), 8.31 (s, 1H), 8.45 (s, 1H), 8.53 (s, 1H), 8.98 (s, 1H);
MS m/e MH⁺ 503.

### Intermediate 13

### phenyl 3-ethynylphenylcarbamate

Phenyl chloroformate was added dropwise to a solution of 3-ethynylaniline (20.0 mL) and pyridine (24.1 mL) in THF (300 mL) at 0°C. The resultant solution was stirred at 0°C for 3.5 hours. Pyridine (7.5 mL) was added, followed by additional phenyl chloroformate (6 mL) dropwise. Water (50 mL) was added and the THF removed *in vacuo.* The resultant aqueous suspension was extracted into ether, washed with 1M HCl, water and brine and concentrated *in vacuo* to give the title compound as a pale brown solid (27.5 g, 78%).
¹H NMR (CDCl₃) 3.06 (s, 1H), 7.00 (s, br, 1H), 7.14--7.31 (m, 5H), 7.35-7.51 (m, 3H), 7.56 (s, 1H);
MS m/e (M-H)- 236.

### Intermediate 14

### N-(3-tert-butyl-1-methyl-1H-pyrazol-5-yl)-N'-(3-ethynylphenyl)urea.

A solution of 3-*tert*-butyl-1-methyl-1*H*-pyrazol-5-amine (792 mg), phenyl 3-ethynylphenylcarbamate (Intermediate 13) (1.19 g) and triethylamine (1.4 mL) in THF (30 mL) was heated to 60°C for 24 hours. Additional triethylamine (0.5 mL) and 3-*tert*-butyl-1-methyl-1*H*-pyrazol-5-amine (200 mg) were added and the mixture heated for a further 4 hours and concentrated *in vacuo.* The residue was partitioned between DCM and 50% saturated aqueous Na₂CO₃ solution. The aquous phase was extracted with DCM and the combined organics washed with 50% saturated aqueous Na₂CO₃ solution, water and brine and concentrated *in vacuo.* Purification by flash chromatography on silica using 0-5% MeOH in DCM as eluent, followed by 20-50% EtOAc in DCM gave the title compound as a white solid (725 mg, 49%).
¹H NMR (CDCl₃) 1.30 (s, 9H), 3.08 (s, 1H), 3.75 (s, 3H), 6.11 (s, 1H), 6.63 (s, 1H), 6.84 (s, 1H), 7.19-7.30 (m, 2H), 7.40 (dt, 1H), 7.46 (s, 1H);
MS m/e MH⁺ 297.

### Intermediate 15

### N-(3-tert-butyl-1-methyl-1H-pyrazol-5-yl)-N'-[3-({4-chloropyrimidin-5-yl}ethynyl)phenyl]urea

Triethylamine (5.0 mL) was added to a degassed solution of 4-chloro-5-iodopyrimidine (Chem.Pharm.Bull. 1986, 34(7), 2719-2724) (590 mg), *N*-(3-*tert*-butyl-1-methyl-1*H*-pyrazol-5-yl)-*N'*-(3-prop-1-ynylphenyl)urea (Intermediate 14) (725 mg) PdCl₂(PPh₃)₂ (40 mg) and cuprous iodide (3 mg) in DMF (12.5 mL). The mixture was heated to 60°C for 6.5 hours and concentrated *in vacuo.* The residue was dissolved in THF (18 mL) and used without further purification.
MS m/e MH⁺ 409,411.

**The following Example was made in a similar way to Example 21:**

### Example 51

### N-{3-[(4,6-diaminopyrimidin-5-yl)ethynyl]phenyl}-N'-(2,3-dihydro-1H-inden-1-yl)urea

SM: Phenyl {3-[(4,6-diaminopyrimidin-5-yl)ethynyl]phenyl}carbamate (Example 10), 1-Indanamine
¹H NMR (DMSO-d₆) 1.74 -1.86 (m, 1H), 2.43 - 2.53 (m, 1H), 2.75 - 3.01 (m, 2H), 5.19 (q, 1H), 6.54 (s, 4H), 6.59 (d, 1H), 7.22 - 7.32 (m, 6H), 7.40 - 7.44 (m, 1H), 7.69 (s, 1H), 7.86 (s, 1H), 8.40 (s, 1H);
MS m/e MH⁺ 385.

## Claims

1. A compound of the Formula I: 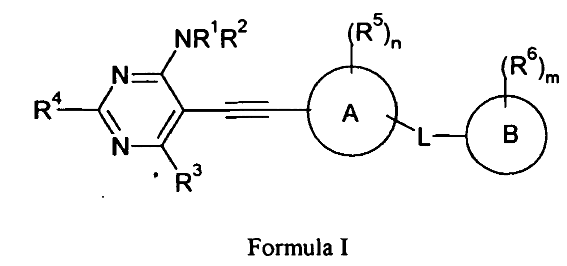 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6 , or a 5 or 6 membered heteroaryl ring, or **R¹** and **R²** together with the nitrogen atom to which they are attached represent a saturated or partially saturated 3 to 7 membered heterocyclic ring optionally containing another heteroatom selected from N or O;
wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring,
wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more hydroxy groups;
wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups independently selected from hydroxy, amino, mono(1-6C)alkylamino or di-[(1-6C)alkyl]amino;
and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino or di-[(1-6C)alkyl]amino, or a saturated or partially saturated 3 to 7 membered heterocyclic ring, or -C(O)(CH₂)_{z}Y wherein z is 0,1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
and provided that when R¹ and/or R² is a (1C)alkanoyl group, then the (1C)alkanoyl is not substituted by fluoro or hydroxy;
**R³** is selected from hydrogen, (1-6C)alkyl or (1-6C)alkoxy, (1-6C)alkoxy wherein the alkyl and the alkoxy groups are optionally substituted by one or more groups selected from: fluoro, hydroxy, (1-6C)alkyl, (1-6C)alkoxy, carbamoyl, mono(1-6C)alkylcarbamoyl or di-[(1-6C)alkyl]carbamoyl, amino, mono(1-6C)alkylamino or di(1-6C)alkylamino, a saturated or partially saturated 3 to 7 membered heterocyclic ring or a 5 or 6 membered heteroaryl ring wherein said heterocyclic and heteroaryl rings are optionally independently substituted by one or more of the following: (1-4C)alkyl, (1-4C)alkoxy,hydroxy, amino, mono(1-6C)alkylamino or di(1-6C)alkylamino or a saturated or partially saturated 3 to 7 membered heterocyclic ring;
or **R³** represents a group **-NR¹R²** as defined above;
**R⁴** is selected from hydrogen, (1-6C)alkyl or (1-6C)alkoxy;
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O) O-, or -OC(O) -N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
**R⁶** is selected from halo, cyano, oxo, a (3-7C)cycloalkyl ring, a saturated or partially saturated 3 to 7 membered heterocyclic ring -S(O)ₚ-(1-6C)alkyl wherein p is 0, 1 or 2, -N(R^{a})C(O)(1-6C)alkyl in which **R^{a}** is hydrogen or (1-6C)alkyl; or
**R⁶** is selected from (1-6C)alkyl or (1-6C)alkoxy, wherein the (1-6C)alkyl, -S(O)ₚ-(1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; wherein the (3-7C)cycloalkyl ring and saturated or partially saturated 3 to 7 membered heterocyclic ring are optionally independently substituted by one or more groups selected from (1-6C)alkyl;
and
**m** is 0, 1, 2 or 3;
and when B is a (3-7C)cycloalkyl ring or a saturated or partially saturated 3 to 7 membered heterocyclic ring or a saturated or partially saturated 8, 9 or 10 membered bicyclic group, the rings and bicyclic group optionally bear 1 or 2 oxo or thioxo substituents;
and salts thereof.

2. A compound of Formula I according to Claim 1, wherein:
**R⁶** is selected from halo, cyano, a (3-7C)cycloalkyl ring, a saturated or partially saturated 3 to 7 membered heterocyclic ring or an alkanoylamino group--N(R^{c})C(O)(1-6C)alkyl in which R^{c} is hydrogen or (1-6C)alkyl; or **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
and salts thereof.

3. A compound of the Formula I according to claim 1, wherein
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, or (3-6C)cycloalkyl(CH₂)ₓ- in which x is 0, 1, 2, 3, 4, 5 or 6, or **R¹** and **R²** together with the nitrogen atom to which they are attached represent a saturated or partially saturated 3 to 7 membered heterocyclic ring optionally containing another heteroatom selected from N or O;
wherein the alkyl and the cycloalkyl groups are optionally substituted by one or more groups selected from fluoro, hydroxy, (1-6C)alkyl, (1-6C)alkoxy, amino, mono(1-6C)alkylamino or di-[(1-6C)alkyl]amino, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, hydroxy, amino, mono(1-6C)alkylamino or di-[(1-6C)alkyl]amino or a saturated or partially saturated 3 to 7 membered heterocyclic ring;
and wherein the phenyl is optionally substituted by one or more groups selected from halo, (1-6C)alkyl, (1-6C)alkoxy, amino, mono(1-6C)alkylamino or di-[(1-6C)alkyl]amino, wherein the (1-6C)alkyl or (1-6C)alkoxy are optionally substituted by hydroxy, amino, mono(1-6C)alkylamino or di-[(1-6C)alkyl]amino;
**R³** is selected from hydrogen, (1-6C)alkyl or (1-6C)alkoxy, wherein the alkyl and the alkoxy groups are optionally substituted by one or more groups selected from fluoro, hydroxy, (1-6C)alkyl, (1-6C)alkoxy, amino, mono(1-6C)alkylamino or di-[(1-6C)alkyl]amino, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, hydroxy, amino, mono(1-6C)alkylamino or di-[(1-6C)alkyl]amino or a saturated or partially saturated 3 to 7 membered heterocyclic ring;
or **R³** represents a group **-NR¹R²** as defined above;
**R⁴** is selected from hydrogen, (1-6C)alkyl or (1-6C)alkoxy;
**A** represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyano, halo, (1-6C)alkoxy or (1-6C)alkyl optionally substituted by cyano or by one or more fluoro;
**n** is 0, 1, 2 or 3;
L is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O)O-, or -OC(O) N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁶** is selected from halo, cyano, a saturated or partially saturated 3 to 7 membered heterocyclic ring or an alkanoylamino group -N(R^{a})C(O)(1-6C)alkyl in which **R^{a}** is hydrogen or (1-6C)alkyl; or **R⁶** is selected from (1-6C)alkyl or (1-6C)alkoxy, wherein the alkyl and the alkoxy groups are optionally substituted by one or more groups selected from cyano, fluoro, hydroxy, (1-6C)alkoxy, amino, mono(1-6C)alkylamino, di-[(1-6C)alkyl]amino, or a saturated or partially saturated 3 to 7 membered heterocyclic ring; and
m is 0, 1, 2 or 3;
and when m is at least 2 then two substituents on adjacent carbon atoms in ring B may together represent a methylenedioxy group;
and salts thereof.

4. A compound according to any one of Claims 1, 2 and 3 wherein A is selected from phenyl, pyridyl, thiazolyl, thiadiazolyl or pyrimidinyl.

5. A compound accordingly to any one of the preceding claims wherein B is selected from phenyl, 2,3-di-hydro-indenyl, piperidinyl, pyridyl, pyrazolyl, isothiazolyl, thiadiazolyl, isoxazolyl, benzodioxinyl, benzodioxolyl or tetrahydropyranyl

6. A compound accordingly to any one of the preceding claims wherein L is selected from -N(R⁸)C(O)N(R⁹)-, -N(R⁸)C(O)O- or -N(R⁸)C(O)CH₂- wherein R⁸ and R⁹ independently represent hydrogen or (1-6C)alkyl.

7. A compound accordingly to any one of the preceding claims wherein R¹ and R² are both hydrogen or R¹ is hydrogen or (1-6C)alkyl and R² is (1-6C)alkyl
wherein (1-6Calkyl) is optionally substituted by hydroxy, amino, mono(1-6C)alkylamino or di(1-6C)alkylamino, carbamoyl, (1-6C)alkoxy, (1-6C)alkoxy(1-6C)alkoxy, -N(R^{d})C(O)(1-6C)alkyl in which R^{d} is hydrogen or (1-6C)alkyl, aryl (particularily phenyl), a saturated or partially saturated 3 to 7 membered heterocyclic ring or a 5 or 6 membered heteroaryl ring;
wherein the (1-6C)alkoxy, mono(1-6C)alkylamino and -N(R^{d})C(O)(1-6C)alkyl groups are optionally substituted by hydroxy; and
wherein an aryl ring, a saturated or partially saturated 3 to 7 membered heterocyclic ring or a 5 or 6 membered heteroaryl ring is optionally substituted by (1-4C)alkyl, (1-4C)alkoxy or -C(O)CH₂Y wherein Y is selected from hydroxy or di(1-6C)alkylamino.

8. A compound accordingly to any one of the preceding claims wherein R³ and R⁴ are both hydrogen.

9. A compound accordingly to any one of the preceding claims wherein R⁶ is independently selected from halo, cyano, oxo, (3-7C)cycloalkyl, a saturated 3 to 7 membered heterocyclic ring (optionally substituted by (1-4C)alkyl), -N(R^{C})C(O)(1-6C)alkyl wherein R^{c} is hydrogen or (1-6C)alkyl (particularily (1-4C)alkyl), (1-6C)alkyl (optionally substituted by halo) or (1-6C)alkoxy and m is selected from 1 or 2.

10. A compound according to Claim 1 selected from:
N-{3-[(4,6-diaminopyrimidin-5-yl)ethynyl]phenyl}-N-phenylurea;
N-(3-[(4,6-diaminopyrimidin-5-yl)ethynyl]phenyl)-2-phenylacetamide;
N-{3-[(4,6-diaminopyrimidin-5-yl)ethynyl]phenyl}-N'-(3,4-dichlorophenyl)urea;
N-{3-[(4,6-diaminopyrimidin-5-yl)ethynyl]phenyl}-N'-[2-(trifluoromethyl)phenyl]urea;
N-{3-[(4,6-diaminopyrimidin-5-yl)ethynyl]phenyl}-N'-[3-(trifluoromethyl)phenyl]urea;
N-{3-[(4,6-diaminopyrimidin-5-yl)ethynyl]phenyl}-N'-[4-(trifluoromethyl)phenyl]urea;
N-{4-[(4,6-diaminopyrimidin-5-yl)ethynyl]phenyl}-N'-[2-fluoro-5-(trifluoromethyl)phenyl]urea;
N-{3-[(4,6-diaminopyrimidin-5-yl)ethynyl]phenyl}-N'-(3-methoxyphenyl)urea;
Phenyl{4-[(4,6-diaminopyrimidin-5-yl)ethynyl]phenyl}carbamate;
Phenyl {3-[(4,6-diaminopyrimidin-5-yl)ethynyl]phenyl}carbamate;
N-(5-tert-butyl-1,3,4-thiadiazol-2-yl)-N'-{4-[(4,6-diaminopyrimidin-5-yl)ethynyl]phenyl}urea;
N-{4-[(4,6-diaminopyrimidin-5-yl)ethynyl]phenyl}-N'-(3-methylisothiazol-5-yl)urea;
N-{4-[(4,6-diaminopyrimidin-5-yl)ethynyl]phenyl }-N'-(3-methylisoxazol-5-yl)urea;
N-{4-[(4,6-diaminopyrimidin-5-yl)ethynyl]phenyl }-N'-[4-(trifluoromethyl)pyridin-2-yl]urea;
N-(3-{[({4-[(4,6-diaminopyrimidin-5-yl)ethynyl]phenyl}amino)carbonyl]amino}phenyl)acetamide;
N-{ 3-[(4,6-diaminopyrimidin-5-yl)ethynyl]phenyl }-N'-(3-methylisothiazol-5-yl)urea;
N-(3-{[({3-[(4,6-diaminopyrimidin-5-yl)ethynyl]phenyl}amino)carbonyl]amino}phenyl)acetamide;
N-{3-[(4,6-diaminopyrimidin-5-yl)ethynyl]phenyl}-N'-[4-(trifluoromethyl)pyridin-2-yl]urea;
N-{ 3-[(4,6-diaminopyrimidin-5-yl)ethynyl]phenyl }-N'-(3-methylisoxazol-5-yl)urea;
N-(5-tert-butyl-1,3,4-thiadiazol-2-yl)-N'-{3-[(4,6-diaminopyrimidin-5-yl)ethynyl]phenyl}urea;
N-(5-tert-butylisoxazol-3-yl)-N'-{3-[(4,6-diaminopyrimidin-5-yl)ethynyl]phenyl}urea;
N-{3-[(4,6-diaminopyrimidin-5-yl)ethynyl]phenyl}-N'-(2,3-dihydro-1,4-benzodioxin-6-yl)urea;
N-{3-[(4,6-diaminopyrimidin-5-yl)ethynyl]phenyl}-N'-(2-morpholin-4-ylphenyl)urea;
N-{3-[(4,6-diaminopyrimidin-5-yl)ethynyl]phenyl}-N'-(1-methylpiperidin-4-yl)urea;
N-{3-[(4,6-diaminopyrimidin-5-yl)ethynyl]phenyl}-N'-(1-propylpiperidin-4-yl)urea;
*N*-{3-[(4,6-diaminopyrimidin-5-yl)ethynyl]phenyl}-2-phenylacetamide;
N-{3-[(4,6-diaminopyrimidin-5-yl)ethynyl]phenyl}-2-(2-methoxyphenyl)acetamide;
N-{3-[(4,6-dtaminopyrimidin-5-yt)ethynyt]phenyl}-2-[3-(trifluoromethyl)phenyl]acetamide;
N-{3-[(4,6-diaminopyrimidin-5-yl)ethynyl]phenyl}-2-[4-(trifluoromethyl)phenyl]acetamide
N-(3-[(4,6-diaminopyrimidin-5-yl)ethynyl]phenyl)-2-(3-methoxyphenyl)acetamide;
*N*-(5-*tert*-butylisoxazol-3-yl)-N-{3-[(4-chloropyrimidin-5-yl)ethynyl]phenyl}urea;
*N*-(5-*tert*-butylisoxazol-3-yl)-*N*'-(3-{[4-(methylamino)pyrimidin-5-yl]ethynyl}phenyl)urea;
N-(5-tert-butylisoxazol-3-yl)-N'-{3-[(4-{[3-(isopropylamino)propyl]amino}pyrimidin-5-yl)ethynyl]phenyl}urea;
*N*-(5-*tert*-butylisoxazol-3-yl)-*N*'-[3-({4-[(2-pyrrolidin-1-ylethyl)amino]pyrimidin-5-yl}ethynyl)phenyl]urea;
*N*-(5-*tert*-butylisoxazol-3-yl)-*N*'-[3-({4-[(5-*tert*-butylisoxazol-3-yl)amino]pyrimidin-5-yl}ethynyl)phenyl]urea;
N-(S-tert-butylisoxazol-3-yl)-N'-{3-[(4-{[3-(dimethylamino)propyl]amino}pyrimidin-5-yl)ethynyl]phenyl}urea;
N-(5-tert-butylisoxazol-3-yl)-N'-[3-({4-[(2-hydroxyethyl)amino]pyrimidin-5-yl}ethynyl)phenyl]urea;
N-(5-tert-butylisoxazol-3-yl)-N'-[3-({4-[(2-morpholin-4-ylethyl)amino]pyrimidin-5-yl}ethynyl)phenyl]urea;
N-[3-({4-[(4-aminobutyl)amino]pyrimidin-5-yl}ethynyl)phenyl]-N'-(5-tert-butylisoxazol-3-yl)urea;
N-(S-tert-butylisoxazol-3-yl)-N'-[3-({4-[(3-pyrrolidin-1-ylpropyl)amino]pyrimidin-5-yl}ethynyl)phenyl]urea;
N-(5-tert-butylisoxazol-3-yl)-N'-[3-({4-[(2,4-dimethoxybenzyl)amino]pyrimidin-5-yl}ethynyl)phenyl]urea;
N-[3-({4-[(2-aminoethyl)amino]pyrimidin-5-yl}ethynyl)phenyl]-N'-(5-tert-butylisoxazol-3-yl)urea;
N-(5-tert-butylisoxazol-3-yl)-N'-{3-[(4-{[2-(dimethylamino)ethyl]amino}pyrimidin-5-yl)ethynyl]phenyl}urea;
N-(5-tert-butylisoxazol-3-yl)-N'-{3-[(4-{[4-(dimethylamino)butyl]amino}pyrimidin-5-yl)ethynyl]phenyl urea;
N-(5-tert-butylisoxazol-3-yl)-N'-[3-({4-[N-{2-(dimethylamino)ethyl}-N-methyl-amino]pyrimidin-5-yl}ethynyl)phenyl]urea;
N-(5-tert-butylisoxazol-3-yl)-N'-[3-({4-[(2-piperidin-1-ylethyl)amino]pyrimidin-5-yl}ethynyl)phenyl]urea;
N-(5-tert-butylisoxazol-3-yl)-N'-[3-({4-[(3-morpholin-4-ylpropyl)amino]pyrimidin-5-yl} ethynyl)phenyl]urea;
N-(5-tert-butylisoxazol-3-yl)-N'-[3-({4-[(3-piperidin-1-ylpropyl)amino]pyrimidin-5-yl}ethynyl)phenyl]urea;
N-(5-tert-butylisoxazol-3-yl)-N'-{3-[(4-{[3-(4-methylpiperazin-1-yl)propyl]amino}pyrimidin-5-yl)ethynyl]phenyl}urea;
N-(3-tert-butyl-1-methyl-1H-pyrazol-5-yl)-N'-[3-({4-[(2-morpholin-4-ylethyl)amino]pyrimidin-5-yl}ethynyl)phenyl]urea; or
N-{3-[(4,6-diaminopyrimidin-5-yl)ethynyl]phenyl}-N'-(2,3-dihydro-1H-inden-1-yl)urea
or a salt thereof.

11. A pharmaceutical composition which comprises a compound of the Formula I, or a pharmaceutically acceptable salt thereof, as defined in any one of claims 1 to 10 in association with a pharmaceutically acceptable diluent or carrier.

12. A compound of the Formula I, or a pharmaceutically acceptable salt thereof, as defined in any one of claims 1 to 10, for use as a medicament.

13. Use of a compound of the Formula I, or a pharmaceutically acceptable salt thereof, as defined in any one of claims 1 to 10, in the manufacture of a medicament for use in the treatment of a disease where Tie2 receptor tyrosine kinase inhibition is required in a warm-blooded animal such as man.

14. Use of a compound of the Formula I, or a pharmaceutically acceptable salt thereof, as defined in any one of claims I to 10, in the manufacture of a medicament for use in the treatment or prophylaxis of a disease associated with undesirable or pathological angiogenesis in a warm-blooded animal such as man.

15. A process for preparing a compound of formula I, as defined in Claim 1, or a pharmaceutically acceptable salt thereof (wherein R¹, R², R³, R⁴, R⁵, R⁶, R⁷, R⁸, R⁹, R¹⁰, R¹¹ L , ring A and ring B, n and m are, unless otherwise specified, as defined in Claim 1) comprising:
(a) For compounds of the formula I wherein L is -N(R⁸)C(O)N(H)-, the reaction of a compound of the formula II: 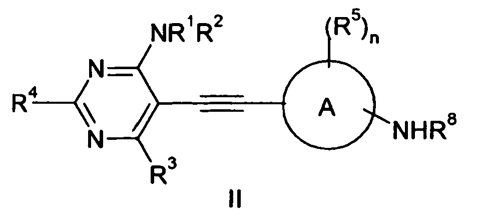 wherein R¹, R², R³, R⁴, R⁵, R⁸, n and A have any of the meanings defined hereinbefore except that any functional group is protected if necessary, with an isocyanate of the formula IV: 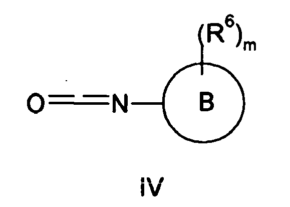 wherein R⁶, m and B have any of the meanings defined hereinbefore except that any functional group is protected if necessary; or
(b) For compounds of the formula I wherein L is -N(R⁸)C(O)N(H)-, the reaction of a compound of the formula II as defined above with an aryl carbamate of the formula III: 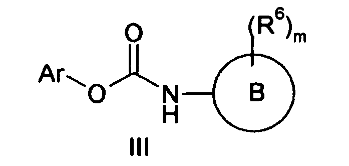 wherein Ar is a suitable aryl group, for example phenyl, and R⁶, m and B have any of the meanings defined hereinbefore except that any functional group is protected if necessary; or
(c) For compounds of the formula I wherein L is N(R⁸)C(O)-O-, the reaction of a compound of the formula II as defined above with a compound of the formula XI: 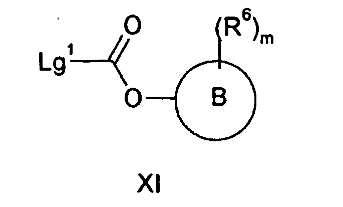 wherein Lg¹ is a suitable displaceable group for example halogeno (such as fluoro, chloro or bromo) and R⁶, m and B have any of the meanings defined hereinbefore except that any functional group is protected if necessary; or
(d) For compounds of the formula I wherein L is N(R⁸)C(O)C(R^{a}R^{b}), the reaction of a compound of the formula **II** as defined above with a compound of the formula IX: 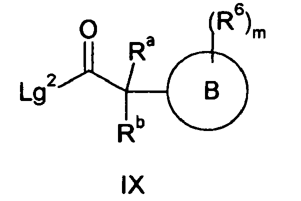 wherein Lg² is a suitable displaceable group for example hydroxy, halogeno (such as fluoro, chloro or bromo), R^{x}-C(O)-O- or R^{x}-O- (wherein R^{x} is a suitable alkyl or aryl group) and R⁶, R^{a}, R^{b}, m and B have any of the meanings defined hereinbefore except that any functional group is protected if necessary; or
(e) For compounds of the formula I wherein L is -N(R⁸)C(O)N(H)-, the reaction of a compound of the formula II as defined above with a trichloroacetylamine of the formula XIII: 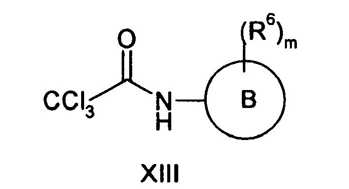 wherein R⁶, m and B have any of the meanings defined hereinbefore except that any functional group is protected if necessary; or
(f) For compounds of the formula I wherein L is -C(R^{a}R^{b})C(O)N(R⁹)-, the reaction of a compound of the formula XIV: 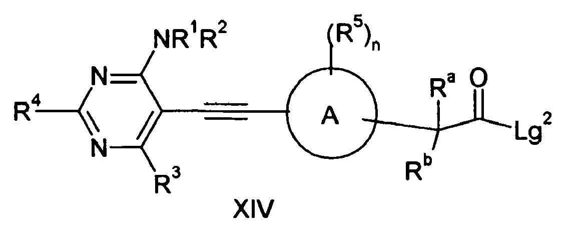 wherein Lg² is a suitable displaceable group as described above and R¹, R², R³, R⁴, R⁵, R^{a}, R^{b}, n and A have any of the meanings defined hereinbefore except that any functional group is protected if necessary, with an amine of the formula XV: 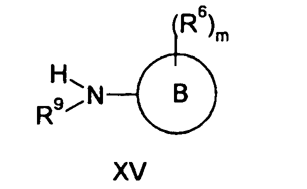 wherein R⁶, R⁹, m and B have any of the meanings defined hereinbefore except that any functional group is protected if necessary; or
(g) The reaction of a compound of the formula XVI: 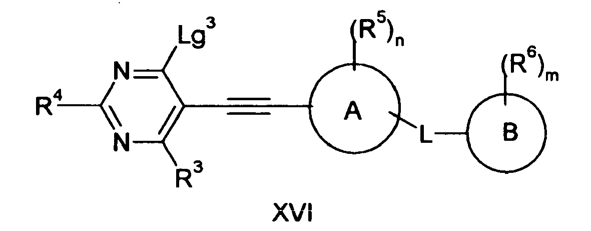 wherein Lg³ is a suitable displaceable group for example halogeno (such as fluoro, chloro, bromo or iodo), methyl sulfonyl, methylthio or aryloxy (such as phenoxy) and R³, R⁴, R⁵, R⁶, n, m, A, B and L have any of the meanings defined hereinbefore except that any functional group is protected if necessary, with an amine of the formula HNR¹R², wherein R¹ and R² have any of the meanings defined hereinbefore except that any functional group is protected if necessary; or
(h) The reaction of a compound of the formula XVII: 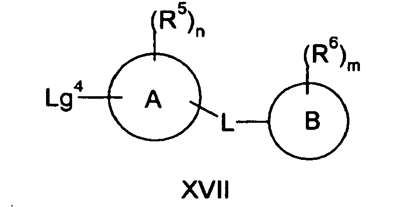 wherein Lg⁴ is a suitable displaceable group for example halogeno (such as chloro, bromo or iodo) or a sulfonyloxy group (such as trifluoromethylsulfonyloxy) and R⁵, R⁶, n, m, A, B and L have any of the meanings defined hereinbefore except that any functional group is protected if necessary, with an alkyne of the formula XVIII: 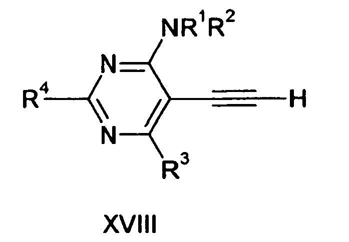 wherein R¹, R², R³ and R⁴ have any of the meanings defined hereinbefore except that any functional group is protected if necessary; or
(i) For compounds of the formula I wherein L is -N(H)C(O)N(R⁹)-, the reaction of an isocyanate of the formula XIX: 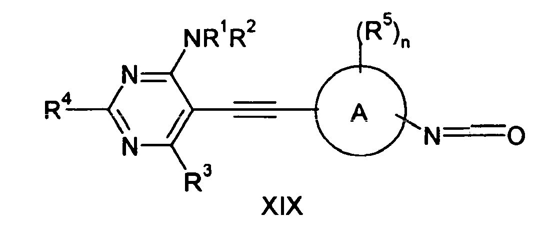 wherein R¹, R², R³, R⁴, R⁵, n and A have any of the meanings defined hereinbefore except that any functional group is protected if necessary, with an amine of the formula XV as defined above; or
(j) For compounds of the formula I wherein L is -N(H)C(O)N(R⁹)-, the reaction of a compound of the formula XX: 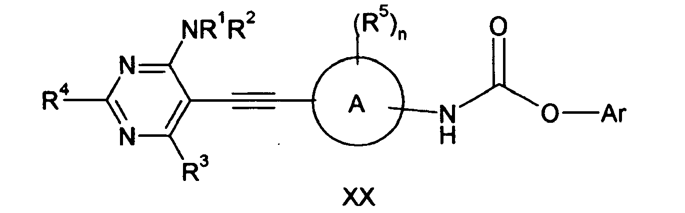 wherein Ar is a suitable aryl group, for example phenyl, and R¹, R², R³, R⁴, R⁵, n and A have any of the meanings defined hereinbefore except that any functional group is protected if necessary, with an amine of the formula XV as defined above.
and thereafter if necessary:
i) converting a compound of the Formula (I) into another compound of the Formula (I);
ii) removing any protecting groups;
iii) forming a salt.

16. A compound selected from Formulae II, XIV, XVI, XIX and XX as defined in Claim 15 or a compound of Formula VIc: 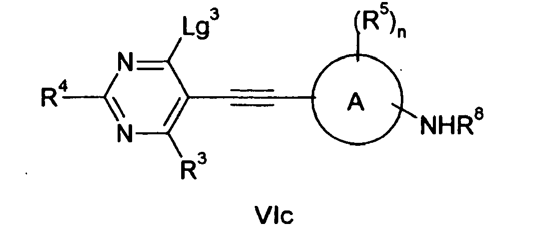 or salt thereof, wherein ring A, R¹, R², R³, R⁴, R⁵, R⁶, R⁸, L, m, ring B and n are as defined in claim 1, Ar is an aryl group, Lg² is hydroxyl, halogeno, R^{x}-C(O)-O- or R^{x}-O-(wherein R^{x} is a suitable alkyl or aryl group) and Lg³ is halogeno, methylsulphonyl, methylthio or aryloxy.

## Patentansprüche

1. Verbindung der Formel I 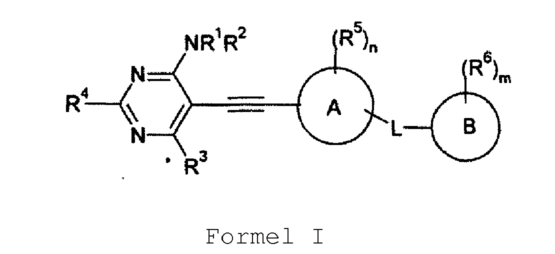 worin:
**R¹ und R²** unabhängig voneinander unter Wasserstoff, C₁₋₆-Alkylsulfonyl, Phenyl (CH₂)ᵤ, worin u für 0, 1, 2, 3, 4, 5 oder 6 steht, C₁₋₆-Alkanoyl, C₁₋₆-Alkyl, C₁₋₆-Alkoxycarbonyl, C₃₋₆-Cycloalkyl (CH₂)ₓ, worin x für 0, 1, 2, 3, 4, 5 oder 6 steht, oder einem 5- oder 6-gliedrigen Heteroarylring ausgewählt sind, oder **R¹ und R²** gemeinsam mit dem Stickstoffatom, an das sie gebunden sind, für einen gesättigten oder teilweise gesättigten 3- bis 7-gliedrigen heterocyclischen Ring, der gegebenenfalls ein unter N oder O ausgewähltes anderes Heteroatom enthält, stehen;
worin die C₁₋₆-Alkyl-, die C₁₋₆-Alkanoyl- und die C₃₋₆-Cycloalkylgruppen gegebenenfalls durch eine oder mehrere unabhängig voneinander unter Fluor, Hydroxy, C₁₋₆-Alkyl, C₁₋₆-Alkoxy, C₁₋₆-Alkoxy-C₁₋₆-alkoxy, C₁₋₆-Alkoxy-C₁₋₆-alkoxy-C₁₋₆-alkoxy, Amino, Mono-C₁₋₆-alkylamino, Di(C₁₋₆-alkyl)amino, Carbamoyl, Mono-C₁₋₆-alkylcarbamoyl, Di(C₁₋₆-alkyl)carbamoyl oder -N(R^{d})C(O)-C₁₋₆-Alkyl, worin R^{d} für Wasserstoff oder C₁₋₆-Alkyl steht, oder einem gesättigten oder teilweise gesättigten 3- bis 7-gliedrigen heterocyclischen Ring oder einem 5- oder 6-gliedrigen Heteroarylring ausgewählte Gruppen substituiert sind,
worin die C₁₋₆-Alkoxy-, C₁₋₆-Alkoxy-C₁₋₆-alkoxy- und C₁₋₆-Alkoxy-C₁₋₆-alkoxy-C₁₋₆-alkoxygruppen und die C₁₋₆-Alkylgruppen der Mono-C₁₋₆-alkylamino-, Di(C₁₋₆-alkyl)amino-, Mono-C₁₋₆-alkylcarbamoyl-, Di(C₁₋₆-alkyl)carbamoyl- und/oder -N(R^{d})C(O)-C₁₋₆-Alkylgruppen gegebenenfalls durch eine oder mehrere Hydroxygruppen substituiert sind;
worin das Phenyl gegebenenfalls durch eine oder mehrere unabhängig voneinander unter Halogen, C₁₋₆-Alkyl, C₁₋₆-Alkoxy, Amino, Mono-C₁₋₆-alkylamino oder Di (C₁₋₆-alkyl) amino ausgewählte Gruppen substituiert ist, worin die C₁₋₆-Alkyl- und C₁₋₆-Alkoxygruppen gegebenenfalls durch eine oder mehrere unabhängig voneinander unter Hydroxy, Amino, Mono-C₁₋₆-alkylamino oder Di-(C₁₋₆-alkyl)amino ausgewählte Gruppen substituiert sind;
und worin alle heterocyclischen Ringe und Heteroarylringe in R¹ und/oder R² gegebenenfalls unabhängig voneinander durch eine oder mehrere der folgenden Gruppen substituiert sind: C₁₋₄-Alkyl, C₁₋₄-Alkoxy, C₁₋₄-Alkoxy-C₁₋₄-alkyl, Hydroxy, Amino, Mono-C₁₋₆-alkylamino oder Di-(C₁₋₆-alkyl)amino oder einen gesättigten oder teilweise gesättigten 3- bis 7-gliedrigen heterocyclischen Ring oder -C(O)(CH₂)_{z}Y, worin z für 0, 1, 2 oder 3 steht und Y unter Wasserstoff, Hydroxy, C₁₋₄-Alkoxy, Amino, Mono-C₁₋₆-alkylamino, Di(C₁₋₆-alkyl)amino oder einem gesättigten oder teilweise gesättigten 3- bis 7-gliedrigen heterocyclischen Ring ausgewählt ist;
und mit der Maßgabe, daß dann, wenn R¹ und/oder R² für eine C₁-Alkanoylgruppe steht, das C₁-Alkanoyl nicht durch Fluor oder Hydroxy substituiert ist;
**R³** unter Wasserstoff, C₁₋₆-Alkyl oder C₁₋₆-Alkoxy ausgewählt ist, worin die Alkyl- und die Alkoxygruppen gegebenenfalls durch eine oder mehrere unter Fluor, Hydroxy, C₁₋₆-Alkyl, C₁₋₆-Alkoxy, Carbamoyl, Mono-C₁₋₆-alkylcarbamoyl oder Di(C₁₋₆-alkyl)carbamoyl, Amino, Mono-C₁₋₆-alkylamino oder Di(C₁₋₆-alkyl)amino, einem gesättigten oder teilweise gesättigten 3- bis 7-gliedrigen heterocyclischen Ring oder einem 5- oder 6-gliedrigen Heteroarylring ausgewählte Gruppen substituiert sind, worin die heterocyclischen Ringe und Heteroarylringe gegebenenfalls unabhängig voneinander durch eine oder mehrere der folgenden Gruppen substituiert sind: C₁₋₄-Alkyl, C₁_₄-Alkoxy, Hydroxy, Amino, Mono-C₁₋₆-alkylamino oder Di(C₁₋₆-alkyl)amino oder einen gesättigten oder teilweise gesättigten 3- bis 7-gliedrigen heterocyclischen Ring;
oder **R³** für eine Gruppe **-NR¹R²** gemäß obiger Definition steht;
**R⁴** unter Wasserstoff, C₁₋₆-Alkyl oder C₁₋₆-Alkoxy ausgewählt ist;
A für eine Arylgruppe oder einen unter Furyl, Pyrrolyl, Thienyl, Oxazolyl, Isoxazolyl, Imidazolyl, Pyrazolyl, Thiazolyl, Isothiazolyl, Oxadiazolyl, Thiadiazolyl, Triazolyl, Tetrazolyl, Pyridyl, Pyridazinyl, Pyrimidinyl, Pyrazinyl oder 1,3,5-Triazinyl ausgewählten 5- oder 6-gliedrigen Heteroarylring steht;
**R⁵** unter Cyclopropyl, Cyano, Halogen, C₁₋₆-Alkoxy oder C₁₋₆-Alkyl ausgewählt ist, worin die C₁₋₆-Alkyl- und die C₁₋₆-Alkoxygruppen gegebenenfalls durch Cyano oder ein oder mehrere Fluor substituiert sind;
**n** für 0, 1, 2 oder 3 steht;
**L** in meta- oder para-Stellung bezüglich des Anbindungspunkts der Ethinylgruppe an den Ring A gebunden ist und für -C(R^{a}R^{b})C(O)N(R⁹)-, -N(R⁸)C(O)C(R^{a}R^{b})-, -N(R⁸)C(O)N(R⁹)-, -N(R⁸)C(O)O- oder -OC(O)-N(R⁹)- steht, worin R⁸ und R⁹ unabhängig voneinander für Wasserstoff oder C₁₋₆-Alkyl stehen und worin R^{a} und R^{b} unabhängig voneinander für Wasserstoff oder C₁₋₆-Alkyl stehen oder R^{a} und R^{b} gemeinsam mit dem Kohlenstoffatom, an das sie gebunden sind, für C₃₋₆-Cycloalkyl stehen;
**B** für einen C₃₋₇-Cycloalkylring, einen gesättigten oder teilweise gesättigten 3- bis 7-gliedrigen heterocyclischen Ring, eine Arylgruppe, einen unter Furyl, Pyrrolyl, Thienyl, Oxazolyl, Isoxazolyl, Imidazolyl, Pyrazolyl, Thiazolyl, Isothiazolyl, Oxadiazolyl, Thiadiazolyl, Triazolyl, Tetrazolyl, Pyridyl, Pyridazinyl, Pyrimidinyl, Pyrazinyl oder 1,3,5-Triazinyl ausgewählten 5- oder 6-gliedrigen Heteroarylring oder eine 8-, 9- oder 10-gliedrige bicyclische Gruppe, die gegebenenfalls 1, 2, 3 oder 4 unabhängig voneinander unter N, O und S ausgewählte Heteroatome enthält und gesättigt, teilweise gesättigt oder aromatisch ist, steht;
**R⁶** unter Halogen, Cyano, Oxo, einem C₃₋₇-Cycloalkylring, einem gesättigten oder teilweise gesättigten 3- bis 7-gliedrigen heterocyclischen Ring, -S(O)ₚ-C₁₋₆-Alkyl, worin p für 0, 1 oder 2 steht, -N(R^{a})C(O)-C₁₋₆-Alkyl, worin R^{a} für Wasserstoff oder C₁₋₆-Alkyl steht, ausgewählt ist; oder
**R⁶** unter C₁₋₆-Alkyl oder C₁₋₆-Alkoxy ausgewählt ist, worin die C₁₋₆-Alkyl-, -S(O)ₚ-C₁₋₆-Alkyl- und die C₁₋₆-Alkoxygruppen gegebenenfalls durch eine oder mehrere unabhängig voneinander unter Cyano, Fluor, Hydroxy, C₁₋₆-Alkoxy, Amino, Mono-C₁₋₆-alkylamino, Di(C₁₋₆-alkyl)amino, einem C₃₋₇-Cycloalkylring oder einem gesättigten oder teilweise gesättigten 3- bis 7-gliedrigen heterocyclischen Ring ausgewählte Gruppen substituiert sind; worin der C₃₋₇-Cycloalkylring und der gesättigte oder teilweise gesättigte 3- bis 7-gliedrige heterocyclische Ring gegebenenfalls unabhängig voneinander durch eine oder mehrere unter C₁₋₆-Alkyl ausgewählte Gruppen substituiert sind; und
**m** für 0, 1, 2 oder 3 steht;
und dann, wenn B für einen C₃₋₇-Cycloalkylring oder einen gesättigten oder teilweise gesättigten 3- bis 7-gliedrigen heterocyclischen Ring oder eine gesättigte oder teilweise gesättigte 8-, 9- oder 10-gliedrige bicyclische Gruppe steht, die Ringe und die bicyclische Gruppe gegebenenfalls 1 oder 2 Oxo- oder Thioxosubstituenten tragen;
und Salze davon.

2. Verbindung der Formel I nach Anspruch 1, worin:
**R⁶** unter Halogen, Cyano, einem C₃₋₇-Cycloalkylring, einem gesättigten oder teilweise gesättigten 3- bis 7-gliedrigen heterocyclischen Ring oder einer Alkanoylaminogruppe -N (R^{c})C(O)-C₁₋₆-Alkyl, worin R^{c} für Wasserstoff oder C₁₋₆-Alkyl steht, ausgewählt ist; oder
**R⁶** unter C₁₋₆-Alkyl oder C₁₋₆-Alkoxy ausgewählt ist, worin die C₁₋₆-Alkyl- und die C₁₋₆-Alkoxygruppen gegebenenfalls durch eine oder mehrere unabhängig voneinander unter Cyano, Fluor, Hydroxy, C₁₋₆-Alkoxy, Amino, Mono-C₁₋₆-alkylamino, Di(C₁₋₆-alkyl)amino, einem C₃₋₇-Cycloalkylring oder einem gesättigten oder teilweise gesättigten 3- bis 7-gliedrigen heterocyclischen Ring ausgewählte Gruppen substituiert sind; und Salze davon.

3. Verbindung der Formel I nach Anspruch 1, worin:
**R¹ und R²** unabhängig voneinander unter Wasserstoff, C₁₋₆-Alkylsulfonyl, Phenyl(CH₂)ᵤ, worin u für 0, 1, 2, 3, 4, 5 oder 6 steht, C₁₋₆-Alkanoyl, C₁₋₆-Alkyl, C₁₋₆-Alkoxycarbonyl oder C₃₋₆-Cycloalkyl(CH₂)ₓ, worin x für 0, 1, 2, 3, 4, 5 oder 6 steht, ausgewählt sind, oder
**R¹ und R²** gemeinsam mit dem Stickstoffatom, an das sie gebunden sind, für einen gesättigten oder teilweise gesättigten 3- bis 7-gliedrigen heterocyclischen Ring, der gegebenenfalls ein unter N oder O ausgewähltes anderes Heteroatom enthält, stehen;
worin die Alkyl- und die Cycloalkylgruppen gegebenenfalls durch eine oder mehrere unabhängig voneinander unter Fluor, Hydroxy, C₁₋₆-Alkyl, C₁₋₆-Alkoxy, Amino, Mono-C₁₋₆-alkylamino oder Di(C₁₋₆-alkyl)amino, einem gesättigten oder teilweise gesättigten 3- bis 7-gliedrigen heterocyclischen Ring oder einem 5- oder 6-gliedrigen Heteroarylring ausgewählte Gruppen substituiert sind, worin die heterocyclischen Ringe und Heteroarylringe gegebenenfalls unabhängig voneinander durch eine oder mehrere der folgenden Gruppen substituiert sind: C₁₋₄-Alkyl, Hydroxy, Amino, Mono-C₁₋₆-alkylamino oder Di (C₁₋₆-alkyl) amino oder einen gesättigten oder teilweise gesättigten 3- bis 7-gliedrigen heterocyclischen Ring;
und worin das Phenyl gegebenenfalls durch eine oder mehrere unter Halogen, C₁₋₆-Alkyl, C₁₋₆-Alkoxy, Amino, Mono-C₁₋₆-alkylamino oder Di(C₁₋₆-alkyl)amino ausgewählte Gruppen substituiert ist, worin die C₁₋₆-Alkyl- oder C₁₋₆-Alkoxygruppen gegebenenfalls durch Hydroxy, Amino, Mono-C₁₋₆-alkylamino oder Di(C₁₋₆-alkyl)amino substituiert sind;
**R³** unter Wasserstoff, C₁₋₆-Alkyl oder C₁₋₆-Alkoxy ausgewählt ist, worin die Alkyl- und die Alkoxygruppen gegebenenfalls durch eine oder mehrere unter Fluor, Hydroxy, C₁₋₆-Alkyl, C₁₋₆-Alkoxy, Amino, Mono-C₁₋₆-alkylamino oder Di(C₁₋₆-alkyl)amino, einem gesättigten oder teilweise gesättigten 3- bis 7-gliedrigen heterocyclischen Ring oder einem 5- oder 6-gliedrigen Heteroarylring ausgewählte Gruppen substituiert sind, worin die heterocyclischen Ringe und Heteroarylringe gegebenenfalls unabhängig voneinander durch eine oder mehrere der folgenden Gruppen substituiert sind: C₁₋₄-Alkyl, Hydroxy, Amino, Mono-C₁₋₆-alkylamino oder Di(C₁₋₆-alkyl)amino oder einen gesättigten oder teilweise gesättigten 3- bis 7-gliedrigen heterocyclischen Ring; oder **R³** für eine Gruppe **-NR¹R²** gemäß obiger Definition steht;
**R⁴** unter Wasserstoff, C₁₋₆-Alkyl oder C₁₋₆-Alkoxy ausgewählt ist;
**A** für eine Arylgruppe oder einen unter Furyl, Pyrrolyl, Thienyl, Oxazolyl, Isoxazolyl, Imidazolyl, Pyrazolyl, Thiazolyl, Isothiazolyl, Oxadiazolyl, Thiadiazolyl, Triazolyl, Tetrazolyl, Pyridyl, Pyridazinyl, Pyrimidinyl, Pyrazinyl oder 1,3,5-Triazinyl ausgewählten 5- oder 6-gliedrigen Heteroarylring steht;
**R⁵** unter Cyano, Halogen, C₁₋₆-Alkoxy oder C₁₋₆-Alkyl, gegebenenfalls substituiert durch Cyano oder ein oder mehrere Fluor, ausgewählt ist;
**n** für 0, 1, 2 oder 3 steht;
**L** in meta- oder para-Stellung bezüglich des Anbindungspunkts der Ethinylgruppe an den Ring A gebunden ist und für -C(R^{a}R^{b})C(O)N(R⁹)-, -N(R⁸)C(O)C(R^{a}R^{b})-, -N(R⁸)C(O)N(R⁹)-, -N(R⁸)C(O)O- oder -OC(O)N(R⁹)- steht, worin R⁸ und R⁹ unabhängig voneinander für Wasserstoff oder C₁₋₆-Alkyl stehen und worin R^{a} und R^{b} unabhängig voneinander für Wasserstoff oder C₁₋₆-Alkyl stehen oder R^{a} und R^{b} gemeinsam mit dem Kohlenstoffatom, an das sie gebunden sind, für C₃₋₆-Cycloalkyl stehen;
**B** für einen C₃₋₇-Cycloalkylring, eine Arylgruppe oder einen unter Furyl, Pyrrolyl, Thienyl, Oxazolyl, Isoxazolyl, Imidazolyl, Pyrazolyl, Thiazolyl, Isothiazolyl, Oxadiazolyl, Thiadiazolyl, Triazolyl, Tetrazolyl, Pyridyl, Pyridazinyl, Pyrimidinyl, Pyrazinyl oder 1,3,5-Triazinyl ausgewählten 5- oder 6-gliedrigen Heteroarylring steht;
**R⁶** unter Halogen, Cyano, einem gesättigten oder teilweise gesättigten 3- bis 7-gliedrigen heterocyclischen Ring oder einer Alkanoylaminogruppe -N (R^{a})C(O)-C₁₋₆-Alkyl, worin R^{a} für Wasserstoff oder C₁₋₆-Alkyl steht, ausgewählt ist; oder **R⁶** unter C₁₋₆-Alkyl oder C₁₋₆-Alkoxy ausgewählt ist, worin die Alkyl- und die Alkoxygruppen gegebenenfalls durch eine oder mehrere unter Cyano, Fluor, Hydroxy, C₁₋₆-Alkoxy, Amino, Mono-C₁₋₆-alkylamino, Di(C₁₋₆-alkyl)amino oder einem gesättigten oder teilweise gesättigten 3- bis 7-gliedrigen heterocyclischen Ring ausgewählte Gruppen substituiert sind; und
**m** für 0, 1, 2 oder 3 steht;
und dann, wenn m mindestens für 2 steht, zwei Substituenten an benachbaren Kohlenstoffatomen im Ring B gemeinsam für eine Methylendioxygruppe stehen können;
und Salze davon.

4. Verbindung nach einem der Ansprüche 1, 2 und 3, in der A unter Phenyl, Pyridyl, Thiazolyl, Thiadiazolyl oder Pyrimidinyl ausgewählt ist.

5. Verbindung nach einem der vorhergehenden Ansprüche, in der B unter Phenyl, 2,3-Dihydroindenyl, Piperidinyl, Pyridyl, Pyrazolyl, Isothiazolyl, Thiadiazolyl, Isoxazolyl, Benzodioxinyl, Benzodioxolyl oder Tetrahydropyranyl ausgewählt ist.

6. Verbindung nach einem der vorhergehenden Ansprüche, in der L unter -N(R⁸)C(O)N(R⁹)-, -N(R⁸)C(O)O- oder -N(R⁸)C(O)CH₂-, worin R⁸ und R⁹ unabhängig voneinander für Wasserstoff oder C₁₋₆-Alkyl stehen, ausgewählt ist.

7. Verbindung nach einem der vorhergehenden Ansprüche, in der R¹ und R² beide für Wasserstoff stehen oder R¹ für Wasserstoff oder C₁₋₆-Alkyl steht und R² für C₁₋₆-Alkyl steht,
worin C₁₋₆-Alkyl gegebenenfalls durch Hydroxy, Amino, Mono-C₁₋₆-alkylamino oder Di(C₁₋₆-alkyl)amino, Carbamoyl, C₁₋₆-Alkoxy, C₁₋₆-Alkoxy-C₁₋₆-alkoxy, -N(R^{d})C(O)-C₁₋₆-Alkyl, worin R^{d} für Wasserstoff oder C₁₋₆-Alkyl steht, Aryl (insbesondere Phenyl), einen gesättigten oder teilweise gesättigten 3- bis 7-gliedrigen heterocyclischen Ring oder einen 5- oder 6-gliedrigen Heteroarylring substituiert ist;
worin die C₁₋₆-Alkoxy-, Mono-C₁₋₆-alkylamino- und -N (R^{d}) C (O) -C₁₋₆-Alkyl-Gruppen gegebenenfalls durch Hydroxy substituiert sind; und
worin ein Arylring, ein gesättigter oder teilweise gesättigter 3- bis 7-gliedriger heterocyclischer Ring oder ein 5- oder 6-gliedriger Heteroarylring gegebenenfalls durch C₁₋₄-Alkyl, C₁₋₄-Alkoxy oder -C(O)CH₂Y, worin Y unter Hydroxy oder Di-C₁₋₆-alkylamino ausgewählt ist, substituiert ist.

8. Verbindung nach einem der vorhergehenden Ansprüche, in der R³ und R⁴ beide für Wasserstoff stehen.

9. Verbindung nach einem der vorhergehenden Ansprüche, in der R⁶ unabhängig voneinander unter Halogen, Cyano, Oxo, C₃₋₇-Cycloalkyl, einem gesättigten 3- bis 7-gliedrigen heterocyclischen Ring (gegebenenfalls substituiert durch C₁₋₄-Alkyl), -N (R^{c})C(O)-C₁₋₆-Alkyl, worin R^{c} für Wasserstoff oder C₁₋₆-Alkyl (insbesondere C₁₋₄-Alkyl) steht, C₁₋₆-Alkyl (gegebenenfalls substituiert durch Halogen) oder C₁₋₆-Alkoxy ausgewählt ist und m unter 1 und 2 ausgewählt ist.

10. Verbindung nach Anspruch 1, ausgewählt unter:
N-{3-[(4,6-Diaminopyrimidin-5-yl)ethinyl]phenyl}-N'-phenylharnstoff;
N-{3-[(4,6-Diaminopyrimidin-5-yl)ethinyl]phenyl}-2-phenylacetamid;
N-{3-[(4,6-Diaminopyrimidin-5-yl)ethinyl]phenyl}-N'-(3,4-dichlorphenyl)harnstoff;
N-{3-[(4,6-Diaminopyrimidin-5-yl)ethinyl]phenyl}-N'-[2-(trifluormethyl)phenyl]harnstoff;
N-{3-[(4,6-Diaminopyrimidin-5-yl)ethinyl]phenyl}-N'-[3-(trifluormethyl)phenyl]harnstoff;
N-{3-[(4,6-Diaminopyrimidin-5-yl)ethinyl]phenyl}-N'-[4-(trifluormethyl)phenyl]harnstoff;
N-{4-[(4,6-Diaminopyrimidin-5-yl)ethinyl]phenyl}-N'-[2-fluor-5-(trifluormethyl)phenyl]harnstoff;
N-{3-[(4,6-Diaminopyrimidin-5-yl)ethinyl]phenyl}-N'-(3-methoxyphenyl)harnstoff;
{4-[(4,6-Diaminopyrimidin-5-yl)ethinyl]phenyl}-carbamidsäurephenylester;
{3-[(4,6-Diaminopyrimidin-5-yl)ethinyl]phenyl}-carbamidsäurephenylester;
N-(5-tert-Butyl-1,3,4-thiadiazol-2-yl)-N'-{4-[(4,6-diaminopyrimidin-5-yl)ethinyl]phenyl}harnstoff;
N-{4-[(4,6-Diaminopyrimidin-5-yl)ethinyl]phenyl}-N'-(3-methylisothiazol-5-yl)harnstoff;
N-{4-[(4,6-Diaminopyrimidin-5-yl)ethinyl]phenyl}-N'-(3-methylisoxazol-5-yl)harnstoff;
N-{4-[(4,6-Diaminopyrimidin-5-yl)ethinyl]phenyl}-N'-[4-(trifluormethyl)pyridin-2-yl]harnstoff;
N-(3-{[({4-[(4,6-Diaminopyrimidin-5-yl)ethinyl]-phenyl}amino)carbonyl]amino}phenyl)acetamid;
N-{3-[(4,6-Diaminopyrimidin-5-yl)ethinyl]phenyl}-N'-(3-methylisothiazol-5-yl)harnstoff;
N-(3-{[({3-[(4,6-Diaminopyrimidin-5-yl)ethinyl]-phenyl}amino)carbonyl]amino}phenyl)acetamid;
N-{3-[(4,6-Diaminopyrimidin-5-yl)ethinyl]phenyl}-N'-[4-(trifluormethyl)pyridin-2-yl]harnstoff;
N-{3-[(4,6-Diaminopyrimidin-5-yl)ethinyl]phenyl}-N'-(3-methylisoxazol-5-yl)harnstoff;
N-(5-tert-Butyl-1,3,4-thiadiazol-2-yl)-N'-{3-[(4,6-diaminopyrimidin-5-yl)ethinyl]phenyl}harnstoff;
N-(5-tert-Butylisoxazol-3-yl)-N'-{3-[(4,6-diaminopyrimidin-5-yl)ethinyl]phenyl}harnstoff;
N-{3-[(4,6-Diaminopyrimidin-5-yl)ethinyl]phenyl}-N'-(2,3-dihydro-1,4-benzodioxin-6-yl)harnstoff;
N-{3-[(4,6-Diaminopyrimidin-5-yl)ethinyl]phenyl}-N'-(2-morpholin-4-ylphenyl)harnstoff;
N-{3-[(4,6-Diaminopyrimidin-5-yl)ethinyl]phenyl}-N'-(1-methylpiperidin-4-yl)harnstoff;
N-{3-[(4,6-Diaminopyrimidin-5-yl)ethinyl]phenyl}-N'-(1-propylpiperidin-4-yl)harnstoff;
N-{3-[(4,6-Diaminopyrimidin-5-yl)ethinyl]phenyl}-2-phenylacetamid;
N-{3-[(4,6-Diaminopyrimidin-5-yl)ethinyl]phenyl}-2-(2-methoxyphenyl)acetamid;
N-{3-[(4,6-Diaminopyrimidin-5-yl)ethinyl]phenyl}-2-[(3-trifluormethyl)phenyl]acetamid;
N-{3-[(4,6-Diaminopyrimidin-5-yl)ethinyl]phenyl}-2-[(4-trifluormethyl)phenyl]acetamid;
N-{3-[(4,6-Diaminopyrimidin-5-yl)ethinyl]phenyl}-2-(3-methoxyphenyl)acetamid;
N-(5-tert-Butylisoxazol-3-yl)-N'-{3-[(4-chlorpyrimidin-5-yl)ethinyl]phenyl}harnstoff;
N-(5-tert-Butylisoxazol-3-yl)-N'-(3-{[4-(methylamino)pyrimidin-5-yl]ethinyl}phenyl)harnstoff;
N-(5-tert-Butylisoxazol-3-yl)-N'-{3-[(4-{[3-(isopropylamino)propyl]amino}pyrimidin-5-yl]-ethinyl]phenyl}harnstoff;
N-(5-tert-Butylisoxazol-3-yl)-N'-[3-({4-[(2-pyrrolidin-1-ylethyl)amino]pyrimidin-5-yl}-ethinyl)phenyl]harnstoff;
N-(5-tert-Butylisoxazol-3-yl)-N'-[3-({4-[(5-tert-butylisoxazol-3-yl)amino]pyrimidin-5-yl}ethinyl)-phenyl]harnstoff;
N-(5-tert-Butylisoxazol-3-yl)-N'-{3-[(4-{[3-(dimethylamino)propyl]amino}pyrimidin-5-yl]-ethinyl]phenyl}harnstoff;
N-(5-tert-Butylisoxazol-3-yl)-N'-[3-({4-[(2-hydroxyethyl)amino]pyrimidin-5-yl}ethinyl)phenyl]-harnstoff;
N-(5-tert-Butylisoxazol-3-yl)-N'-[3-({4-[(2-morpholin-4-ylethyl)amino]pyrimidin-5-yl}ethinyl)-phenyl]harnstoff;
N-[3-({4-[(4-Aminobutyl)amino]pyrimidin-5-yl}-ethinyl)phenyl]-N'-(5-tert-butylisoxazol-3-yl)-harnstoff;
N-(5-tert-Butylisoxazol-3-yl)-N'-[3-({4-[(3-pyrrolidin-1-ylpropyl)amino]pyrimidin-5-yl}-ethinyl)phenyl]harnstoff;
N-(5-tert-Butylisoxazol-3-yl)-N'-[3-({4-[(2,4-dimethoxybenzyl)amino]pyrimidin-5-yl}ethinyl)-phenyl]harnstoff;
N-[3-({4-[(2-Aminoethyl)amino]pyrimidin-5-yl}-ethinyl)phenyl]-N'-(5-tert-butylisoxazol-3-yl)-harnstoff;
N-(5-tert-Butylisoxazol-3-yl)-N'-{3-[(4-{[2-(dimethylamino)ethyl]amino}pyrimidin-5-yl)-ethinyl]phenyl}harnstoff;
N-(5-tert-Butylisoxazol-3-yl)-N'-{3-[(4-{[4-(dimethylamino)butyl]amino}pyrimidin-5-yl)-ethinyl]phenyl}harnstoff;
N-(5-tert-Butylisoxazol-3-yl)-N'-[3-({4-[N-{2-(dimethylamino)ethyl}-N-methylamino]pyrimidin-5-yl}ethinyl)phenyl]harnstoff;
N-(5-tert-Butylisoxazol-3-yl)-N'-[3-({4-[(2-piperidin-1-ylethyl)amino]pyrimidin-5-yl}ethinyl)-phenyl]harnstoff;
N-(5-tert-Butylisoxazol-3-yl)-N'-[3-({4-[(3-morpholin-4-ylpropyl)amino]pyrimidin-5-yl}-ethinyl)phenyl]harnstoff;
N-(5-tert-Butylisoxazol-3-yl)-N'-[3-({4-[(3-piperidin-1-ylpropyl)amino]pyrimidin-5-yl}-ethinyl)phenyl]harnstoff;
N-(5-tert-Butylisoxazol-3-yl)-N'-{3-[(4-{[3-(4-methylpiperazin-1-yl)propyl]amino}pyrimidin-5-yl)-ethinyl]phenyl}harnstoff;
N-(3-tert-Butyl-1-methyl-1H-pyrazol-5-yl)-N'-[3-({4-[(2-morpholin-4-ylethyl)amino]pyrimidin-5-yl}-ethinyl)phenyl]harnstoff oder
N-{3-[(4,6-Diaminopyrimidin-5-yl)ethinyl]phenyl}-N'-(2,3-dihydro-1H-inden-1-yl)harnstoff;
oder ein Salz davon.

11. Pharmazeutische Zusammensetzung, die eine Verbindung der Formel I oder ein pharmazeutisch annehmbares Salz davon gemäß einem der Ansprüche 1 bis 10 zusammen mit einem pharmazeutisch annehmbaren Verdünnungsmittel oder Träger enthält.

12. Verbindung der Formel I oder ein pharmazeutisch annehmbares Salz davon nach einem der Ansprüche 1 bis 10 zur Verwendung als Arzneimittel.

13. Verwendung einer Verbindung der Formel I oder eines pharmazeutisch annehmbaren Salzes davon gemäß einem der Ansprüche 1 bis 10 bei der Herstellung eines Arzneimittels zur Verwendung bei der Behandlung einer Erkrankung, bei der die Inhibierung von Tie2-Rezeptor-Tyrosinkinase bei einem Warmblüter wie dem Menschen gefordert ist.

14. Verwendung einer Verbindung der Formel I oder eines pharmazeutisch annehmbaren Salzes davon gemäß einem der Ansprüche 1 bis 10 bei der Herstellung eines Arzneimittels zur Verwendung bei der Behandlung oder Prophylaxe einer Erkrankung, die mit unerwünschter oder pathologischer Angiogenese bei einem Warmblüter wie dem Menschen assoziiert ist.

15. Verfahren zur Herstellung einer Verbindung der Formel I gemäß Anspruch 1 oder eines pharmazeutisch annehmbaren Salzes davon (worin R¹, R², R³, R⁴, R⁵, R⁶, R⁷, R⁸, R⁹, R¹⁰, R¹¹, L, Ring A und Ring B, n und m die in Anspruch 1 angegebene Bedeutung besitzen, sofern nicht anders vermerkt), bei dem man:
(a) für Verbindungen der Formel I, worin L für -N(R⁸)C(O)N(H)- steht, eine Verbindung der Formel II: 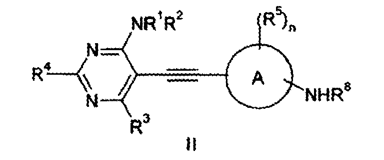 worin R¹, R², R³, R⁴, R⁵, R⁸, n und A eine der oben angegebenen Bedeutungen besitzen, wobei jedoch jede funktionelle Gruppe gegebenenfalls geschützt ist, mit einem Isocyanat der Formel IV: 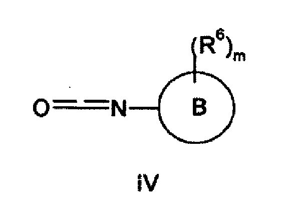 worin R⁶, m und B eine der oben angegebenen Bedeutungen besitzen, wobei jedoch jede funktionelle Gruppe gegebenenfalls geschützt ist, umsetzt; oder
(b) für Verbindungen der Formel I, worin L für -N(R⁸)C(O)N(H)- steht, eine Verbindung der Formel II gemäß obiger Definition mit einem Arylcarbamat der Formel III: 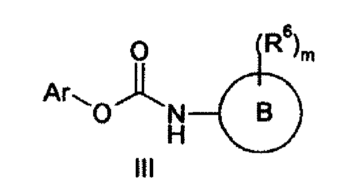 worin Ar für eine geeignete Arylgruppe, beispielsweise Phenyl, steht und R⁶, m und B eine der oben angegebenen Bedeutungen besitzen, wobei jedoch jede funktionelle Gruppe gegebenenfalls geschützt ist, umsetzt; oder
(c) für Verbindungen der Formel I, worin L für N(R⁸)C(O)-O- steht, eine Verbindung der Formel II gemäß obiger Definition mit einer Verbindung der Formel XI: 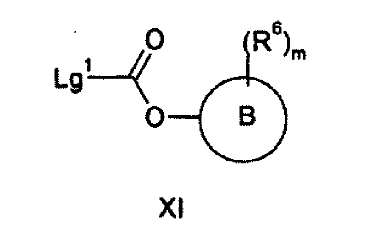 worin Lg¹ für eine geeignete Abgangsgruppe, beispielsweise Halogen (wie Fluor, Chlor oder Brom), steht und R⁶, m und B eine der oben angegebenen Bedeutungen besitzen, wobei jedoch jede funktionelle Gruppe gegebenenfalls geschützt ist, umsetzt; oder
(d) für Verbindungen der Formel I, worin L für N(R⁸)C(O)C(R^{a}R^{b}) steht, eine Verbindung der Formel II gemäß obiger Definition mit einer Verbindung der Formel IX: 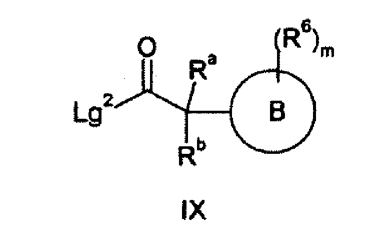 worin Lg² für eine geeignete Abgangsgruppe, beispielsweise Hydroxy, Halogen (wie Fluor, Chlor oder Brom), R^{x}-C(O)-O- oder R^{x}-O (worin R^{x} eine geeignete Alkyl- oder Arylgruppe bedeutet), steht und R⁶, R^{a}, R^{b}, m und B eine der oben angegebenen Bedeutungen besitzen, wobei jedoch jede funktionelle Gruppe gegebenenfalls geschützt ist, umsetzt; oder
(e) für Verbindungen der Formel I, worin L für -N(R⁸)C(O)N(H)- steht, eine Verbindung der Formel II gemäß obiger Definition mit einem Trichloracetylamin der Formel XIII: 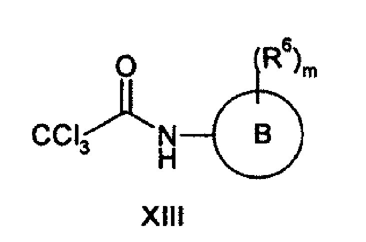 worin R⁶, m und B eine der oben angegebenen Bedeutungen besitzen, wobei jedoch jede funktionelle Gruppe gegebenenfalls geschützt ist, umsetzt; oder
(f) für Verbindungen der Formel I, worin L für - C(R^{a}R^{b})C(O)N(R⁹) - steht, eine Verbindung der Formel XIV: 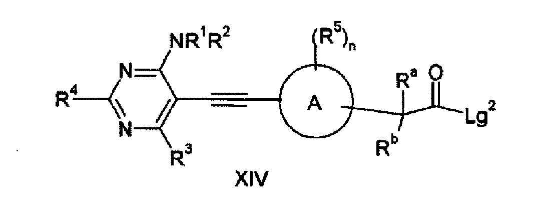 worin Lg² für eine geeignete Abgangsgruppe gemäß obiger Beschreibung steht und R¹, R², R³, R⁴, R⁵, R^{a}, R^{b}, n und A eine der oben angegebenen Bedeutungen besitzen, wobei jedoch jede funktionelle Gruppe gegebenenfalls geschützt ist, mit einem Amin der Formel XV: 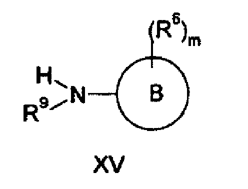 worin R⁶, R⁹, m und B eine der oben angegebenen Bedeutungen besitzen, wobei jedoch jede funktionelle Gruppe gegebenenfalls geschützt ist, umsetzt; oder
(g) eine Verbindung der Formel XVI: 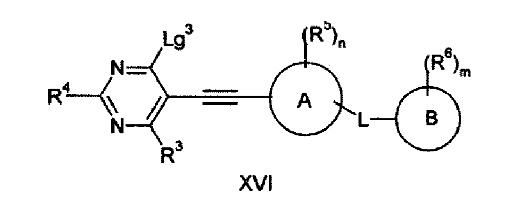 worin Lg³ für eine geeignete Abgangsgruppe, beispielsweise Halogen (wie Fluor, Chlor, Brom oder Iod), Methylsulfonyl, Methylthio oder Aryloxy (wie Phenoxy), steht und R³, R⁴, R⁵, R⁶, n, m, A, B und L eine der oben angegebenen Bedeutungen besitzen, wobei jedoch jede funktionelle Gruppe gegebenenfalls geschützt ist, mit einem Amin der Formel HNR¹R², worin R¹ und R² eine der oben angegebenen Bedeutungen besitzen, wobei jedoch jede funktionelle Gruppe gegebenenfalls geschützt ist, umsetzt; oder
(h) eine Verbindung der Formel XVII: 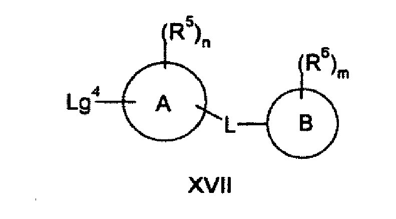 worin Lg⁴ für eine geeignete Abgangsgruppe, beispielsweise Halogen (wie Chlor, Brom oder Iod) oder eine Sulfonyloxygruppe (wie Trifluormethylsulfonyloxy) steht und R⁵, R⁶, n, m, A, B und L eine der oben angegebenen Bedeutungen besitzen, wobei jedoch jede funktionelle Gruppe gegebenenfalls geschützt ist, mit einem Alkin der Formel XVIII: 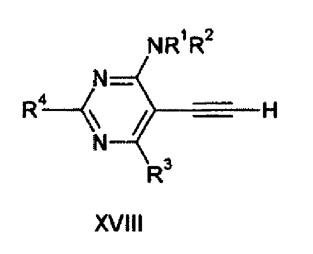 worin R¹, R², R³ und R⁴ eine der oben angegebenen Bedeutungen besitzen, wobei jedoch jede funktionelle Gruppe gegebenenfalls geschützt ist, umsetzt; oder
(i) für Verbindungen der Formel I, worin L für -N(H)C(O)N(R⁹)- steht, ein Isocyanat der Formel XIX: 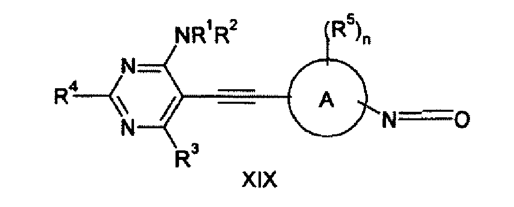 worin R¹, R², R³, R⁴, R⁵, n und A eine der oben angegebenen Bedeutungen besitzen, wobei jedoch jede funktionelle Gruppe gegebenenfalls geschützt ist, mit einem Amin der Formel XV gemäß obiger Definition umsetzt; oder
(j) für Verbindungen der Formel I, worin L für -N(H)C(O)N(R⁹)- steht, eine Verbindung der Formel XX: 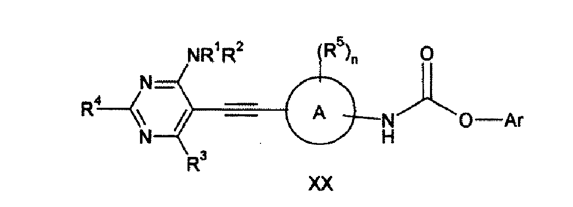 worin Ar für eine geeignete Arylgruppe, beispielsweise Phenyl, steht und R¹, R², R³, R⁴, R⁵, n und A eine der oben angegebenen Bedeutungen besitzen, wobei jedoch jede funktionelle Gruppe gegebenenfalls geschützt ist, mit einem Amin der Formel XV gemäß obiger Definition umsetzt;
und danach gegebenenfalls:
i) eine Verbindung der Formel (I) in eine andere Verbindung der Formel (I) umwandelt;
ii) jegliche Schutzgruppen abspaltet;
iii) ein Salz bildet.

16. Verbindung, ausgewählt unter den Formeln II, XIV, XVI, XIX und XX gemäß Anspruch 15 oder einer Verbindung der Formel VIc: 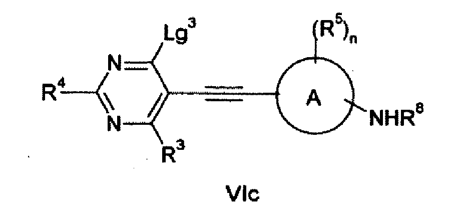 oder ein Salz davon, wobei Ring A, R¹, R², R³, R⁴, R⁵, R⁶, R⁸, L, m, Ring B und n die in Anspruch 1 angegebene Bedeutung besitzen, Ar für eine Arylgruppe steht, Lg² für Hydroxyl, Halogen, R^{x}-C(O)-O- oder R^{x}-O (worin R^{x} eine geeignete Alkyl- oder Arylgruppe bedeutet), steht und Lg³ für Halogen, Methylsulfonyl, Methylthio oder Aryloxy steht.

## Revendications

1. Composé de formule I 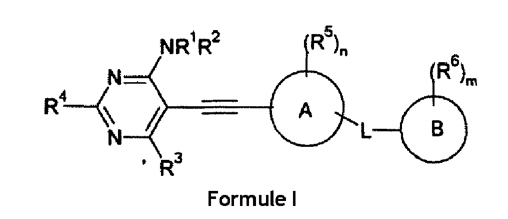 dans laquelle :
**R¹ et R²** sont choisis indépendamment parmi un atome d'hydrogène, un groupe alkylsulfonyle en (1-6C), un groupe phényl(CH₂)ᵤ- dans lequel u vaut 0, 1, 2, 3, 4, 5 ou 6, un groupe alcanoyle en (1-6C), un groupe alkyle en (1-6C), un groupe (1-6C) alcoxy-carbonyle, un groupe (3-6C)cycloalkyl(CH₂)ₓ- dans lequel x vaut 0, 1, 2, 3, 4, 5 ou 6, ou un noyau hétéroaryle à 5 ou 6 chaînons, ou **R¹ et R²,** conjointement avec l'atome d'azote auquel ils sont fixés, représentent un noyau hétérocyclique, saturé ou partiellement saturé, à 3 à 7 chaînons, renfermant éventuellement un autre hétéroatome choisi parmi un atome d'azote ou un atome d'oxygène ;
où les groupes alkyle en (1-6C), alcanoyle en (1-6C) et cycloalkyle en (3-6C) sont éventuellement substitués par un ou plusieurs groupes choisis indépendamment parmi un groupe fluoro, un groupe hydroxy, un groupe alkyle en (1-6C), un groupe alcoxy en (1-6C), un groupe (1-6C) alcoxy (1-6C) alcoxy, un groupe (1-6C)-alcoxy(1-6C)alcoxy(1-6C)alcoxy, un groupe amino, un groupe mono(1-6C)alkylamino, un groupe di-[(1-6C)alkyl]amino, un groupe carbamoyle, un groupe mono(1-6C)alkyl-carbamoyle, un groupe di-[(1-6C)alkyl]-carbamoyle ou un groupe -N (R^{d}) C (O) (1-6C) alkyle dans lequel R^{d} est un atome d'hydrogène ou un groupe alkyle en (1-6C), ou un noyau hétérocyclique, saturé ou partiellement saturé, à 3 à 7 chaînons, ou un noyau hétéroaryle à 5 ou 6 chaînons,
où les groupes alcoxy en (1-6C), (1-6C)-alcoxy(1-6C)alcoxy et (1-6C)alcoxy(1-6C)-alcoxy(1-6C)alcoxy et les groupes alkyle en (1-6C) des groupes mono(1-6C)alkylamino, di-[(1-6C)alkyl]amino, mono(1-6C)alkylcarbamoyle, di- [(1-6C)alkyl]carbamoyle et/ou -N(R^{d})C(O)-(1-6C)alkyle sont éventuellement substitués par un ou plusieurs groupes hydroxy ;
où le groupe phényle est éventuellement substitué par un ou plusieurs groupes choisis indépendamment parmi un groupe halogéno, un groupe alkyle en (1-6C), un groupe alcoxy en (1-6C), un groupe amino, un groupe mono(1-6C)-alkylamino ou un groupe di-[(1-6C)alkyl]amino, où les groupes alkyle en (1-6C) et alcoxy en (1-6C) sont éventuellement substitués par un ou plusieurs groupes choisis indépendamment parmi un groupe hydroxy, un groupe amino, un groupe mono(1-6C)alkylamino ou un groupe di-[(1-6C)alkyl]amino ;
et où tout noyau hétérocyclique et hétéroaryle dans R¹ et/ou R² est éventuellement substitué indépendamment par l'un ou plusieurs parmi les suivants : un groupe alkyle en (1-4C), un groupe alcoxy en (1-4C), un groupe (1-4C)-alcoxy(1-4C)alkyle, un groupe hydroxy, un groupe amino, un groupe mono(1-6C)alkylamino ou un groupe di-[(1-6C)alkyl]amino, ou un noyau hétérocyclique, saturé ou partiellement saturé, à 3 à 7 chaînons, ou un groupe -C(O)(CH₂)_{z}Y dans lequel z vaut 0, 1, 2 ou 3 et Y est choisi parmi un atome d'hydrogène, un groupe hydroxy, un groupe alcoxy en (1-4C), un groupe amino, un groupe mono(1-6C)alkylamino, un groupe di-[(1-6C)alkyl]amino ou un noyau hétérocyclique, saturé ou partiellement saturé, à 3 à 7 chaînons ;
et à condition que, lorsque R¹ et/ou R² sont un groupe alcanoyle en (1C), alors le groupe alcanoyle en (1C) ne soit pas substitué par un groupe fluoro ou un groupe hydroxy ;
**R³** est choisi parmi un atome d'hydrogène, un groupe alkyle en (1-6C) ou un groupe alcoxy en (1-6C), un groupe alcoxy en (1-6C) où les groupes alkyle et alcoxy sont éventuellement substitués par un ou plusieurs groupes choisis parmi : un groupe fluoro, un groupe hydroxy, un groupe alkyle en (1-6C), un groupe alcoxy en (1-6C), un groupe carbamoyle, un groupe mono(1-6C)alkylcarbamoyle ou un groupe di([(1-6C)alkyl]carbamoyle, un groupe amino, un groupe mono(1-6C)alkylamino ou un groupe di(1-6C)alkylamino, un noyau hétérocyclique, saturé ou partiellement saturé, à 3 à 7 chaînons ou un noyau hétéroaryle à 5 ou 6 chaînons, où lesdits noyaux hétérocyclique et hétéroaryle sont éventuellement substitués indépendamment par l'un ou plusieurs parmi les suivants : un groupe alkyle en (1-4C), un groupe alcoxy en (1-4C), un groupe hydroxy, un groupe amino, un groupe mono-(1-6C)alkylamino ou un groupe di(1-6C)alkylamino, ou un noyau hétérocyclique, saturé ou partiellement saturé, à 3 à 7 chaînons ;
ou **R³** représente un groupe **-NR¹R²** tel que défini ci-dessus ;
**R⁴** est choisi parmi un atome d'hydrogène, un groupe alkyle en (1-6C) ou un groupe alcoxy en (1-6C) ;
A représente un groupe aryle ou un noyau hétéroaryle à 5 ou 6 chaînons choisi parmi un groupe furyle, un groupe pyrrolyle, un groupe thiényle, un groupe oxazolyle, un groupe isoxazolyle, un groupe imidazolyle, un groupe pyrazolyle, un groupe thiazolyle, un groupe isothiazolyle, un groupe oxadiazolyle, un groupe thiadiazolyle, un groupe triazolyle, un groupe tétrazolyle, un groupe pyridyle, un groupe pyridazinyle, un groupe pyrimidinyle, un groupe pyrazinyle ou un groupe 1,3,5-triazinyle ;
**R⁵** est choisi parmi un groupe cyclopropyle, un groupe cyano, un groupe halogéno, un groupe alcoxy en (1-6C) ou un groupe alkyle en (1-6C), où les groupes alkyle en (1-6C) et alcoxy en (1-6C) sont éventuellement substitués par un groupe cyano ou par un ou plusieurs groupes fluoro ;
**n** vaut 0, 1, 2 ou 3 ;
**L** est fixé en méta ou para sur le noyau A par rapport au point de fixation du groupe éthynyle et représente un groupe -C(R^{a}R^{b}) C(O)N(R⁹)-, un groupe -N(R⁸)C(O)C(R^{a}R^{b})-, un groupe -N(R⁸)C(O)N(R⁹)-, un groupe -N(R⁸)C(O)O- ou un groupe -OC(O)-N(R⁹)-, dans lesquels R⁸ et R⁹ représentent indépendamment un atome d'hydrogène ou un groupe alkyle en (1-6C) et dans lesquels R^{a} et R^{b} représentent indépendamment un atome d'hydrogène ou un groupe alkyle en (1-6C), ou R^{a} et R^{b}, conjointement avec l'atome de carbone auquel ils sont fixés, représentent un groupe cycloalkyle en (3-6C) ;
**B** représente un noyau cycloalkyle en (3-7C), un noyau hétérocyclique, saturé ou partiellement saturé, à 3 à 7 chaînons, un groupe aryle, un noyau hétéroaryle à 5 ou 6 chaînons choisi parmi un groupe furyle, un groupe pyrrolyle, un groupe thiényle, un groupe oxazolyle, un groupe isoxazolyle, un groupe imidazolyle, un groupe pyrazolyle, un groupe thiazolyle, un groupe isothiazolyle, un groupe oxadiazolyle, un groupe thiadiazolyle, un groupe triazolyle, un groupe tétrazolyle, un groupe pyridyle, un groupe pyridazinyle, un groupe pyrimidinyle, un groupe pyrazinyle ou un groupe 1,3,5-triazinyle, ou un groupe bicyclique à 8, 9 ou 10 chaînons qui renferme éventuellement 1, 2, 3 ou 4 hétéroatomes choisis indépendamment parmi un atome d'azote, un atome d'oxygène et un atome de soufre, et qui est saturé, partiellement saturé ou aromatique ;
**R⁶** est choisi parmi un groupe halogéno, un groupe cyano, un groupe oxo, un noyau cycloalkyle en (3-7C), un noyau hétérocyclique, saturé ou partiellement saturé, à 3 à 7 chaînons, un groupe -S(O)ₚ(1-6C)alkyle dans lequel p vaut 0, 1 ou 2, un groupe -N(R^{a})C(O) (1-6C) alkyle dans lequel R^{a} est un atome d'hydrogène ou un groupe alkyle en (1-6C) ;
ou
**R⁶** est choisi parmi un groupe alkyle en (1-6C) ou un groupe alcoxy en (1-6C), où les groupes alkyle en (1-6C), -S(O)ₚ(1-6C)alkyle et alcoxy en (1-6C) sont éventuellement substitués par un ou plusieurs groupes choisis indépendamment parmi un groupe cyano, un groupe fluoro, un groupe hydroxy, un groupe alcoxy en (1-6C), un groupe amino, un groupe mono(1-6C)alkylamino, un groupe di-[(1-6C)-alkyl]amino, un noyau cycloalkyle en (3-7C) ou un noyau hétérocyclique, saturé ou partiellement saturé, à 3 à 7 chaînons ; où le noyau cycloalkyle en (3-7C) et le noyau hétérocyclique, saturé ou partiellement saturé, à 3 à 7 chaînons sont éventuellement substitués indépendamment par un ou plusieurs groupes choisis parmi un groupe alkyle en (1-6C) ; et
**m** vaut 0, 1, 2 ou 3 ;
et, lorsque B est un noyau cycloalkyle en (3-7C) ou un noyau hétérocyclique, saturé ou partiellement saturé, à 3 à 7 chaînons, ou un groupe bicyclique, saturé ou partiellement saturé, à 8, 9 ou 10 chaînons, les noyaux et le groupe bicyclique portent éventuellement 1 ou 2 substituants oxo ou thioxo ;
et des sels de celui-ci.

2. Composé de formule I selon la revendication 1, dans laquelle :
**R⁶** est choisi parmi un groupe halogéno, un groupe cyano, un noyau cycloalkyle en (3-7C), un noyau hétérocyclique, saturé ou partiellement saturé, à 3 à 7 chaînons ou un groupe alcanoylamino -N(R^{c})C(O) (1-6C) alkyle dans lequel R^{c} est un atome d'hydrogène ou un groupe alkyle en (1-6C) ; ou
**R⁶** est choisi parmi un groupe alkyle en (1-6C) ou un groupe alcoxy en (1-6C), où les groupes alkyle en (1-6C) et alcoxy en (1-6C) sont éventuellement substitués par un ou plusieurs groupes choisis indépendamment parmi un groupe cyano, un groupe fluoro, un groupe hydroxy, un groupe alcoxy en (1-6C), un groupe amino, un groupe mono-(1-6C)alkylamino, un groupe di-[(1-6C)alkyl]amino, un noyau cycloalkyle en (3-7C) ou un noyau hétérocyclique, saturé ou partiellement saturé, à 3 à 7 chaînons ;
et des sels de celui-ci.

3. Composé de formule I selon la revendication 1, dans laquelle :
**R¹ et R²** sont choisis indépendamment parmi un atome d'hydrogène, un groupe alkylsulfonyle en (1-6C), un groupe phényl(CH₂)ᵤ- dans lequel u vaut 0, 1, 2, 3, 4, 5 ou 6, un groupe alcanoyle en (1-6C), un groupe alkyle en (1-6C), un groupe (1-6C) alcoxy-carbonyle, ou un groupe (3-6C)cycloalkyl(CH₂)ₓ- dans lequel x vaut 0, 1, 2, 3, 4, 5 ou 6, ou
**R¹ et R²,** conjointement avec l'atome d'azote auquel ils sont fixés, représentent un noyau hétérocyclique, saturé ou partiellement saturé, à 3 à 7 chaînons, renfermant éventuellement un autre hétéroatome choisi parmi un atome d'azote ou un atome d'oxygène ;
où les groupes alkyle et cycloalkyle sont éventuellement substitués par un ou plusieurs groupes choisis parmi un groupe fluoro, un groupe hydroxy, un groupe alkyle en (1-6C), un groupe alcoxy en (1-6C), un groupe amino, un groupe mono(1-6C)alkylamino ou di-[(1-6C)-alkyl]amino, un noyau hétérocyclique, saturé ou partiellement saturé, à 3 à 7 chaînons ou un noyau hétéroaryle à 5 ou 6 chaînons, où lesdits noyaux hétérocyclique et hétéroaryle sont éventuellement substitués indépendamment par l'un ou plusieurs parmi les suivants : un groupe alkyle en (1-4C), un groupe hydroxy, un groupe amino, un groupe mono(1-6C)alkylamino ou un groupe di-[(1-6C)alkyl]amino, ou un noyau hétérocyclique, saturé ou partiellement saturé, à 3 à 7 chaînons ;
et où le groupe phényle est éventuellement substitué par un ou plusieurs groupes choisis parmi un groupe halogéno, un groupe alkyle en (1-6C), un groupe alcoxy en (1-6C), un groupe amino, un groupe mono(1-6C)alkylamino ou un groupe di-[(1-6C)alkyl]amino, où les groupes alkyle en (1-6C) ou alcoxy en (1-6C) sont éventuellement substitués par un groupe hydroxy, un groupe amino, un groupe mono-(1-6C)alkylamino ou un groupe di-[(1-6C)-alkyl]amino ;
**R³** est choisi parmi un atome d'hydrogène, un groupe alkyle en (1-6C) ou un groupe alcoxy en (1-6C), où les groupes alkyle et alcoxy sont éventuellement substitués par un ou plusieurs groupes choisis parmi un groupe fluoro, un groupe hydroxy, un groupe alkyle en (1-6C), un groupe alcoxy en (1-6C), un groupe amino, un groupe mono-(1-6C)alkylamino ou un groupe di([(1-6C)alkyl]-amino, un noyau hétérocyclique, saturé ou partiellement saturé, à 3 à 7 chaînons ou un noyau hétéroaryle à 5 ou 6 chaînons, où lesdits noyaux hétérocyclique et hétéroaryle sont éventuellement substitués indépendamment par l'un ou plusieurs parmi les suivants : un groupe alkyle en (1-4C), un groupe hydroxy, un groupe amino, un groupe mono(1-6C)alkylamino ou un groupe di-[(1-6C)-alkyl]amino ou un noyau hétérocyclique, saturé ou partiellement saturé, à 3 à 7 chaînons ;
ou **R³** représente un groupe **-NR¹R²** tel que défini ci-dessus ;
**R⁴** est choisi parmi un atome d'hydrogène, un groupe alkyle en (1-6C) ou un groupe alcoxy en (1-6C) ;
**A** représente un groupe aryle ou un noyau hétéroaryle à 5 ou 6 chaînons choisi parmi un groupe furyle, un groupe pyrrolyle, un groupe thiényle, un groupe oxazolyle, un groupe isoxazolyle, un groupe imidazolyle, un groupe pyrazolyle, un groupe thiazolyle, un groupe isothiazolyle, un groupe oxadiazolyle, un groupe thiadiazolyle, un groupe triazolyle, un groupe tétrazolyle, un groupe pyridyle, un groupe pyridazinyle, un groupe pyrimidinyle, un groupe pyrazinyle ou un groupe 1,3,5-triazinyle ;
**R⁵** est choisi parmi un groupe cyano, un groupe halogéno, un groupe alcoxy en (1-6C) ou un groupe alkyle en (1-6C) éventuellement substitué par un groupe cyano ou par un ou plusieurs groupes fluoro ;
**n** vaut 0, 1, 2 ou 3 ;
**L** est fixé en méta ou para sur le noyau A par rapport au point de fixation du groupe éthynyle et représente un groupe -C(R^{a}R^{b}) C(O)N(R⁹)-, un groupe -N(R⁸)C(O)C(R^{a}R^{b})-, un groupe -N(R⁸)C(O)N(R⁹)-, un groupe -N(R⁸)C(O)O- ou un groupe -OC(O)N(R⁹)-, dans lesquels R⁸ et R⁹ représentent indépendamment un atome d'hydrogène ou un groupe alkyle en (1-6C) et dans lesquels R^{a} et R^{b} représentent indépendamment un atome d'hydrogène ou un groupe alkyle en (1-6C), ou R^{a} et R^{b}, conjointement avec l'atome de carbone auquel ils sont fixés, représentent un groupe cycloalkyle en (3-6C) ;
**B** représente un noyau cycloalkyle en (3-7C), un groupe aryle ou un noyau hétéroaryle à 5 ou 6 chaînons choisi parmi un groupe furyle, un groupe pyrrolyle, un groupe thiényle, un groupe oxazolyle, un groupe isoxazolyle, un groupe imidazolyle, un groupe pyrazolyle, un groupe thiazolyle, un groupe isothiazolyle, un groupe oxadiazolyle, un groupe thiadiazolyle, un groupe triazolyle, un groupe tétrazolyle, un groupe pyridyle, un groupe pyridazinyle, un groupe pyrimidinyle, un groupe pyrazinyle ou un groupe 1,3,5-triazinyle ;
**R⁶** est choisi parmi un groupe halogéno, un groupe cyano, un noyau hétérocyclique, saturé ou partiellement saturé, à 3 à 7 chaînons ou un groupe alcanoylamino -N (R^{a}) C (O) (1-6C) alkyle dans lequel R^{a} est un atome d'hydrogène ou un groupe alkyle en (1-6C) ; ou **R⁶** est choisi parmi un groupe alkyle en (1-6C) ou un groupe alcoxy en (1-6C), où les groupes alkyle et alcoxy sont éventuellement substitués par un ou plusieurs groupes choisis parmi un groupe cyano, un groupe fluoro, un groupe hydroxy, un groupe alcoxy en (1-6C), un groupe amino, un groupe mono(1-6C)alkylamino, un groupe di-[(1-6C)alkyl]amino, ou un noyau hétérocyclique, saturé ou partiellement saturé, à 3 à 7 chaînons ; et
**m** vaut 0, 1, 2 ou 3 ;
et, lorsque m vaut au moins 2, alors deux substituants sur des atomes de carbone adjacents dans le noyau B peuvent représenter conjointement un groupe méthylènedioxy ;
et des sels de celui-ci.

4. Composé selon l'une quelconque des revendications 1, 2 et 3, dans lequel A est choisi parmi un groupe phényle, un groupe pyridyle, un groupe thiazolyle, un groupe thiadiazolyle ou un groupe pyrimidinyle.

5. Composé selon l'une quelconque des revendications précédentes, dans lequel B est choisi parmi un groupe phényle, un groupe 2,3-dihydro-indényle, un groupe pipéridinyle, un groupe pyridyle, un groupe pyrazolyle, un groupe isothiazolyle, un groupe thiadiazolyle, un groupe isoxazolyle, un groupe benzodioxinyle, un groupe benzodioxolyle ou un groupe tétrahydropyranyle.

6. Composé selon l'une quelconque des revendications précédentes, dans lequel L est choisi parmi un groupe -N(R⁸)C(O)N(R⁹)-, un groupe -N(R⁸)C(O)O- ou un groupe -N(R⁸)C(O)CH₂-, dans lesquels R⁸ et R⁹ représentent indépendamment un atome d'hydrogène ou un groupe alkyle en (1-6C).

7. Composé selon l'une quelconque des revendications précédentes, dans lequel R¹ et R² sont tous les deux un atome d'hydrogène ou R¹ est un atome d'hydrogène ou un groupe alkyle en (1-6C) et R² est un groupe alkyle en (1-6C),
où le groupe alkyle en (1-6C) est éventuellement substitué par un groupe hydroxy, un groupe amino, un groupe mono-(1-6C)alkylamino ou un groupe di-[(1-6C)-alkyl]amino, un groupe carbamoyle, un groupe alcoxy en (1-6C), un groupe (1-6C) alcoxy-(1-6C)alcoxy, un groupe -N(R^{d})C(O) (1-6C) alkyle dans lequel R^{d} est un atome d'hydrogène ou un groupe alkyle en (1-6C), un groupe aryle (en particulier, un groupe phényle), un noyau hétérocyclique, saturé ou partiellement saturé, à 3 à 7 chaînons, ou un noyau hétéroaryle à 5 ou 6 chaînons ;
où les groupes alcoxy en (1-6C), mono-(1-6C)alkylamino et -N(R^{d})C(O) (1-6C)alkyle sont éventuellement substitués par un groupe hydroxy ; et
où un noyau aryle, un noyau hétérocyclique, saturé ou partiellement saturé, à 3 à 7 chaînons ou un noyau hétéroaryle à 5 ou 6 chaînons est éventuellement substitué par un groupe alkyle en (1-4C), un groupe alcoxy en (1-4C) ou un groupe -C(O)(CH₂)Y dans lequel Y est choisi parmi un groupe hydroxy ou un groupe di(1-6C)alkylamino.

8. Composé selon l'une quelconque des revendications précédentes, dans lequel R³ et R⁴ sont tous les deux un atome d'hydrogène.

9. Composé selon l'une quelconque des revendications précédentes, dans lequel R⁶ est choisi indépendamment parmi un groupe halogéno, un groupe cyano, un groupe oxo, un groupe cycloalkyle en (3-7C), un noyau hétéroaryle saturé à 3 à 7 chaînons (éventuellement substitué par un groupe alkyle en (1-4C)), un groupe -N(R^{c})C(O) (1-6C) alkyle dans lequel R^{c} est un atome d'hydrogène ou un groupe alkyle en (1-6C) (en particulier, un groupe alkyle en (1-4C)), un groupe alkyle en (1-6C) (éventuellement substitué par un groupe halogéno)
ou un groupe alcoxy en (1-6C), et m est choisi parmi 1 ou 2.

10. Composé selon la revendication 1, choisi parmi :
la N-{3-[(4,6-diaminopyrimidin-5-yl)éthynyl]-phényl}-*N'*-phénylurée ;
le N-{3-[(4,6-diaminopyrimidin-5-yl)éthynyl]-phényl}-2-phénylacétamide ;
la N-{3-[(4,6-diaminopyrimidin-5-yl)éthynyl]-phényl}-N'-(3,4-dichlorophényl)urée ;
la N-{3-[(4,6-diaminopyrimidin-5-yl)éthynyl]-phényl}-N'-[2-(trifluorométhyl)phényl]urée ;
la N-{3-[(4,6-diaminopyrimidin-5-yl)éthynyl]-phényl}-N'-[3-(trifluorométhyl)phényl]urée ;
la N-{3-[(4,6-diaminopyrimidin-5-yl)éthynyl]-phényl}-N'-[4-(trifluorométhyl)phényl]urée ;
la N-{4-[(4,6-diaminopyrimidin-5-yl)éthynyl]-phényl}-N'-[2-fluoro-5-(trifluorométhyl)phényl]-urée ;
la N-{3-[(4,6-diaminopyrimidin-5-yl)éthynyl]-phényl}-N'-(3-méthoxyphényl)urée ;
le {4-[(4,6-diaminopyrimidin-5-yl)éthynyl]phényl}-carbamate de phényle ;
le {3-[(4,6-diaminopyrimidin-5-yl)éthynyl]phényl}-carbamate de phényle ;
la N-(5-tert-butyl-l,3,4-thiadiazol-2-yl)-N'-{4-[(4,6-diaminopyrimidin-5-yl)éthynyl]phényl}urée ;
la N-{4-[(4,6-diaminopyrimidin-5-yl)éthynyl]-phényl}-N'-(3-méthylisothiazol-5-yl)urée ;
la N-{4-[(4,6-diaminopyrimidin-5-yl)éthynyl]-phényl}-N'-(3-méthylisoxazol-5-yl)urée ;
la N-{4-[(4,6-diaminopyrimidin-5-yl)éthynyl]-phényl}-N'-[4-(trifluorométhyl)pyridin-2-yl]urée ;
le N-(3-{[({4-[(4,6-diaminopyrimidin-5-yl)-éthynyl]phényl}amino)carbonyl]amino}phényl)-acétamide ;
la N-{3-[(4,6-diaminopyrimidin-5-yl)éthynyl]-phényl}-N'-(3-méthylisothiazol-5-yl)urée ;
le N-(3-{[({3-[(4,6-diaminopyrimidin-5-yl)-éthynyl]phényl}amino)carbonyl]amino}phényl)-acétamide ;
la N-{3-[(4,6-diaminopyrimidin-5-yl)éthynyl]-phényl}-N'-[4-(trifluorométhyl)pyridin-2-yl]urée ;
la N-{3-[(4,6-diaminopyrimidin-5-yl)éthynyl]-phényl}-N'-(3-méthylisoxazol-5-yl)urée ;
la N-(5-tert-butyl-1,3,4-thiadiazol-2-yl)-N'-{3-[(4,6-diaminopyrimidin-5-yl)éthynyl]phényl}urée ;
la N-(5-tert-butylisoxazol-3-yl)-N'-{3-[(4,6-diaminopyrimidin-5-yl)éthynyl]phényl}urée ;
la N-{3-[(4,6-diaminopyrimidin-5-yl)éthynyl]-phényl}-N'-(2,3-dihydro-1,4-benzodioxin-6-yl)-urée ;
la N-{3-[(4,6-diaminopyrimidin-5-yl)éthynyl]-phényl}-N'-(2-morpholin-4-ylphényl)urée ;
la N-{3-[(4,6-diaminopyrimidin-5-yl)éthynyl]-phényl}-N'-(1-méthylpipéridin-4-yl)urée ;
la N-{3-[(4,6-diaminopyrimidin-5-yl)éthynyl]-phényl}-N'-(1-propylpipéridin-4-yl)urée ;
le *N*-{3-[(4,6-diaminopyrimidin-5-yl)éthynyl]-phényl}-2-phénylacétamide ;
le N-{3-[(4,6-diaminopyrimidin-5-yl)éthynyl]-phényl}-2-(2-méthoxyphényl)acétamide ;
le N-{3-[(4,6-diaminopyrimidin-5-yl)éthynyl]-phényl}-2-[(3-trifluorométhyl)phényl]acétamide ;
le N-{3-[(4,6-diaminopyrimidin-5-yl)éthynyl]-phényl}-2-[(4-trifluorométhyl)phényl]acétamide ;
le N-{3-[(4,6-diaminopyrimidin-5-yl)éthynyl]-phényl}-2-(3-méthoxyphényl)acétamide ;
la *N*-(5-tert-butylisoxazol-3-yl)-*N'*-{3-[(4-chloro-pyrimidin-5-yl)éthynyl]phényl}urée ;
la *N*-(5-tert-butylisoxazol-3-yl)-*N'*-(3-{[4-(méthylamino)pyrimidin-5-yl]éthynyl}phényl)urée ;
la N-(5-tert-butylisoxazol-3-yl)-N'-{3-[(4-{[3-(isopropylamino)propyl]amino}pyrimidin-5-yl)-éthynyl]phényl}urée ;
la *N*-(5-tert-butylisoxazol-3-yl)-*N*'-[3-({4-[(2-pyrrolidin-1-yléthyl)amino]pyrimidin-5-yl}-éthynyl)phényl]urée ;
la N-(5-tert-butylisoxazol-3-yl)-*N*'-[3-({4-[(5-tert-butylisoxazol-3-yl)amino]pyrimidin-5-yl}-éthynyl)phényl]urée ;
la N-(5-tert-butylisoxazol-3-yl)-N'-{3-[(4-{[3-(diméthylamino)propyl]amino}pyrimidin-5-yl)-éthynyl]phényl}urée ;
la N-(5-tert-butylisoxazol-3-yl)-N'-[3-({4-[(2-hydroxyéthyl)amino]pyrimidin-5-yl}éthynyl)phényl]-urée ;
la N-(5-tert-butylisoxazol-3-yl)-N'-[3-({4-[(2-morpholin-4-yléthyl)amino]pyrimidin-5-yl}éthynyl)-phényl]urée ;
la N-[3-({4-[(4-aminobutyl)amino]pyrimidin-5-yl}-éthynyl)phényl]-N'-(5-tert-butylisoxazol-3-yl)-urée ;
la N-(5-tert-butylisoxazol-3-yl)-N'-[3-({4-[(3-pyrrolidin-1-ylpropyl)amino]pyrimidin-5-yl}-éthynyl)phényl]urée ;
la N-(5-tert-butylisoxazol-3-yl)-N'-[3-({4-[(2,4-diméthoxybenzyl)amino]pyrimidin-5-yl}éthynyl)-phényl]urée ;
la N-[3-({4-[(2-aminoéthyl)amino]pyrimidin-5-yl}-éthynyl)phényl]-N'-(5-tert-butylisoxazol-3-yl)-urée ;
la N-(5-tert-butylisoxazol-3-yl)-N'-{3-[(4-{[2-(diméthylamino)éthyl]amino}pyrimidin-5-yl)-éthynyl]phényl}urée ;
la N-(5-tert-butylisoxazol-3-yl)-N'-{3-[(4-{[4-(diméthylamino)butyl]amino}pyrimidin-5-yl)-éthynyl]phényl}urée ;
la N-(5-tert-butylisoxazol-3-yl)-N'-[3-({4-[N-{2-(diméthylamino)éthyl}-N-méthylamino]pyrimidin-5-yl}éthynyl)phényl]urée ;
la N-(5-tert-butylisoxazol-3-yl)-N'-[3-({4-[(2-pipéridin-1-yléthyl)amino]pyrimidin-5-yl}éthynyl)-phényl]urée ;
la N-(5-tert-butylisoxazol-3-yl)-N'-[3-({4-[(3-morpholin-4-ylpropyl)amino]pyrimidin-5-yl}-éthynyl)phényl]urée ;
la N-(5-tert-butylisoxazol-3-yl)-N'-[3-({4-[(3-pipéridin-1-ylpropyl)amino]pyrimidin-5-yl}-éthynyl)phényl]urée ;
la N-(5-tert-butylisoxazol-3-yl)-N'-{3-[(4-{[3-(4-méthylpipérazin-1-yl)propyl]amino}pyrimidin-5-yl)-éthynyl]phényl}urée ;
la N-(3-tert-butyl-1-méthyl-1H-pyrazol-5-yl)-N'-[3-({4-[(2-morpholin-4-yléthyl)amino]pyrimidin-5-yl}éthynyl)phényl]urée ; ou
la N-{3-[(4,6-diaminopyrimidin-5-yl)éthynyl]-phényl}-N'-(2,3-dihydro-1H-indén-1-yl)urée ;
ou un sel de ceux-ci.

11. Composition pharmaceutique comprenant un composé de formule I, ou un sel pharmaceutiquement acceptable de celui-ci, tel que défini dans l'une quelconque des revendications 1 à 10, en association avec un diluant ou un support pharmaceutiquement acceptable.

12. Composé de formule I, ou un sel pharmaceutiquement acceptable de celui-ci, tel que défini dans l'une quelconque des revendications 1 à 10, destiné à être utilisé en tant que médicament.

13. Utilisation d'un composé de formule I, ou d'un sel pharmaceutiquement acceptable de celui-ci, tel que défini dans l'une quelconque des revendications 1 à 10, pour la fabrication d'un médicament destiné à être utilisé pour le traitement d'une maladie pour laquelle l'inhibition du récepteur tyrosine kinase Tie2 est requise chez un animal à sang chaud tel que l'homme.

14. Utilisation d'un composé de formule I, ou d'un sel pharmaceutiquement acceptable de celui-ci, tel que défini dans l'une quelconque des revendications 1 à 10, pour la fabrication d'un médicament destiné à être utilisé pour le traitement ou la prophylaxie d'une maladie associée à une angiogénèse indésirable ou pathologique chez un animal à sang chaud tel que l'homme.

15. Procédé de préparation d'un composé de formule I, tel que défini dans la revendication 1, ou d'un sel pharmaceutiquement acceptable de celui-ci (dans laquelle R¹, R², R³, R⁴, R⁵, R⁶, R⁷, R⁸, R⁹, R¹⁰, R¹¹, L, le noyau A et le noyau B, n et m sont, sauf indication contraire, tels que définis dans la revendication 1) comprenant :
(a) pour des composés de formule I dans laquelle L est un groupe -N(R⁸)C(O)N(H)-, la réaction d'un composé de formule II : 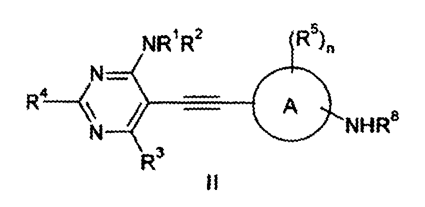 dans laquelle R¹, R², R³, R⁴, R⁵, R⁸, n et A présentent l'une quelconque des significations définies ci-dessus, excepté que tout groupe fonctionnel est protégé, le cas échéant, avec un isocyanate de formule IV : 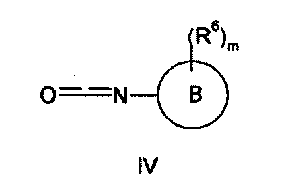 dans laquelle R⁶, m et B présentent l'une quelconque des significations définies ci-dessus, excepté que tout groupe fonctionnel est protégé, le cas échéant ; ou
(b) pour des composés de formule I dans laquelle L est un groupe -N(R⁸)C(O)N(H)-, la réaction d'un composé de formule II telle que définie ci-dessus avec un carbamate d'aryle de formule III : 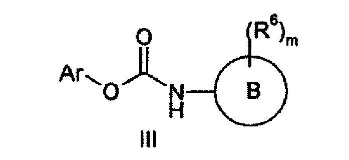 dans laquelle Ar est un groupe aryle approprié, par exemple, un groupe phényle, et R⁶, m et B présentent l'une quelconque des significations définies ci-dessus, excepté que tout groupe fonctionnel est protégé, le cas échéant ; ou
(c) pour des composés de formule I dans laquelle L est un groupe N(R⁸)C(O)-O-, la réaction d'un composé de formule II telle que définie ci-dessus, avec un composé de formule XI : 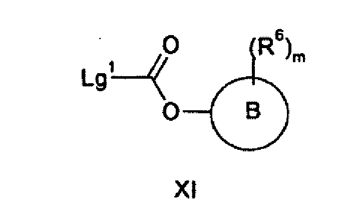 dans laquelle Lg¹ est un groupe remplaçable approprié, par exemple, un groupe halogéno (tel qu'un groupe fluoro, un groupe chloro ou un groupe bromo), et R⁶, m et B présentent l'une quelconque des significations définies ci-dessus, excepté que tout groupe fonctionnel est protégé, le cas échéant ; ou
(d) pour des composés de formule I dans laquelle L est un groupe N(R⁸)C(O)C(R^{a}R^{b}), la réaction d'un composé de formule II telle que définie ci-dessus avec un composé de formule IX : 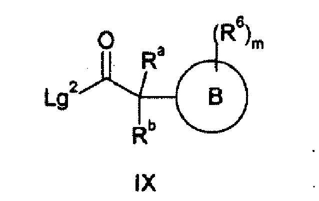 dans laquelle Lg² est un groupe remplaçable approprié, par exemple, un groupe hydroxy, un groupe halogéno (tel qu'un groupe fluoro, un groupe chloro ou un groupe bromo), un groupe R^{x}-C(O)-O- ou un groupe R^{x}-O- (dans lequel R^{x} est un groupe alkyle ou un groupe aryle), et R⁶, R^{a}, R^{b}, m et B présentent l'une quelconque des significations définies ci-dessus, excepté que tout groupe fonctionnel est protégé, le cas échéant ; ou
(e) pour des composés de formule I dans laquelle L est un groupe -N(R⁸)C(O)N(H)-, la réaction d'un composé de formule II telle que définie ci-dessus avec une trichloroacétylamine de formule XIII : 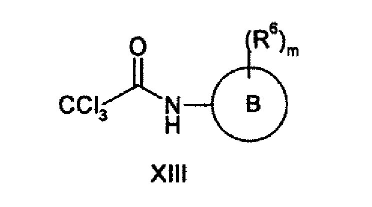 dans laquelle R⁶, m et B présentent l'une quelconque des significations définies ci-dessus, excepté que tout groupe fonctionnel est protégé, le cas échéant ; ou
(f) pour des composés de formule I dans laquelle L est un groupe -C(R^{a}R^{b})C(O)N(R⁹)-, la réaction d'un composé de formule XIV : 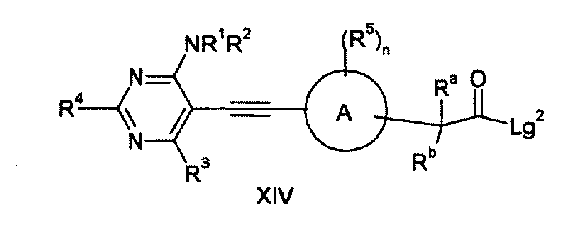 dans laquelle Lg² est un groupe remplaçable approprié tel que décrit ci-dessus, et R¹, R², R³, R⁴, R⁵, R^{a}, R^{b}, n et A présentent l'une quelconque des significations définies ci-dessus, excepté que tout groupe fonctionnel est protégé, le cas échéant, avec une amine de formule XV : 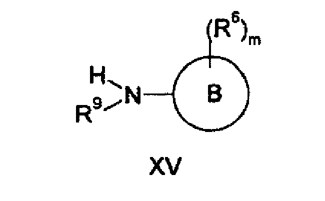 dans laquelle R⁶, R⁹, m et B présentent l'une quelconque des significations définies ci-dessus, excepté que tout groupe fonctionnel est protégé, le cas échéant ; ou
(g) la réaction d'un composé de formule XVI : 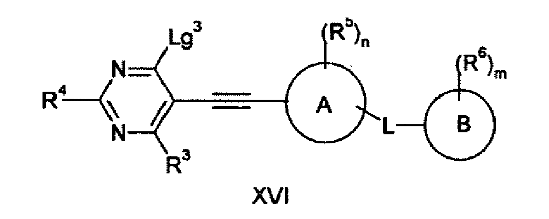 dans laquelle Lg³ est un groupe remplaçable approprié, par exemple, un groupe halogéno (tel qu'un groupe fluoro, un groupe chloro, un groupe bromo ou un groupe iodo), un groupe méthylsulfonyle, un groupe méthylthio ou un groupe aryloxy (tel qu'un groupe phénoxy), et R³, R⁴, R⁵, R⁶, n, m, A, B et L présentent l'une quelconque des significations définies ci-dessus, excepté que tout groupe fonctionnel est protégé, le cas échéant, avec une amine de formule HNR¹R², dans laquelle R¹ et R² présentent l'une quelconque des significations définies ci-dessus, excepté que tout groupe fonctionnel est protégé, le cas échéant ; ou
(h) la réaction d'un composé de formule XVII : 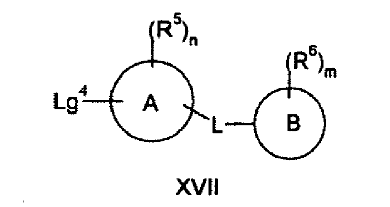 dans laquelle Lg⁴ est un groupe remplaçable approprié, par exemple, un groupe halogéno (tel qu'un groupe chloro, un groupe bromo ou un groupe iodo) ou un groupe sulfonyloxy (tel qu'un groupe trifluorométhylsulfonyloxy), et R⁵, R⁶, n, m, A, B et L présentent l' une quelconque des significations définies ci-dessus, excepté que tout groupe fonctionnel est protégé, le cas échéant, avec un alcyne de formule XVIII : 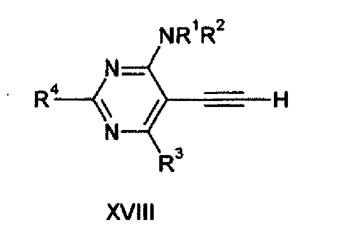 dans laquelle R¹, R², R³ et R⁴ présentent l'une quelconque des significations définies ci-dessus, excepté que tout groupe fonctionnel est protégé, le cas échéant ; ou
(i) pour des composés de formule I dans laquelle L est un groupe -N (H) C (O) N (R⁹) -, la réaction d'un isocyanate de formule XIX : 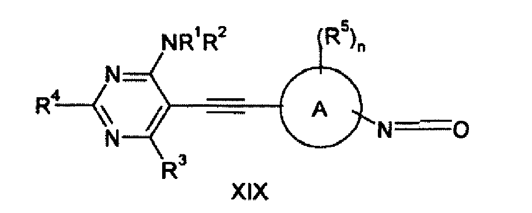 dans laquelle R¹, R², R³, R⁴, R⁵, n et A présentent l'une quelconque des significations définies ci-dessus, excepté que tout groupe fonctionnel est protégé, le cas échéant, avec une amine de formule XV telle que définie ci-dessus ; ou
(j) pour des composés de formule I dans laquelle L est un groupe -N(H)C(O)N(R⁹)-, la réaction d'un composé de formule XX : 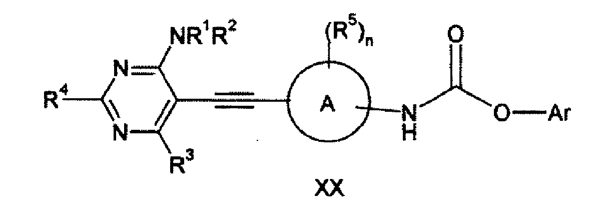 dans laquelle Ar est un groupe aryle approprié, par exemple, un groupe phényle, et R¹, R², R³, R⁴, R⁵, n et A présentent l'une quelconque des significations définies ci-dessus, excepté que tout groupe fonctionnel est protégé, le cas échéant, avec une amine de formule XV telle que définie ci-dessus ;
et ensuite, le cas échéant :
i) la transformation d'un composé de formule (I) en un autre composé de formule (I) ;
ii) l'élimination de tout groupe protecteur ;
iii) la formation d'un sel.

16. Composé choisi parmi les formules II, XIV, XVI, XIX et XX, telles que définies dans la revendication 15, ou composé de formule VIc : 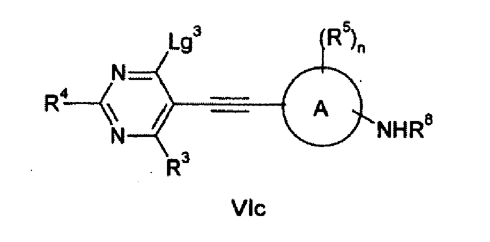 ou un sel de celui-ci, dans laquelle le noyau A, R¹, R², R³, R⁴, R⁵, R⁶, R⁸, L, m, le noyau B et n sont tels que définis dans la revendication 1, Ar est un groupe aryle, Lg² est un groupe hydroxyle, un groupe halogéno, un groupe R^{x}-C(O)-O- ou un groupe R^{x}-O- (dans lequel R^{x} est un groupe alkyle ou un groupe aryle approprié) et Lg³ est un groupe halogéno, un groupe méthylsulfonyle, un groupe méthylthio ou un groupe aryloxy.
